Europäisches Patentamt

European Patent Office

Office européen des brevets

(11) Publication number: **0 250 253**
**A2**

# EUROPEAN PATENT APPLICATION

(21) Application number: **87305452.2**

(22) Date of filing: **19.06.87**

(51) Int. Cl.⁴: **C 07 K 7/00**
**A 61 K 39/29**

(30) Priority: **20.06.86 US 877020   10.06.87 US 60214**

(43) Date of publication of application:
**23.12.87   Bulletin  87/52**

(84) Designated Contracting States:
**AT BE CH DE ES FR GB GR IT LI LU NL SE**

(71) Applicant: **SCRIPPS CLINIC AND RESEARCH FOUNDATION**
**10666 North Torrey Pines Road**
**La Jolla California 92037  (US)**

(72) Inventor: **Millich, David R.**
**11591 Polaris**
**Mica Mesa California 92126  (US)**

**Thornton, George B.**
**11039 Matinal Circle**
**San Diego California 92127  (US)**

(74) Representative: **Fisher, Adrian John et al**
**CARPMAELS & RANSFORD 43 Bloomsbury Square**
**London WC1A 2RA  (GB)**

(54) T and B cell epitopes of the pre-S region of hepatitis B virus surface antigen.

(57) Polypeptides corresponding in amino acid residue sequence to T and B cell epitopes in the pre-S region of HBsAg are disclosed. A method of mitigating nonresponsiveness to an HBV vaccine comprising operatively linking a pre-S1 region T cell epitope to the immunogen of the vaccine is also disclosed.

FIGURE 17

**Description**

# T AND B CELL EPITOPES OF THE PRE-S REGION OF HEPATITIS B VIRUS SURFACE ANTIGEN

Technical Field Of The Invention

The present invention relates to the production of novel synthetic antigens related to the hepatitis B virus surface antigen (HBsAg) protein and to the use of those antigens in the production of vaccines, diagnostic reagents, and the like. More specifically, this invention relates to synthetic antigenic polypeptides that immunologically correspond to a T cell and B cell determinant portion of HBsAg.

Background Of The Invention

Viral hepatitis continues to rank as one of the most important unconquered diseases of mankind. The general term, viral hepatitis, refers principally to hepatitis A (infectious hepatitis), to hepatitis B (serum hepatitis) and to non-A, non-B hepatitis, although other known viruses such as yellow fever virus, Epstein-Barr virus and cytomegalovirus can cause hepatitis in man. Hepatitis is particularly known for its focal attack on the liver (Greek, hepar), but the diesase also influences other organs.

In 1965, Blumberg discovered an antigen circulating in the blood of certain human beings; (1965) J. Am. Med. Assoc., 191:541 and (1967) Ann. Int. Med., 66:924. This substance was subsequently found by Prince to be the surface antigen of hepatitis B virus (HBsAg) that is produced in abundance by individuals who are chronically infected with the viral agent; (1968) Proc. Natl. Acad. Sci. (USA), 60:814.

The naturally occurring hepatitis B surface antigen (HBsAg) is composed of a major polypeptide, referred to as p25, and its glycosylated form, gp28. However, additional polypeptides of higher molecular weight (p39/gp42 and gp33/gp36) have been identified; Heermann et al., (1984) J. Virol. 52:396; Stibbe, et al. (1982) Virol 123:436; Machida, et al. (1984) Gastroenterol. 86:910.

The large open reading frame (ORF) for HBsAg terminates in a single stop codon but can initiate at three possible translational start codons, that define the pre-S1, pre-S2, and S regions, yielding polypeptides referred to as p39, p33 and p25, respectively. All three polypeptides share the 226 amino acid residues of the S region (p25). The p33 consists of the p25 sequence plus an amino-terminal 55 residues (pre-S2); and p39 consists of the p33 sequence plus an amino-terminal 119 residues (pre-S1) in the adw subtype or 108 residues in the ayw subtype due to a $NH_2$-terminal deletion of 11 residues; Tiollais et al., (1981) Science 213:406.

HBsAg has been the subject of extensive immunochemical characterization. Serologic studies show that several strains of the hepatitis B virus (HBV) have one or more determinants in common, which is designated a. Each strain also has two other determinants: either d or y and either w or r. The specificity of HBsAg is associated with a single polypeptide (Gold et al., (1976) J. Immunol. 117:1404 and Shih et al., (1978) J. Immunol., 120:520), the entire 226 amino acid sequence of which is established from the nucleotide sequence of the S gene (Tiollais et al., (1981) Science, 213:406) of HBV; Valenzuela et al., (1979) Nature (London), 280:815; Galibert et al., (1979) Nature (London), 281:646 and Pasek et al., (1979) Nature (London), 282:575.

There is an urgent need for a hepatitis B vaccine for groups that are at an increased risk of acquring this infection. The groups include health care and laboratory personnel, and individuals requiring (1) maintenance hemodialysis; (2) repeated blood transfusions or the administration of blood products; (3) treatment with immunosuppressive or cytotoxic drugs; and (4) treatment for malignant diseases and disorders associated with depression of the immune response. In addition, a vaccine is needed for individuals living in certain tropical areas where hepatitis B infection is prevalent.

Hepatitis A and B viruses, however, do not multiply significantly in cell culture, and there is no current source of laboratory-propagated virus for vaccine preparation. Indeed, there has been a repeated failure to transmit hepatitis B virus (HBV) serially in tissue or organ cultures that has hampered progress towards the development of a conventional vaccine; Zuckerman, (1975) Amer. J. Med. Sci., 270:205.

Classically, a vaccine is manufactured by introducing a killed or attenuated organism into the host along with suitable carriers and/or adjuvants to initiate the normal immune response to the organism while, desirably, avoiding the pathogenic effects of the organism in the host. That approach suffers from several well known limitations. Such vaccines are complex and include not only the antigenic (immunogenic) determinant of interest but many related and unrelated deleterious materials, any number of which can, in some or all individuals, induce an undesirable reaction in the host.

For example, vaccines produced in the classical way can include competing immunogens that are detrimental to the desired immune response, immunogens that induce unrelated immune responses, nucleic acids from the organism or culture, endotoxins and constituents of unknown composition and source. These vaccines, generated from complex materials, inherently have a relatively high probability of inducing competing responses even from the antigen (immunogen) of interest.

Current vaccines for HBV consist of subviral components of the virus surface coat (HBsAg) purified from the plasma of chronically HBV-infected donors and inactivated; MaAuliffe et al., (1980) Rev. Infect. Dis., 2:470. The purification process produces particles containing HBsAg/p25 but not HBsAg/p33 or HBsAg/p39. Clinical trials have demonstrated the safety and efficacy of current HBsAg vaccines but a significant percentage of people are not protected by inoculation with such vaccines due to a genetic inability to respond to HBsAg/p25 (S region nonresponders). Further, such vaccines are limited in supply and are relatively expensive, particularly for those countries with the highest incidence of HBV disease. Chemically synthesized polypeptides, therefore, offer considerable advantages in terms of cost and safety of HBV vaccination programs.

In the past, immunogens (antigens) have been obtained by several methods including derivation from natural materials, coupling of a hapten to a carrier and by recombinant DNA technology. Sela et al. (1971) Proc. Nat. Acad. Sci. (USA), 68:1450; (1969) Science, 166:1365 and (1966) Adv. Immun., 5:129 have also described certain synthetic antigens.

Certain "synthetic" immunogens (antigens) have been prepared by coupling small molecules (for example, dinitrophenol) to carriers (such as keyhole limpet hemocyanin or bovine serum albumin), thus producing immunogens (antigens) that cause the production of antibody to the coupled small molecule. The carrier molecule is usually necessary because the small molecule itself is typically not "recognized" by the immune system of the animal into which it is introduced. This technique has also been employed in isolated instances to prepare immunogens (antigens) by coupling polypeptide fragments of known proteins to carriers.

While this hapten-carrier technique has served the research community well in its investigations of the nature of the immune response, it has not yet been of significant use in the production of immunogens that play a role in diagnostic or therapeutic modalities.

Previous studies by Arnon et al., (1971) Proc. Nat. Acad. Sci. (USA), 68,1450 , Atassi, (1975) Immunochemistry, 12:423 and Vyas et al., (1972) Science, 178:1300 were interpreted by those authors to indicate that short linear amino acid sequences are, in general, unlikely to elicit antibodies reactive with the native protein structure. It was thought that for most regions of most molecules, antigenic determinants resulted from amino acid residues well separated in the linear sequence but close together in the folded protein. The exact three dimensional conformation of the polypeptides used to elicit antibodies was though to be critical in most cases, even for those immunogens and antigens involving amino acid residues close together in a sequence.

However, Sutcliffe et al., (1980) Nature, 287:801 discovered that antibodies to linear polypeptides react with native molecules. Recent investigations have shown that relatively short chemically synthesized polypeptides can elicit antibodies reactive with almost any region of an exposed surface of a protein; Green et al., (1982) Cell, 28:477. Moreover, since amino acid residue sequences can now be determined rapidly with nucleic acid sequencing technology, synthetic polypeptides can be synthesized to make vaccines of a precision not previously possible. Thus, synthetic polypeptides provide an alternative to elaborate biosynthesis.

U.S. Patent No. 4,415,491 to Vyas discloses a series of peptides that correspond to the a determinant of hepatitis B virus surface antigen. Although no data are presented concerning the protection of a host, the peptides are described as being useful in a hepatitis vaccine preparation.

It is known that antisera to synthetic polypeptides predicted from the nucleotide sequence of various regions of the S gene of HBV react with native HBsAg by radioimmunoprecipitation [Lerner et al., (1981) Proc. Natl. Acad. Aci. (USA), 78:3403] and commercial solid phase radioimmunoassays for anti-HBsAg; Gerin et al., (1982) in Viral Hepatitis, Szmuness et al. (eds.), 49-55.

It has also been determined that a pathogen-related protein can be immunologically mimicked by the production of a synthetic polypeptide whose sequence corresponds to that of a determinant domain of the pathogen-related protein. Such findings are reported by Sutcliffe et al., (1980) Nature, 287:801, and Lerner et al., (1981) Proc. Natl. Acad. Sci. (USA), 78:3403.

Moreover, Gerin et al., (1983) Proc. Natl. Acad. Sci. (USA), 80:2365 have recently shown limited protection from hepatitis B virus upon immunization with carrier-bound synthetic polypepides having amino acid residue sequences that correspond to the sequence of a determinant portion of HBsAg; in particular, residues 110-137 of the "S" (surface) region.

Recently two synthetic peptides derived from the pre-S2 region sequence have been reported to elicit antibodies cross-reactive with the native pre-S2 region; Milich et al., (1985) Science, 228:1195; Neurath et al. (1985) Nature (London), 315:154. Denoting the amino-terminus of the pre-S2 region of all subtypes as residue 120 (i.e. p1-119 or p12-119 representing the pre-S1 region), a synthetic peptide corresponding to residues 120 to 145 of the adw2 subtype (p120-145/adw2) was shown to bind human antibodies elicited by HBV infection, and rabbit anti-p120-145 antibodies were able to recognize p33 on native HBsAg particles; Neurath, et al. (1984) Science 224:392. In another study, it was shown that anti-native antibody elicited by immunization with HBsAg/33 particles and anti-p120-145 antibody competed for the same or closely related determinants on native HBsAg/p33 particles; Milich et al., (1985) Science 228:1195.

Similarly, another synthetic peptide designated p133-151/ayw (corresponding to residues 133 to 151 from the amino-terminus of the pre-S region of the ayw subtype) ws utilized to produce monoclonal antibodies that recognized the pre-S2 region on native HBsAg/33 particles; Okamoto et al., (1985) J. Immunol. 134:1. These two pre-S2-specific sequences share residues 133 through 145 with a single subtype-dependent amino acid residue substitution at position 141. The high degree of cross-reactivity between native- and peptide-induced antisera specific for the pre-S2 region or HBsAg, and the resistance of native pre-S2 antigen to reduction and denaturation are consistent with the presence of continuous or sequential, as opposed to conformation-dependent, determinants in the pre-S2 region; Neurath, et al. (1984) Science 224:392; Milich, et al. (1985) Science 228:1195. This is in contrast to the major group and subtype-specific determinants of the S region, which is highly dependent on intact disulphide bonds; Vyas, et al., (1972) Sci. 178:1300; Dreesman et al. (1973) J. Gen. Virol. 19:129.

The construction of a synthetic HBsAg vaccine, however, may require in addition to synthetic polypeptides corresponding to B cell (antibody-inducing) epitopes, synthetic polypeptides corresponding to nonoverlapping T cell determinants (T cell epitopes).

By way of further background, three cellular components of the immune system are B cells (bursa-or bone marrow-derived lymphocytes), T cells (thymus-derived lymphocytes) and macrophages. B cells circulate in the blood and the lymph fluid and are involved in the production of antibodies. T cells amplify or suppress the response by B cells.

Macrophages, on the other hand, are involved in presenting and concentrating antigens to B and T cells. Moreover, macrophages secrete several biologically active mediators that regulate the type and magnitude of both T and B cell responses either by enhancing or suppressing cell division or differentiation. Macrophages are nonspecific and react against any foreign antigen. T and B cells, however, are antigen-specific and react via cell membrane receptors that are specific for the particular antigen.

In mice, the in vivo antibody production to HBsAg is regulated by at least two immune response (Ir) genes, one in the I-A subregion (Ir-HBs-1) and one in the I-C subregion (Ir-HBs-2) of the murine H-2 complex. It was observed that immunization with a chemically synthesized peptide corresponding to the d determinant did not distinguish between high and non-responder murine strains; Milich et al., (1983) J. Immunol., 130:1401. This suggests that Ir-restriction may occur through T cell recognition of addition, perhaps nonoverlapping, regions of the molecule.

The linkage between the major histocompatibility complex and the regulation of immune responsiveness to HBsAg in mice has been extended to the human immune response by the report of an association between HLA-DR phenotype and nonresponsiveness to a recent trial HBsAg vaccine. Thus, the construction of an HBV vaccine ideally includes, in addition to B cell immunogenic epitopes, a sufficient diversity of T cell epitopic determinants to accommodate the genetic variation in epitope recognition of an outbred human population.

Further, while various synthetic polypeptides and purified HBsAg protein preparations are known, the fine specificity of the immunogenicity of pre-S region is not understood. In addition, the relationship between the immunogenicity of the pre-S1 region and the pre-S2 and S regions is not known.

It would be particularly useful to elucidate the relationship between the pre-S1 region and the remainder of HBsAg. It would also be useful to determine the specific location of T and B cell epitopes in the pre-S region of HBsAg so that only sequences containing the amino acid residues of those epitopes need be included in synthetic vaccines. Further, understanding the relationship between the size of a synthetic polypeptide and the effect of using a non-HBsAg carrier as a T cell stimulator, and the specificity of elicited antibodies would enhance the ability to prepare an immunogen for the desired purpose.

## Summary Of The Invention

The present invention relates to polypeptides having amino acid residue sequences that correspond to portions of the pre-S region of the hepatitis B virus surface antigen (HBsAg) protein containing a T cell epitope and/or a B cell epitope.

In one embodiment, the present invention contemplates a polypeptide of about 6 to about 50 amino acid residues. That polypeptide consists essentially of an amino acid residue sequence that corresponds to a portion of the pre-S region of the HBsAg protein located between an amino-terminal and a carboxy-terminal position, respectively, selected from the group consisting of 12-21, 16-27, 94-105, 106-117, 120-126, 120-132, 120-140, 128-138, 133-139, 133-140, 133-143, 133-145, 134-139, 135-143, 135-145, 137-143, 137-145 and 140-151 from the amino-terminus of the pre-S region. The polypeptide is free from amino acid residues at the enumerated amino-terminal and carboxy-terminal positions that correspond to contiguous amino acid residues in the linear sequence of HBsAg.

In another embodiment, the invention contemplates a composite immunogen of at least 14 amino acid residues comprising a first polypeptide (a) having a terminal residue that is operatively linked to a terminal residue of a second polypeptide, polypeptide (b). Polypeptide (a) consists essentially of an amino acid residue sequence T cell epitope that corresponds to a portion of the pre-S region of the HBsAg protein located between an amino-terminal and carboxy-terminal position, respectively, selected from the group consisting of 12-21, 94-105, 106-117, 120-126, 120-132, 120-140 and 140-151 from the amino-terminus of the pre-S region. Polypeptide (b) consists essentially of an amino acid residue sequence that corresponds to an amino acid residue sequence of HBsAg that contains a native B cell epitope. The terminal residue of polypeptide (a) that is operatively linked to the terminal residue of polypeptide (b) correspond to positions separated in the linear sequence of HBsAg by at least 10 amino acid residues.

The invention also contemplates a composite immunogen comprising a polypeptide operatively linked to a non-HBsAg B cell epitope. The polypeptide consists essentially of an amino acid residue sequence T cell epitope that corresponds to a portion of the pre-S region of HBsAg located between an amino-terminal and carboxy-terminal position, respectively, selected from the group consisting of 12-21, 94-105, 106-117, 120-126, 120-132, 120-140 and 133-145 from the amino-terminus of the pre-S region.

A method of enhancing the immunogenicity of an immunogen is also contemplated. The method comprises operatively linking the immunogen to polypeptide consisting essentially of an amino acid residue sequence that corresponds to a portion of the pre-S region of HBsAg containing a T cell epitope. The portion is located between an amino-terminal and carboxy-terminal position, respectively, selected from the group consisting of 12-21, 94-105, 106-117, 120-126, 120-132, 120-140 and 133-151 from the amino-terminus of the pre-S region.

A method of mitigating nonresponsiveness to an HBV vaccine is further contemplated. That method comprises operatively linking an amino acid residue sequence corresponding to a native T cell epitope in the pre-S1 region of HBsAg to the immunogen of the vaccine.

The present invention provides several benefits and advantages. One benefit of the present invention is that nonresponsiveness to an HBV vaccine can be mitigated. Another benefit is that particular T and B cell epitopes have been localized within sequenes of the pre-S region thereby permitting use of specific epitopes that provide particular, desired, features to an inoculum. Thus, a sequence of only those residues need be synthesized for an immunogen. One advantage of the present invention is that the T cell epitopes can be operatively linked to non-HBsAg B cell epitopes to provide immunogens of relatively low molecular weight that can induce secretion of antibodies to a narrow range of epitopes. Still further benefits and advantages of the invention will be apparent to those skilled in the art from the discussion that follows.

Brief Description Of The Drawings

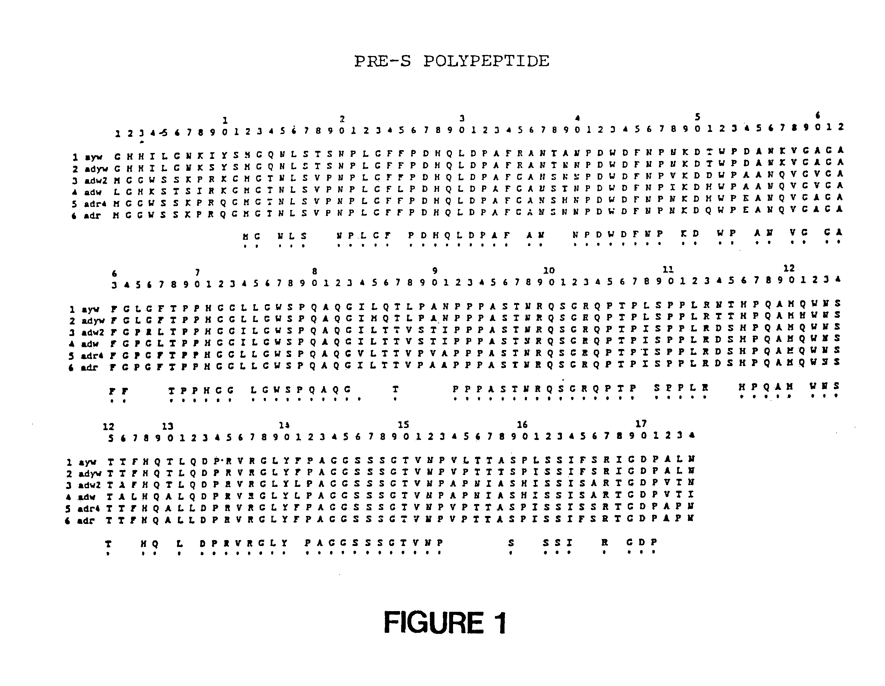

Figure 1 illustrates the amino acid residue sequence from left to right, in the direction from amino-terminus to carboxy-terminus and using single letter amino acid residue code, of the pre-S region of HBsAg of six subtypes of HBV. The additional line of residues beneath the six sequences lists the residues that are homologous in all six subtypes. The numerals above the sequences illustrate the sequence positions from the amino-terminus, with each tenth position numeral being presented vertically rather than in the usual horizontal manner. The sequences for the subtypes were reported as follows: ayw - Galibert et al., (1979) Nature, 281:646; adw/adr - Ono et al., (1983) Nucl. Acids Res., 11:1747; adw2 - Valenzuela et al., (1980) in ICN-UCLA Symposia on Animal Virus Genetics, Fields et al., eds. pp 57-70, Academic Press; adyw - Pasek et al., (1979) Nature, 282:575; and adr4 - Fujiyama et al., (1983) Nucl. Acids Res., 11:4601.

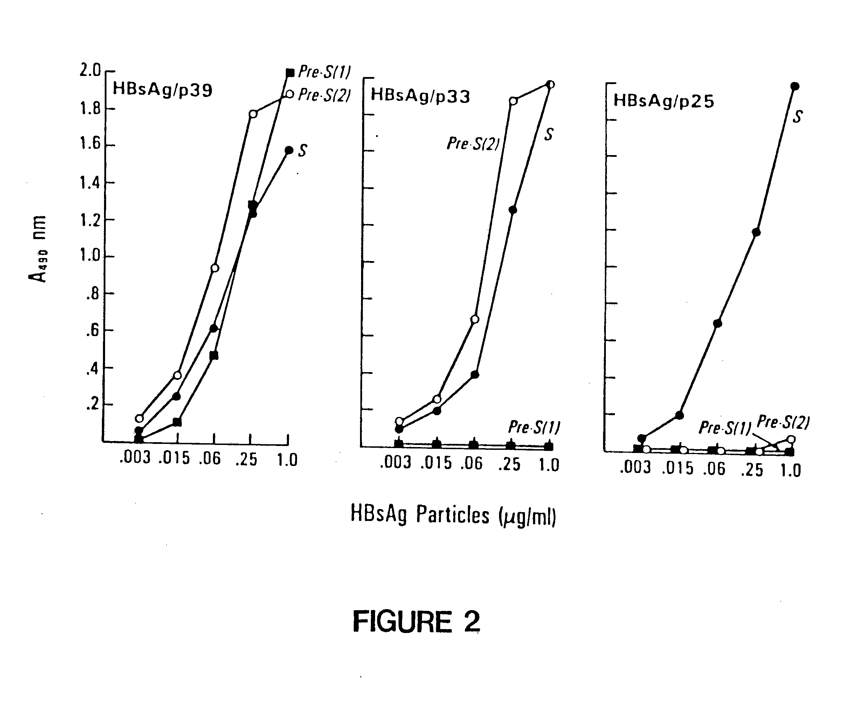

Figure 2 is a series of three graphs that illustrates relative antigenicities of S, pre-S2 and pre-S1 region-containing compositions of the HBsAg particles utilized in the studies that form the basis of the present invention. HBsAg/p39, HBsAg/p33, and HBsAg/p25 particles at the indicated concentrations were assayed for S, pre-S2 and pre-S1 region antigenicity by solid phase, sandwich ELISA methods using monoclonal anti-S-, anti-pre-S2- and anti-pre-S1-specific reagents. The results are expressed as absorbance units at 490 nanometers (nm) ($A_{490}$ units) corrected for background. The X axis represents the concentration of HBsAg particles in micrograms per milliliter (ug/ml). The Y axis represents $A_{490}$ units. The solid squares represent pre-S1; the open circles, pre-S2; and the solid circles, S.

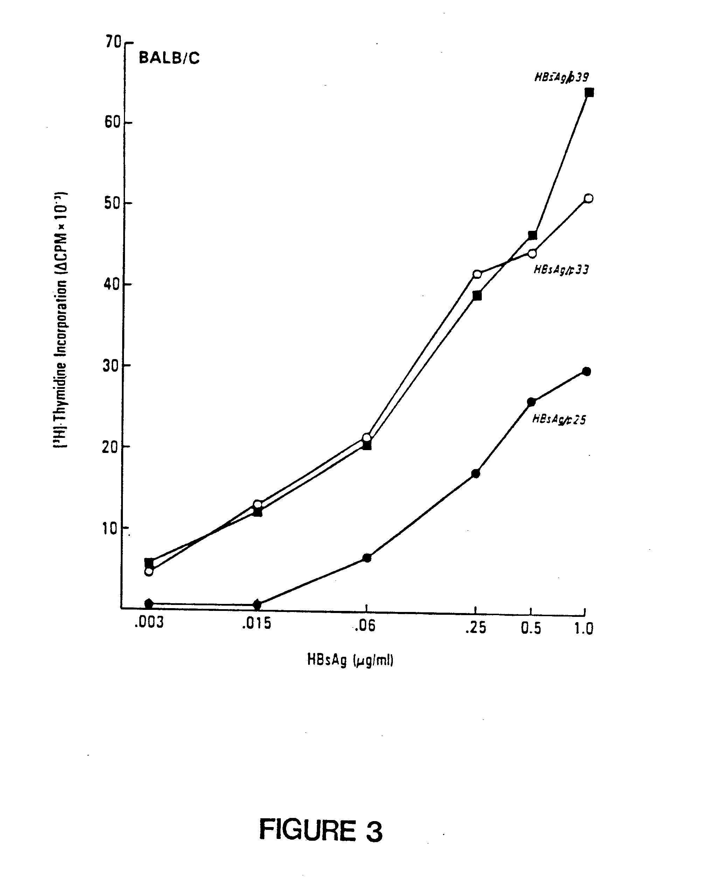

Figure 3 is a graph illustrating the (H-$2^d$) T cell proliferative response of Balb/c mice following immunization with HBsAg/p35. A group of four mice was primed with 4 mg of HBsAg/p39 per mouse. Popliteal lymph node (PLN) T cells were harvested, pooled and cultured in vitro with varying doses (concentrations) of HBsAg/p39, HBsAg/033, HBsAg/p25 or media alone. The T cell proliferative responses were determined. The results are expressed as the amount of [$^3$H]-thymidine incorporation corrected for background proliferation in media alone in counts per minute ($\Delta$ CPM). Background proliferation ranged from 1000 to 4000 cpm. The X axis represents the concentration of HBsAg in ug/ml. The Y axis represents [$^3$H]-thymidine incorporation in CPM ($\Delta$ CMP x $10^{-3}$). The solid squares represent HBsAg/p39; open circles, HBsAg/p33; and solid circles, HBsAg/p25.

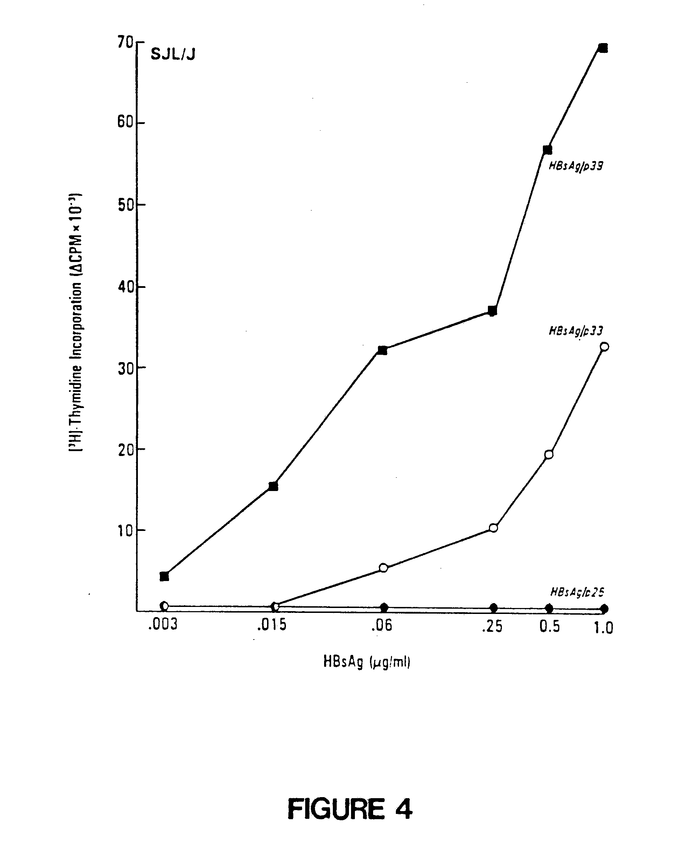

Figure 4 is a graph representing the SJL/J (h-$2^s$) mouse T cell proliferative response following immunization with HBsAg/p39. The T cell response was analyzed as described in Figure 3.

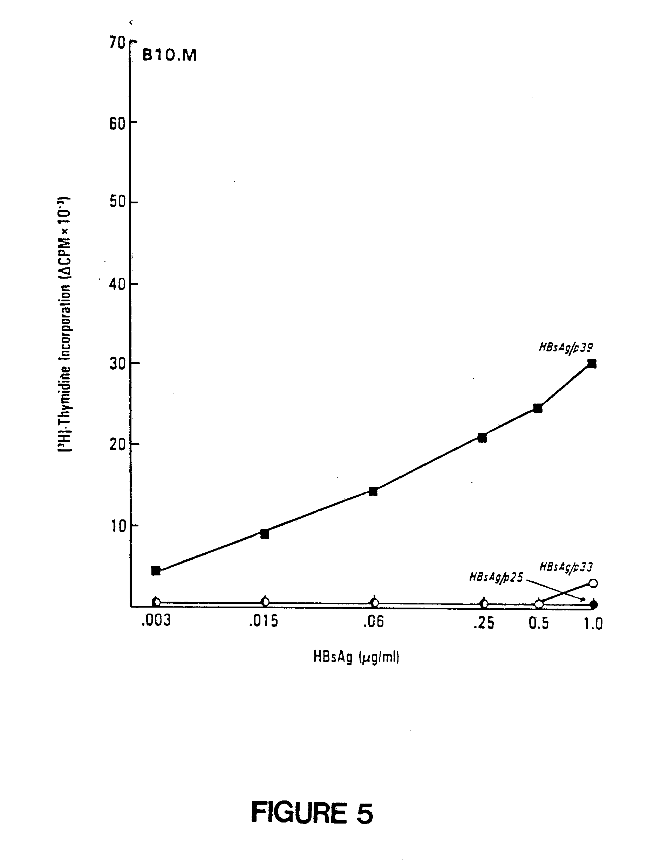

Figure 5 is a graph illustrating the B10.M (H-$2^f$) mouse T cell proliferative response following immunization with HBsAg/p39. The T cell response was analyzed as described in Figure 3. The X and Y axes and symbols representing the HBsAg proteins are as described in Figure 3.

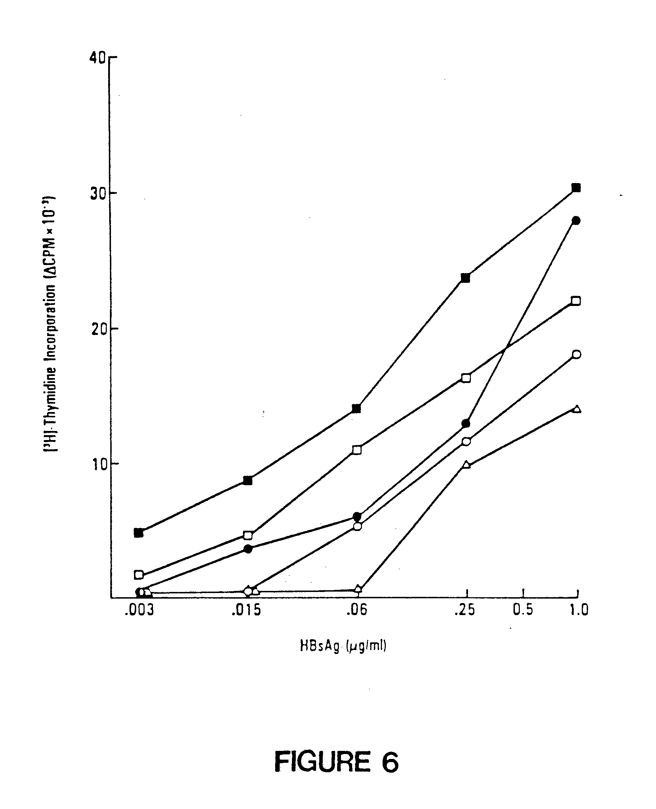

Figure 6 is a graph showing that the B10.M strain T cell proliferative response to HBsAg/p39 was specific for the pre-S1 region. HBsAg/p39-primed T cells were cultured in vitro with five separate HBsAg/p39 preparations possessing equivalent S region antigenicities, and varying percentages of pre-S1-bearing portions. The immunogen represented by solid squares contained 5.1% pre-S1 region-bearing molecules, while the other pre-S1 amounts were as follows: open squares, 2.5%; solid circles, 2.2%, open circles, 2.0%, and open triangles, 1.6%. The T cell proliferative responses were analyzed as described in Figure 3. The X and Y axes are as described in Figure 3.

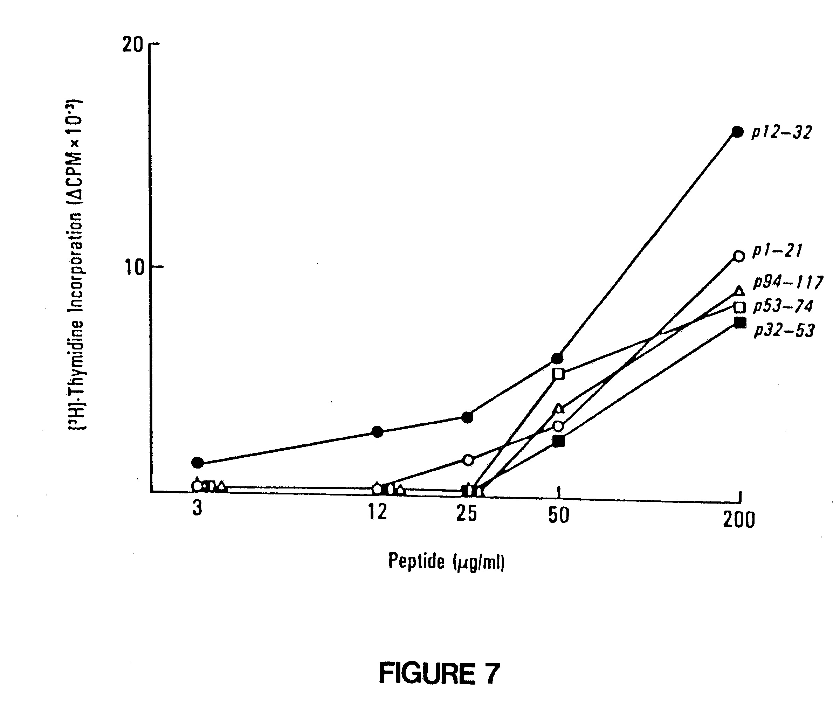

Figure 7 is a graph demonstrating that murine SJL/J strain, HBsAg/p39-primed T cells were activated by pre-S1-specific synthetic polypepotides in vitro. SJL/J mice were immunized with four ug of HBsAg/p39 and draining PLN cells were cultured with varying concentrations of the indicated pre-S1-specific synthetic peptides. The proliferative T cell responses were determined by [$^3$H]-thymidine incorporation, as discussed before. The Y axis is as described in Figure 3. The X axis represents the concentration of the polypeptide (Peptide) in ug/ml. Polypeptides used as named by their amino- and carboxy-terminal amino acid residue sequence positions preceded by the letter "p" to indicate their being polypeptides, and were: p12-32 - solid circles, p1-p21 - open circles, p94-117 - open triangles, p53-74 - open squares, and p32-53 - solid squares.

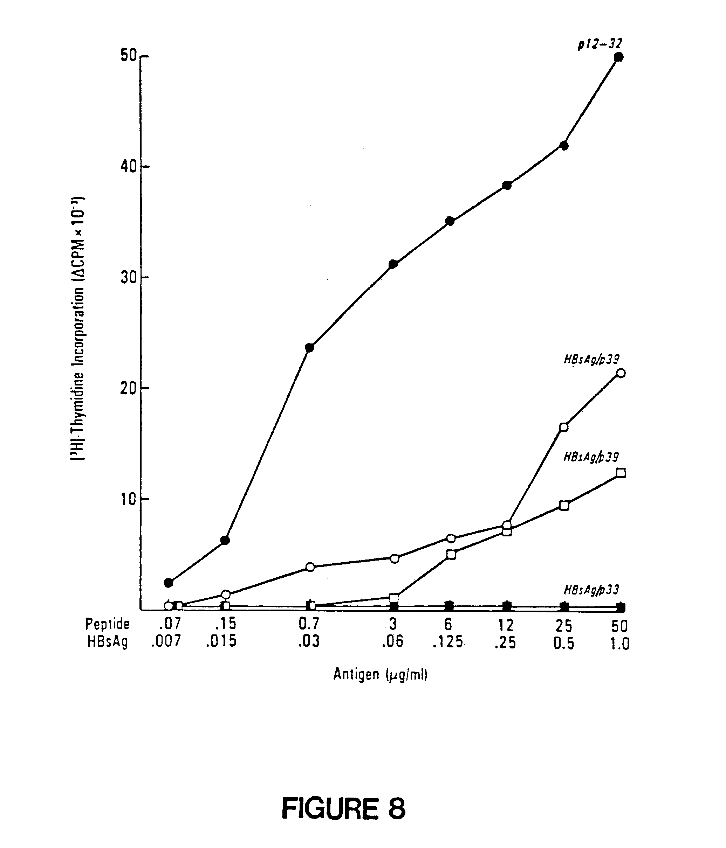

Figure 8 is a graph demonstrating that SJL/J strain, p12-32-primed T cells were activated in vitro by p12-32 and HBsAg/p39. SJL/J mice were immunized with 100 ug of p12-32 and draining PLN cells were cultured with the indicated concentrations of p12-32 (closed circles), each of two HBsAg/p39 preparations (open circles and open squares), or HBsAg/p33 (closed squares) as antigen. The T cell proliferative responses were determined as described in Figure 3. The Y axis is as described in Figure 3. The X axis respresents the concentration of antigen (HBsAg or peptide) in ug/ml.

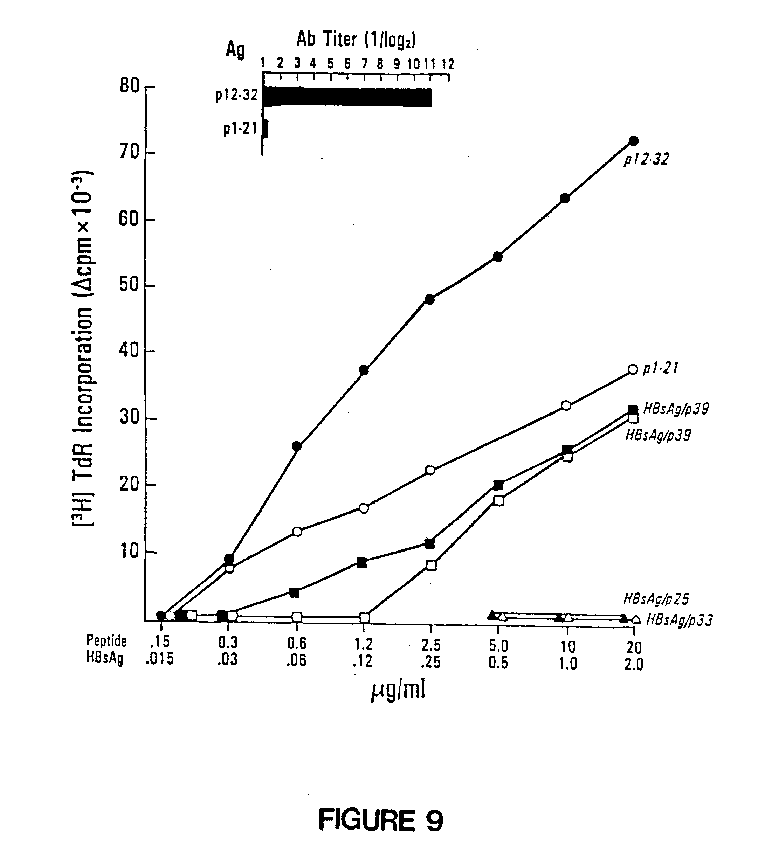

Figure 9 is a graph illustrating the T cell proliferative response of SJL mice (S region nonresponders) immunized with 100 ug of p12-32 per mouse. Popliteal lymph node (PLN) T cells were harvested, pooled and cultured in vitro with varying concentrations of p12-32 (solid circles), p1-21 (open circles), two preparations of HBsAg/p39 (solid and open squares), HBsAg/p33 (open triangles) or HBsAg/p25 (solid triangles) as antigen. The results are expressed as the amount of [$^3$H]-thymidine ([$^3$H]TdR) incorporation corrected for background proliferation in media alone in counts per minute ($\Delta$ cpm). The X axis represents the concentration of antigen, either peptide or particle, in micrograms per milliliter (ug/ml). The y axis represents the amount of [$^3$H]TdR incorporation in cpm. The inset bar graph illustrates the reactivity of antibody induced by p12-32 with p12-32 or p1-21 as determined by ELISA.

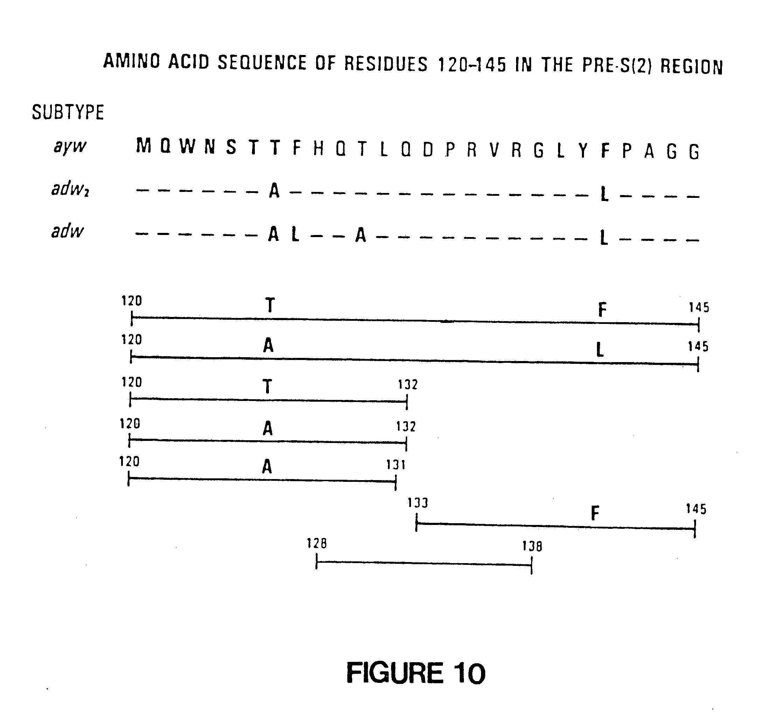

Figure 10 represents the amino acid residue sequences of residue positions 120 through 145 from the amino-terminus of the pre-S2 region of the ayw, adw$_2$ and adw subtypes of HBV, as are also shown in Figure 1. The sequences are presented in the one-letter code system which is included hereinafter in Table 1. A dashed line represents a residue unchanged from the ayw sequence. Also depicted are the sequences of synthetic polypeptides used in the studies that form the basis of the present invention, and in which the sequence utilized was that of the ayw subtype except for substituted residues that are indicated by the letters on the lines. The residues of the ayw sequence at positions 126 and 141 in the line-depicted sequences to illustrate positions of changes in other synthetic polypeptides.

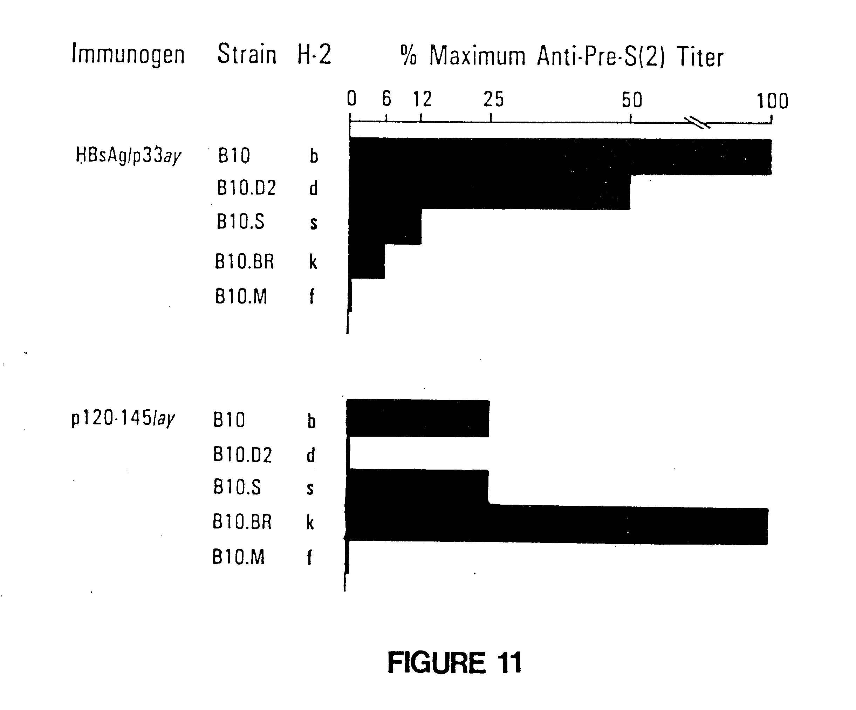

Figure 11 consists of two series of bar graphs that illustrates distinct H-2 restriction of the immune responses to the native pre-S2 region on HBsAg/p33/ay particles and the pre-S2 region synthetic peptide p120-145/ay. Mice from a series of H-2 congenic strains were immunized with either HBsAg/p33/ay (upper panel), or p120-145 /ay (lower panel). Pre-S2-specific antibody production, determined by radioimmunoassay (RIA) using p120-145/ay as the solid phase-bound ligand, was analyzed following primary immunization with HBsAg and secondary immunization with p120-145. The results are expressed as a percentage of the highest responder strain's response; i.e., B10 immunized with HBsAg/p33, 1:4096; B10.BR immunized with p120-145, 1:80,000. The H-2 haplotype of each strain is shown.

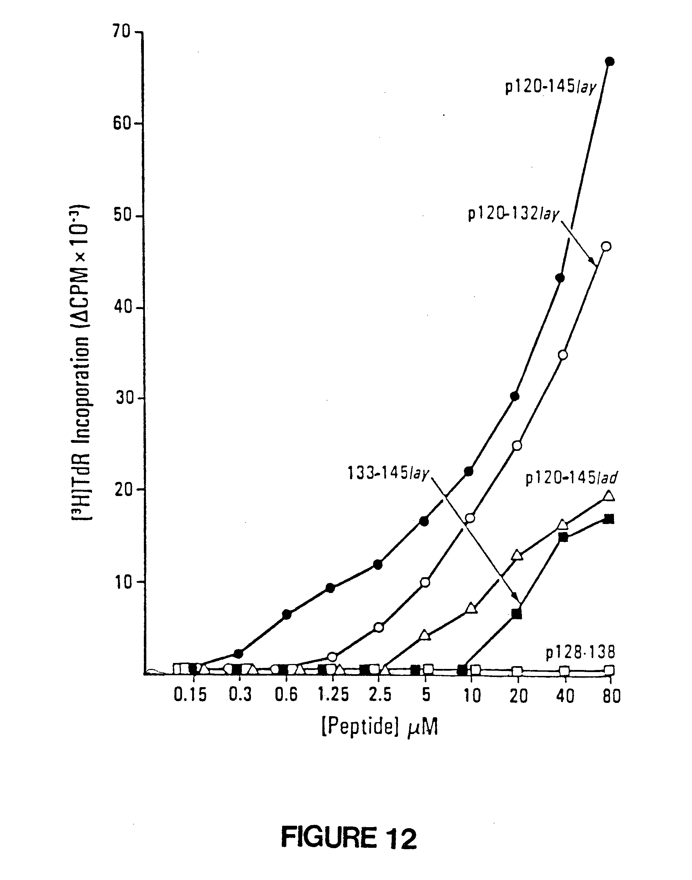

Figure 12 illustrates the C3H.Q mouse T cell proliferative response following p120-145/ay immunization. A group of C3H.Q mice were immunized with 100 ug of p120-145/ayw per mouse. T cell proliferation specific for the indicate polypeptides (Peptides) was determined by incorporation of [$^3$H]thymidine ([$^3$H]TdR) and expressed as counts per minute (cpm) adjusted for background ($\Delta$ cpm). The Y axis is as described in Figure 3. The X axis represents the concentration of polypeptide in micromoles (uM). Polypeptides are represented as follows: p120-145/ay - solid circles, p120-132/ay -open circles, p120-145/ad - open triangles, p133-145/ay - solid squares, and p128-138 - open squares.

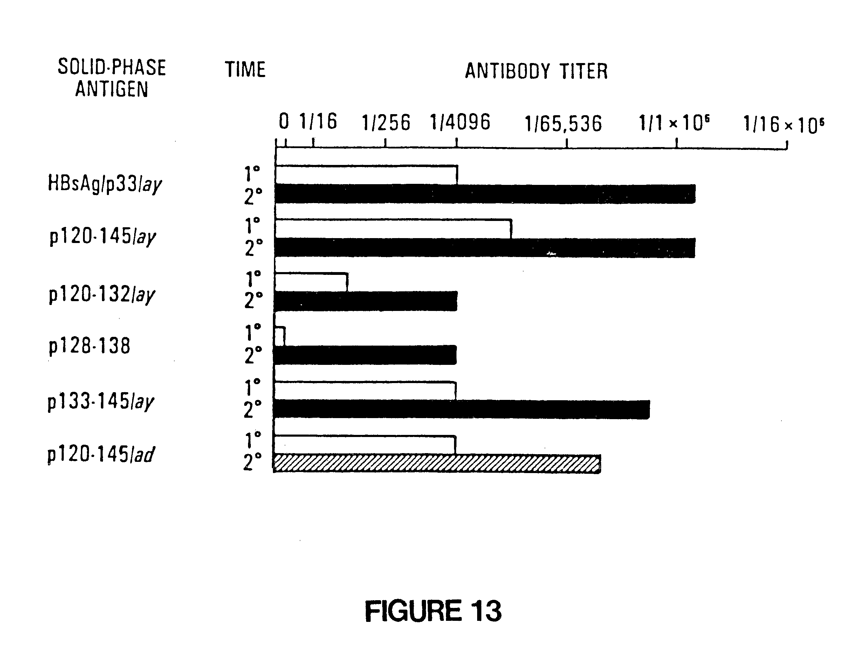

Figure 13 contains a series of bar graphs that illustrate in vivo antibody production following p120-145/ay immunization in C3H.Q mice. A group of five C3H.Q mice were immunized with 100 ug of p120-145./ayw per mouse. Sera were collected 24 days following primary immunization (1°) and 2 weeks following secondary immunization (2°) with a one-half dose of immunogen. Sera were pooled and analyzed by RIA for binding to the indicted solid phase-bound ligands. The results are expressed as the maximum sera dilution to yield three times the counts of preimmunization sera (Titer).

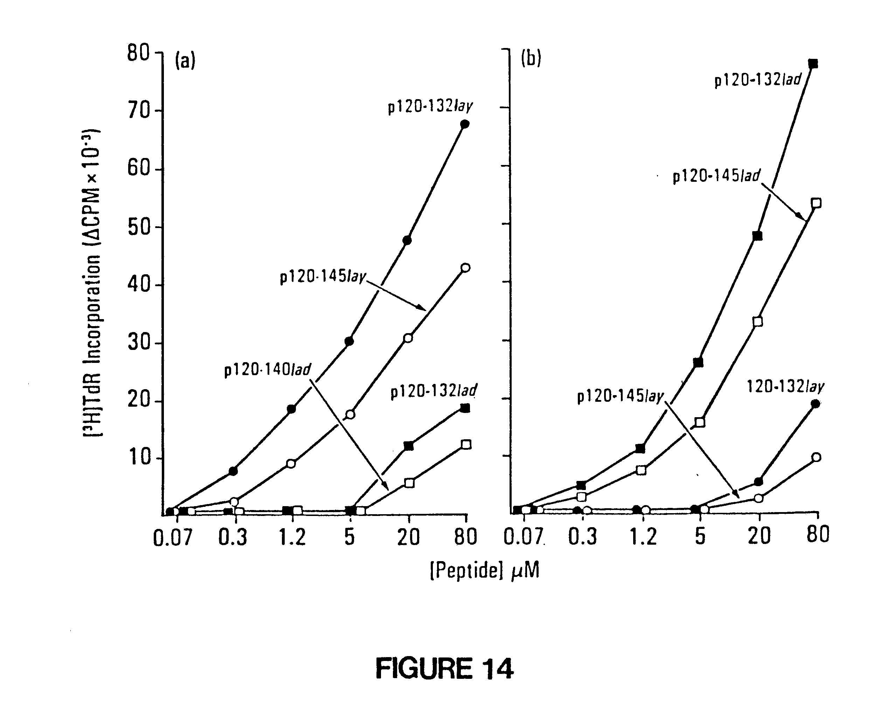

Figure 14 is a series of two graphs that illustrate the C3H.Q T cell proliferative response following immunization using p120-132 as immunogen. Groups of three C3H.Q mice were immunized with 100 ug of either p120-132/ayw (panel a) or p120-132/adw$_2$ (panel b) per mouse. T cell proliferation specific for the indicated peptides was determined as described in Figure 12. The X and Y axes are as described in Figure 12. The antigens are p120-132/ay - solid circles, p120-145/ay - open circles, p120-132.ad - solid squares, and p120-140/ad - open squares.

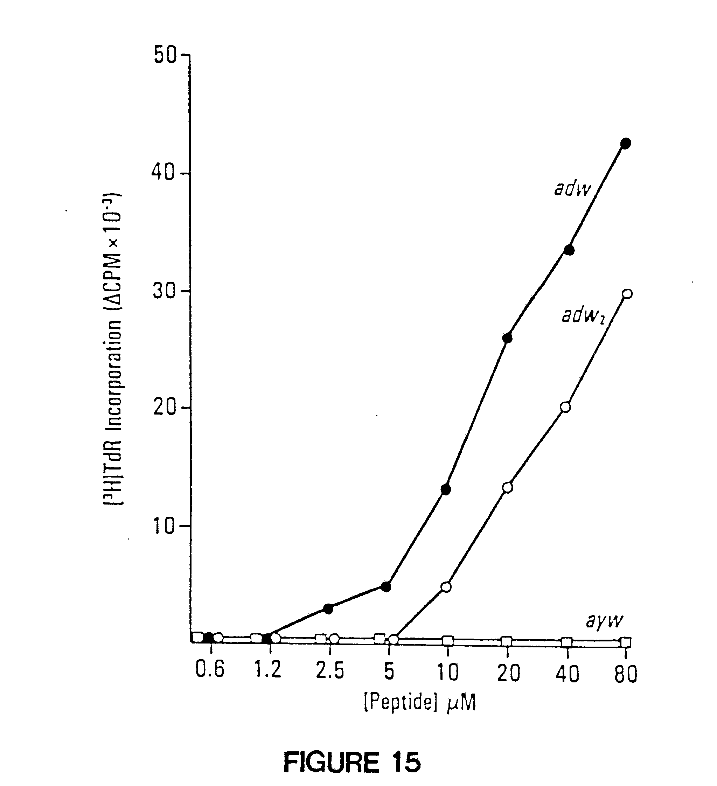

Figure 15 is a graph illustrating the influence of subtype on the C3H.Q T cell proliferative response following immunization with p120-131/adw. A group of three C3H.Q mice were immunized with 100 ug of p120-131./adw. T cell proliferation induced by the homologous peptide (p120-131/adw) - solid circles, p120-132/adw$_2$ -open circles and p120-132.ayw - open squares was determined as described in Figure 12. The X and Y axes are as described in Figure 12.

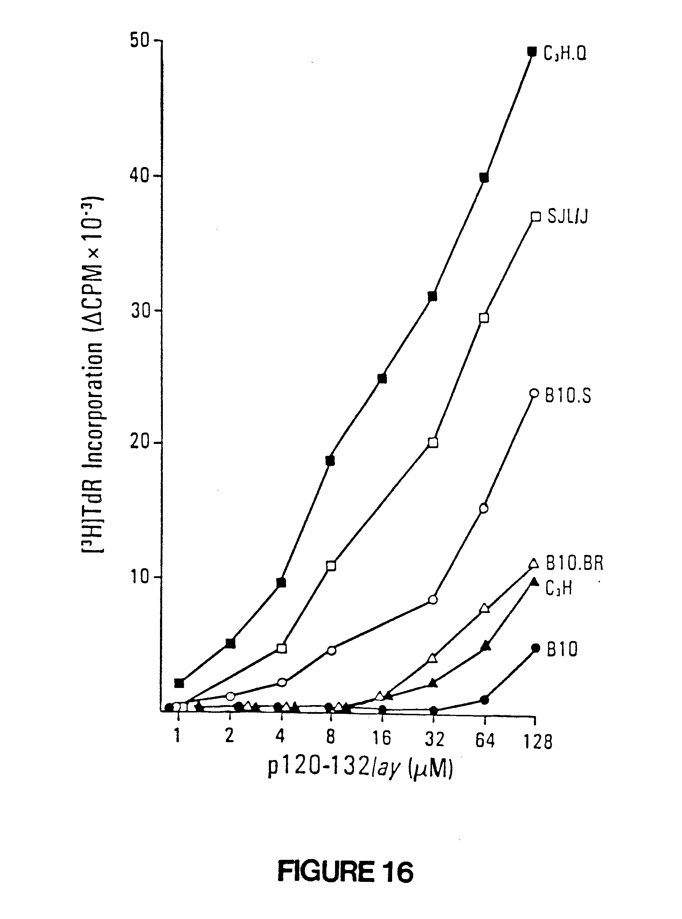

Figure 16 illustrates strain variability of the p120-132-specific T cell response following immunization using p120-145 as immunogen. Groups of three mice each of the indicated strains were immunized with 100 ug of p120-145/ayw per mouse. T cell proliferation induced by p120-132/ayw was -determined as described in Figure 12. Solid squares represent strain C3H.Q, open squares - SJL/J, open circles - B10.S, open triangles - B10.BR, solid triangles – C3H, and solid circles - B10.

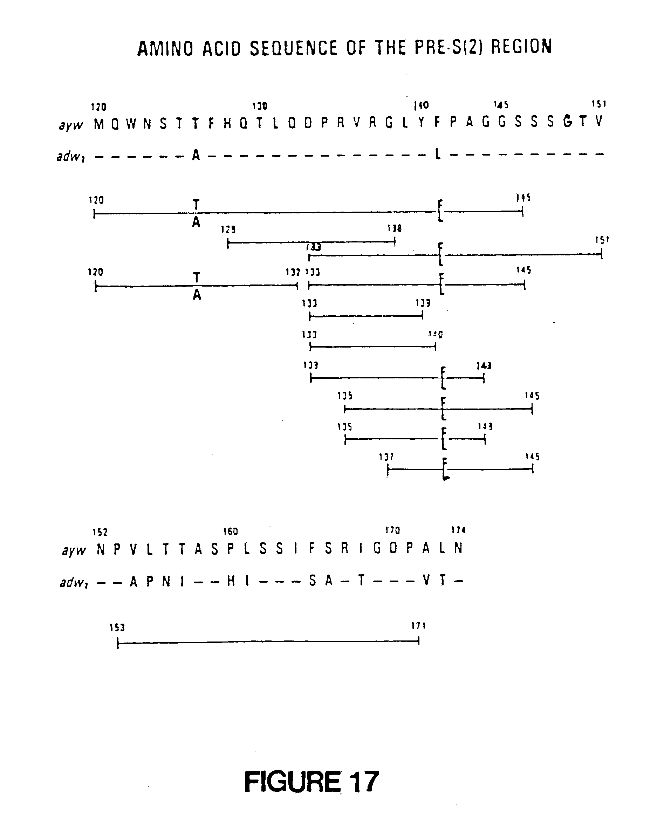

Figure 17 illustrates the amino acid residue sequence of the pre-S2 region of HBsAg of the ayw and adw$_2$ subtypes of HBV as are also shown in Figures 10, 18 and 19. A dashed line represents a residue common to both sequences. Also depicted are synthetic polypeptides used in the studies that form the basis of this invention. In the line-depicted sequences where two sequences of subtypes are shown, a single line is used to represent two polypeptides, with differing residues being shown in either side of the line at the same position, e.g., F and L at position 141.

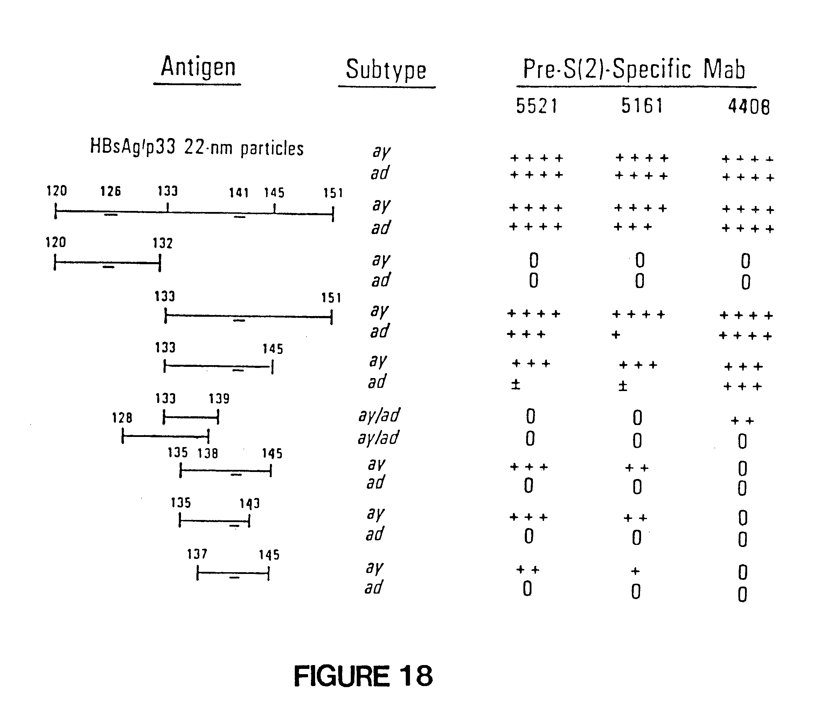

Figure 18 illustrates the reactivity of pre-S2-specific monoclonal antibodies (Mab 5521, 5161 and 4408) with HBsAg/p33 22-nanometer (nm) particles and synthetic polypeptides. Monoclonal antibodies were analyzed for binding to solid phase HBsAg/p33 particles (0.1 ug/well) and synthetic peptides (1.0 ug/well)

6

by RIA. The positions of subtype-dependent, amino acid residue substitutions are indicated by a dashed line. The ayw and adw2 subtype sequences were analyzed. Reactivity is expressed as the minimum concentration (in ng/ml) of monoclonal antibody required to bind five times the background counts: four plus signs (+ + + +), indicate a minimum concentration of from 0.03-0.06 ng/ml; (+ + +), 0.12-0.25; two plus signs (+ +), 0.5-1.0; a plus sign (+), 2.0-4.0; ±, a plus-minus sign 8.0-32; zero 0, less than 500 ng/ml.

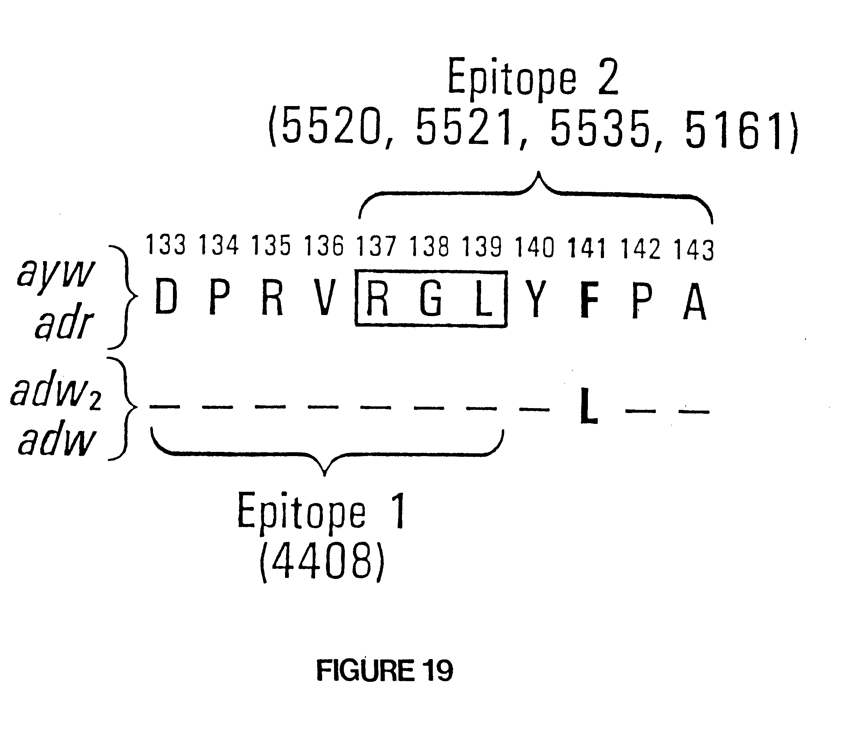

Figure 19 shows the amino acid sequence of two distinct and overlapping antibody binding sites (epitopes) within the pre-S2 region. Epitope 1 (p133-139) was defined by monoclonal 4408, and is conserved in the ayw, adr, adw2 and adw subtypes. Epitope 2 (p137-143) was defined by monclonals 5520, 5521, 5535, and 5161. There was a subtype-dependent, amino acid residue substitution at residue 141 [phenylalanine (ayw, adr); leucine (adw2, adw)]. The three amino acid residue overlap between epitope 1 and 2 is depicted by the residues (RGL) within the rectangle.

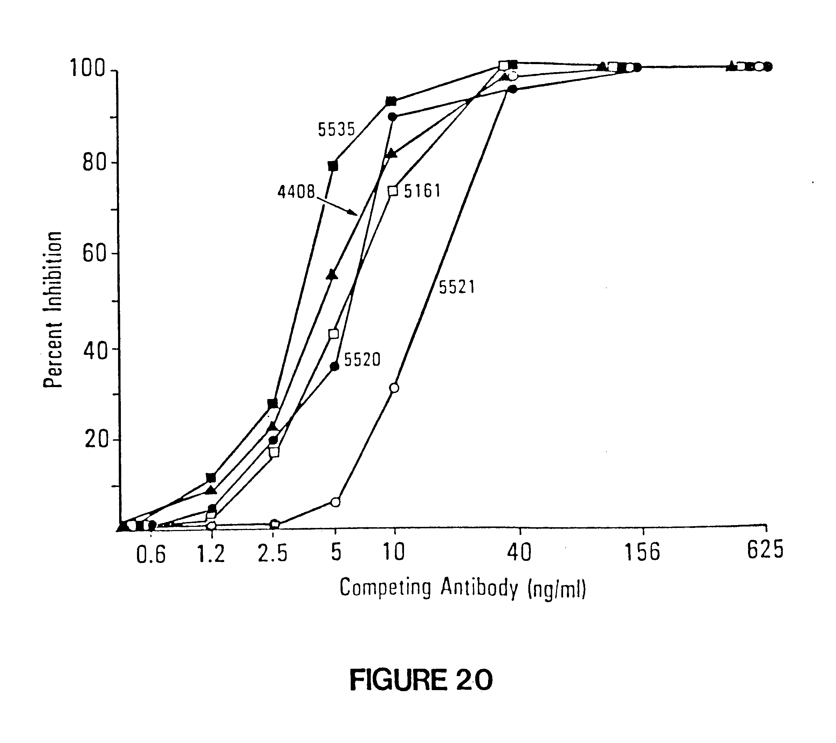

Figure 20 is a graph illustrating quantitative inhibition analysis of monoclonal antibody 4408 binding to HBsAg/p33/ay particles. The indicated pre-S2-specific monoclonal antibodies were preincubated with solid phase HBsAg/p33/ay particles to equilibrium, and subsequently a predetermined concentration of enzyme-labelled monoclonal 4408 was admixed. The percent inhibition by the unlabelled, competing antibodies was determined. Concentrations of the competing antibodies ranged from 0.6 to 625 nanograms per milliliter (ng/ml). The X axis represents the concentration of competing antibody in ng/ml. The Y axis represents the percent inhibition. Solid squares represent Mab 5535, solid triangles - 4408, solid circles - 5520, open squares - 5161 and open circles - 5521.

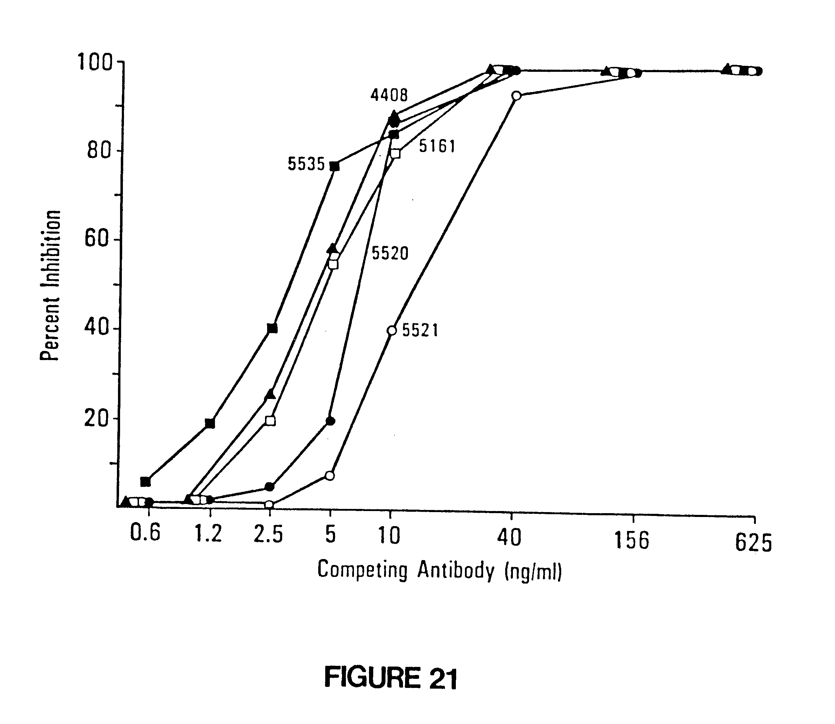

Figure 21 is a graph illustrating the quantitative inhibition analysis of monoclonal antibody 5521 binding to HBsAg/p33/ay particles. The indicated pre-S2-specific monoclonal antibodies were anaylzed for the ability to inhibit enzyme-labelled monoclonal 5521 as described in Figure 20. The X and Y axes and symbols representing the monoclonal antibodies are as described in Figure 20.

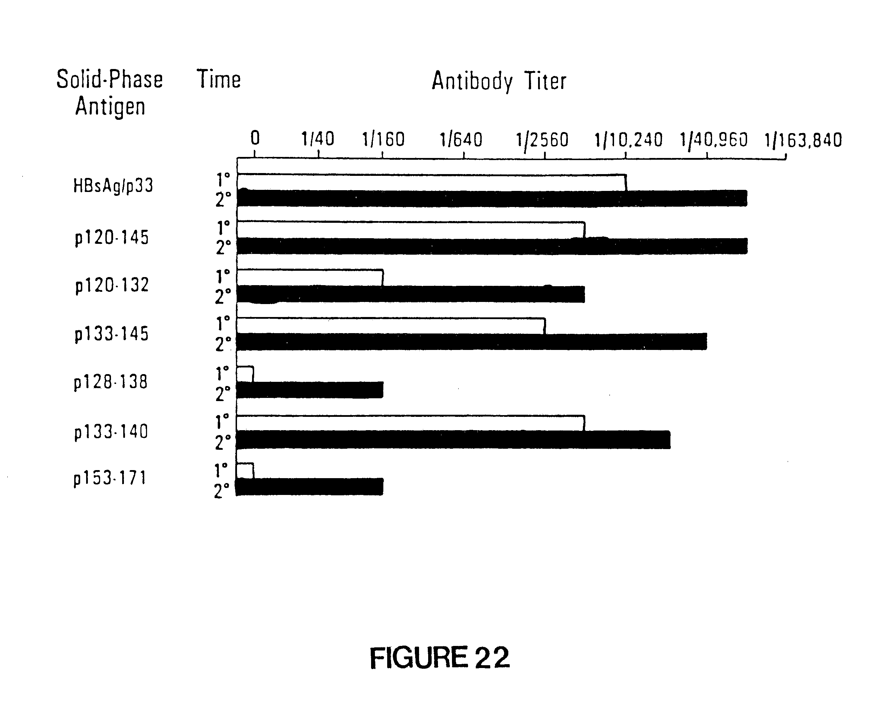

Figure 22 contains a series of bar graphs illustrating the specificity of the pre-S2-specific humoral response following immunization with native HBsAg/p33/ay particles. C3H.Q mice were immunized with 1.0 ug of HBsAg/p33/ay in complete Freund's adjuvant (CFA). Sera were collected 24 days after primary immunization (1°), and two weeks following a secondary (2°) immunization given with 0.5 ug of HBsAg/p33/ay in incomplete Freund's adjuvant (IFA). Sera were analyzed by a solid phase RIA for reactivity with the indicated antigens. Results are expressed as the highest serum dilution to yield three-times the counts of preimmunization sera (Antibody Titer).

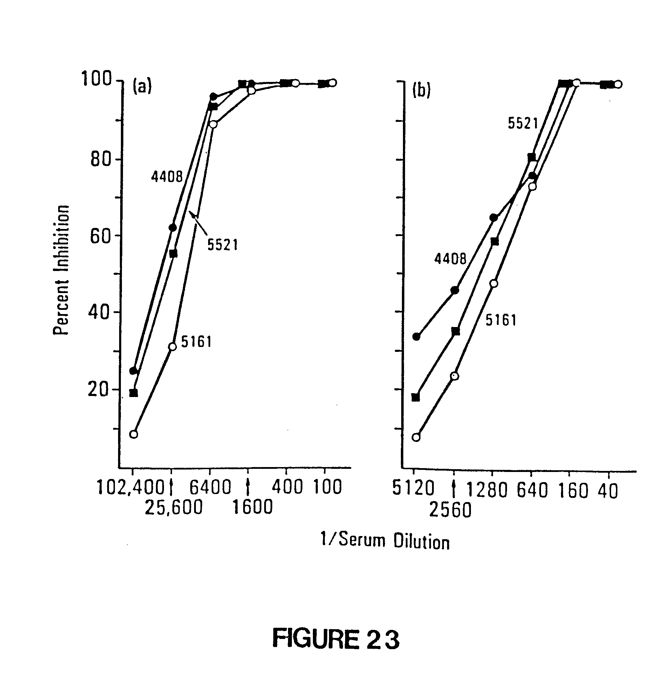

Figure 23 is a series of two graphs that illustrates the quantitative inhibition of pre-2-specific monoclonal antibodies by polyclonal anti-peptide and polyclonal anti-HBsAg/p33 antisera. C3H.Q mice were immunized with either 100 ug of p120-145 (panel a) or 1 ug of HBsAg/p33/ayw (panel b). Six weeks following 1° immunization, mice were given a secondary immunization (2°) with a one half-dose of the same antigen. Fourteen days later sera were collected and examined for the ability to inhibit the binding of the indicated pre-S2-specific, enzyme-labelled, monoclonal antibodies to solid phase-bound HBsAg/p33 particles. Results are expressed as the percent inhibition by a given serum dilution. The X axis represents the reciprocal serum dilution, while the Y axis represents the percent inhibition. Monoclonal antibody 448 is represented by solid circles, 5521 - solid squares, and 5161 -open circles.

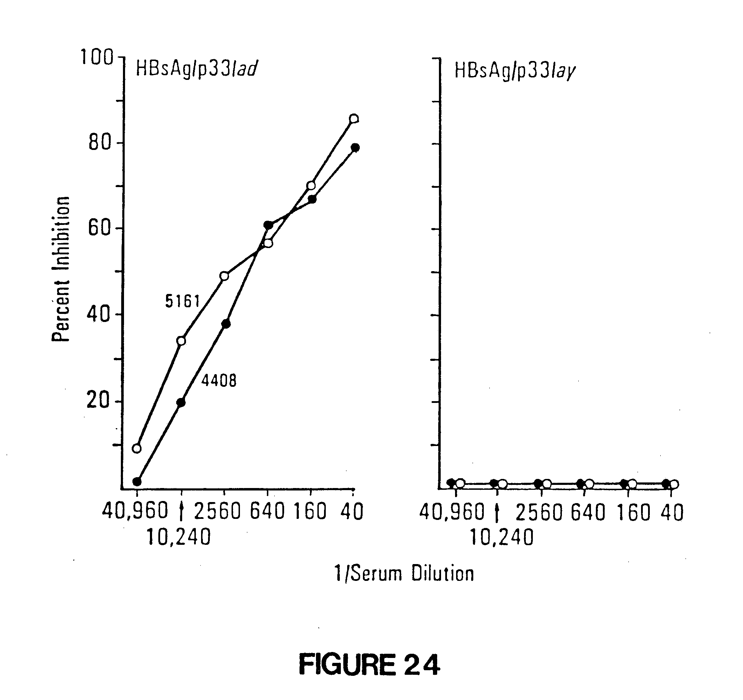

Figure 24 is a graph illustrating the quantitative inhibition of pre-S2-specific monoclonal antibodies by polyclonal anti-peptide antisera specific for a nonoverlapping pre-S2 determinant. Balb/c mice were immunized with 100 ug of p153-171/adw2. After secondary immunization, serum was examined for the ability to inhibit the binding of monoclonal antibodies 5151 and 4408 to HBsAg/p33 particles of the adw2 (left panel; HBsAg/p33/ad) and the ayw (right panel; HBsAg/p33/ay) subtypes. Results are expressed as the percent inhibition by a given serum dilution. The X and Y axes are as described in Figure 23. Solid circles represent Mab 4408, open circles - Mab 5161.

Detailed Description Of The Invention

I. Introduction

It has been found that inoculation of some strains of animals that do not respond to the pre-S2 and S regions of HBsAg with immunogens containing pre-S1 region T cell epitopes stimulated the production of antibody to both the pre-S2 and S regions in addition to pre-S1 specific antibody, thereby bypassing nonresponsiveness.

Further, it has been found that some of the previously known polypeptides related to portions of the pre-S region of HBsAg contain a T cell epitope, a B cell epitope, both T and B cell epitopes and/or additional amino acid residues that are unnecessary to the polypeptides' immunogenic character. In particular, the T and B cell epitopes within sequences 12-32, 94-117, 120-145 and 133-151 of the pre-S region of HBsAg have now been elucidated. The T cell epitopes correspond to portions of the pre-S region located between amino-terminal and carboxy-terminal positions 12-21, 94-105, 106-117, 120-126, 120-132, 120-140 and 140-151 from the amino-terminus of the pre-S region. The B cell epitopes within those sequences are located in portions of the pre-S region between amino-terminal and carboxy-terminal positions, 16-27, 94-105, 106-117, 120-140, 133-139, 133-140, 133-143, 133-145, 134-139, 137-143 and 137-145 from the amino-terminus of the pre-S region.

It has further been found that adding additional residues to a polypeptide altered the peptide's immunogenicity. For example, addition of as few as 13 residues included in the polypeptide sequence altered the antigen binding properties of induced antibodies, as described in detail in Section VI J and illustrated in Table 14. Additionally, coupling a polypeptide to a carrier such as ovalbumin induced subtype-specific antibody as described in detail in Section VI J and illustrated in Table 14.

The amino acid residue sequence of the pre-S region of five subtypes of HBV utilized herein are illustrated in Figure 1. The single letter amino acid code used in the Figure and elsewhere herein is illustrated below in Table 1.

The full names for individual amino acid residues are sometimes used herein as are the well-known three-letter abbreviations. Single-letter abbreviations (code) is most often utilized. Table 1, below, provides the full name as well as the three-letter and single-letter abbreviations for each amino acid residue named herein (See, for example, L. Stryer, Biochemistry, 2nd ed., W. H. Freeman and Company, San Francisco, (1981), page 16.). The amino acid residues utilized herein are in the natural, L, form unless otherwise stated.

## TABLE 1

| Amino acid | Three-letter abbreviation | One-letter symbol |
|---|---|---|
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamic acid | Glu | E |
| Glutamine or glutamic acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

## II. Abbreviations and Definitions

### A. Abbreviations

The following abbreviations are used throughout the specification and claims:

HBV - hepatitis B virus

HBsAg - the hepatitis B virus surface antigen protein. In the early literature HBsAg referred only to the S region of the protein. Now, however, that term is used to refer to all three sizes of HBsAg; that is, the S region (the major polypeptide designated p25), S and pre-S2 regions (p33) and S and pre-S1 and S2 regions (p39). Amino acid residue positions of the pre-S region of HBsAg are numbered to the carboxy terminus and such that position 120 is the first position in the pre-S2 region polypeptide of all subtypes of HBV. The numbering of positions begins again from amino-terminus to carboxy-terminus in the S region polypeptide with position 1 designated as the amino-terminal residue.

HBsAg/p25, HBsAg/p33, HBsAg/p39 - a particle containing HBsAg polypeptides whose longest sequence is S (HBsAg/p25), S plus pre-S2 (HBsAg/p33) and S plus pre-S1 and S2 (HBsAg/p39) regions, respectively.

/ad, /ay, /adw, /ayw, /adw₂, and /adr -used with HBsAg or polypeptides related thereto indicate the subtype of HBV to which the amino acid sequence corresponds.

P__-__- a polypeptide corresponding to a portion of the pre-S region of HBsAg. The blanks contain the amino- and carboxy-terminal position numbers of the pre-S region to which the amino acid residues in the polypeptide correspond; e.g., p12-21 designates a polypeptide corresponding in amino acid residue sequence to residues 12 through 21 from the amino-terminus of the pre-S region.

B. Definitions

The phrase "operatively linked" as used herein means that the linkage does not interfere with the ability of either of the linked groups to function as described; e.g., to function as a T or B cell epitope.

The phrase "native B cell epitope" as used herein means an epitope on a protein in its native, as compared to denatured, form that induces antibody secretion. In particular, a native B cell epitope of HBsAg is an epitope of the protein that induces antibodies in a host animal infected/immunized with HBV.

The phrase "corresponds" as used herein means that an amino acid residue sequence has the same linear arrangement of the same amino acid residues as the sequence to which it "corresponds." However, it is well-known in the art that substitutions for amino acid residues can be made that are equivalent antigenically and immunogenically. In particular, conservative substitutions such as one hydrophobic or polar residue for another at one of many positions in a polypeptide frequently does not alter the immunogenic/antigenic characteristics of a polypeptide. Further, specific examples of substitutions are discussed in the specification. Thus, the present invention contemplates those sequences identical to the sequence of a subtype of HBsAg and those sequences specifically bound by antibody induced by HBsAg sequences (antigenically equivalent sequences) or that induce antibody that specifically bind to HBsAg sequences (immunogenically equivalent sequences).

The words "polypeptide" and "peptide" are used interchangeably throughout the specification. Although the term "protein" is usually used herein to refer to a large polypeptide, it is also used to refer to smaller polypeptides, particularly the S and pre-S region polypeptides.

The word "antigen" has been used historically to designate an entity that is bound by an antibody, and also to designate the entity that induces the production of the antibody. More current usage limits the meaning of antigen to that entity bound by an antibody, while the word "immunogen" is used for the entity that induces antibody production. Where an entity discussed herein is both immunogenic and antigenic, it will generally be termed an antigen.

The words "secrete" and "produce" are often used interchangeably in the art as to cells from which antibody molecules are obtained. Cells that produce antibodies may, however, not secrete those molecules into their environment. Herein, the antibody molecules are secreted and are obtained from the blood stream (humoral antibody). Nevertheless, antibodies are generally referred to as being "produced" in keeping with the phrase utilized in the art.

The phrase "S (or pre-S1, pre-S2 or pre-S) region polypeptide (or protein)" is used herein, as in the art, to refer to the entire designated region of a subtype of the HBsAg protein or a polypeptide having a corresponding amino acid residue sequence. If reference is made to a portion of any of those regions, that reference is explicit, either by stating, for example, that a portion of the S region is referred to or by explicitly designating the particular portion of the sequence, as by indicating the included amino acid residue positions.

The designation "the 25 kilodalton (kd) polypeptides of HBsAg" or "HBsAg 25 kd polypeptide" herein is meant to indicate the S region polypeptide in the glycosylated or nonglycosylated form sometimes also referred to as p25 and gp28. Similarly, "the 33 kd polypeptide of HBsAg" and "HBsAg 39 kd polypeptide" are used to designate the glycosylated or nonglycosylated forms of those polypeptides, and are also referred to as p33 and p39.

III. Polypeptides

The present invention contemplates a polypeptide of about 6 to about 50 amino acid residues that consist essentially of an amino acid residue sequence that corresponds to a portion of the pre-S region of the HBsAg protein. That portion is located between an amino-terminal and a carboxy-terminal position, respectively selected from the group consisting of 12-21, 16-27, 94-105, 106-117, 120-126, 120-132, 120-140, 128-138, 133-139, 133-140, 133-143, 133-145, 134-139, 135-143, 135-145, 137-143, 137-145 and 140-151 from the amino-terminus of the pre-S region. The polypeptide is free from amino acid residues at the enumerated amino-terminal and carboxy-terminal positions that correspond to contiguous amino acid residues in the linear sequence of HBsAg.

The amino acid residues that comprise the above sequences are listed in Figure 1. In examining that Figure, it will be noted that there are sequences illustrated for six HBsAg subtypes, and that all of the amino acid residues are not the same at a given position.

The before-listed HBsAg pre-S region sequences are intended to include the aligned sequences for all HBV subtypes unless otherwise stated. Thus, a T cell epitope polypeptide corresponding to positions 12-21 includes the sequences, from left to right and in the direction from amino-terminus to carboxy-terminus, represented by the formulas:

MGQNLSTSNP; and
MGTNLSVPNP;

wherein the first-listed sequence is exhibited by the ayw and adyw HBV subtypes, and the second-listed sequence is exhibited by the adw$_2$, adw and adr subtypes.

Another way of writing the two sequences shown above is as follows:

MGQ(T)NLST(V)S(P)NP,

wherein each parenthesized amino acid residue is an alternative to the immediately preceding residue. Additional members of the before-described useful group of polypeptides can be written using parentheses for alternative residues, although alternative residues are not always needed as is the situation with polypeptide 94-105 in which there is an identity of residues among the subtypes.

The before-described polypeptides correspond to T and B cell epitopes exhibited by HBV. As a consequence, a polypeptide that binds to a B cell epitope can be induce secretion of antibodes when linked to a T cell epitope-containing polypeptide or protein. A useful T cell epitope-containing polypeptide can be linked to a HBV B cell epitope polypeptide or another immunogen (B cell epitope) and used to induce secretion of antibodies. These and other uses are discussed in further detail hereinafter.

IV. Immunogens

A. Useful For Inducing Antibody To HBsAg

In another aspect the present invention contemplates composite immunogens of at least 14 amino acid residues, and can contain several hundred residues as is illustrated hereinafter. Preferably, the composite immunogen contains a total of about 20 to about 50 amino acid residues. Such immunogens are useful for inducing antibodies to HBsAg. A composite immunogen of the present invention comprises a first polypeptide (a) having a terminal residue that is operatively linked to a terminal residue of a second polypeptide (b).

Polypeptide (a) consists essentially of a T cell epitope having an amino acid residue sequence that corresponds to a portion of the pre-S region of HBsAg located between an amino-terminal and carboxy-terminal position, respectively, selected from the group consisting of 12-21, 94-105, 106-117, 120-126, 120-132, 120-140 and 140-151 from the amino-terminus of the pre-S region. Polypeptide (b) consists essentially of an amino acid residue sequence of HBsAg that contains a native B cell epitope of HBsAg.

The operatively linked terminal residue of polypeptide (a) and the operatively lined terminal residue of polypeptide (b) correspond to positions separated in the linear HBsAg sequence by at least 10 amino acid residues. Preferably, either the carboxy-or the amino-terminal residue of polypeptide (a) is operatively linked directly to the amino- or carboxy-terminal residue, respectively, of polypeptide (b) by a peptide bond, and the whole polypeptide is synthesized as one sequence, as by solid phase synthesis described hereinafter.

Numerous additional methods of operatively linking polypeptide (a) to polypeptide (b) are known in the art. For example, a dialdehyde such as glutaraldehyde and a reductant such as sodium borohydride can be used to link amino-terminal residues of the two polypeptides. A cysteine residue can be added to a terminal residue of polypeptide (a). Using N-maleimidobenzoyl-N-hydroxy succinimide ester (MBS) following the procedure of Liu et al., (1979) Biochemistry, 18:690, the cysteine residue can be covalently linked to the MBS-reacted amino-terminal residue of polypeptide (b). A water-soluble carbodiimide such as 1-ethyl-3-(3-dimethyl-aminopropyl) carbodiimide can also be utilized to operatively link the two polypeptides.

Polypeptides (a) and (b) can also be separated by a spacing group. Such spacing groups are preferably short polymers of one to about ten amino acid residues. Those residues are not contiguous residues in the HBsAg linear sequence, but can be selected from another part of the HBsAg molecule. Further, nonsense sequences such as poly-glycine can be used.

In a one embodiment polypeptide (b) is a 33 kilodalton HBsAg polypeptide or a 25 kilodalton HBsAg polypeptide. In another embodiment, polypeptide (b) corresponds to a portion of the pre-S region of HBsAg located between an amino-terminal and carboxy-terminal position, respectively, selected from the group consisting of 1-21, 16-27, 32-53, 53-74, 94-105, 94-117, 106-117, 120-140, 120-145, 128-138, 133-139, 133-140, 133-143, 133-145, 135-143, 135-145, 133-151, 137-143 and 153-171. In yet another embodiment polypeptide (b) corresponds to a portion of the S region of HBsAg located between an amino-terminal and carboxy-terminal position, respectively, selected from the group consisting of 110-137, 117-137, 122-137 and 135-155. Table 2, below, illustrates preferred immunogens of this invention.

## TABLE 2

| Polypeptide (b) | | | | Polypeptide (a) | | | |
|---|---|---|---|---|---|---|---|
| | 12–21[a] | 94–105[a] | 106–117[a] | 120–126[a] | 120–132[a] | 120–140[a] | 140–151[a] |
| **HBsAg[b]** | | | | | | | |
| HBsAg/p33 | N,C[c] | N,C | N,C | N,C | N,C | N,C | N,C |
| HBsAg/p25 | N,C | N,C | N,C | N,C | N,C | N,C | N,C |
| **Pre-S[d]** | | | | | | | |
| 1–21 | N,C | N,C | N,C | N,C | N,C | N,C | N,C |
| 16–27 | N | N,C | N,C | N,C | N,C | N,C | N,C |
| 32–53 | N,C | N,C | N,C | N,C | N,C | N,C | N,C |
| 53–74 | N,C | N,C | N,C | N,C | N,C | N,C | N,C |
| 94–105 | N,C | N,C | C | N,C | N,C | N,C | N,C |
| 94–117 | N,C | N,C | N,C | N,C | C | N,C | N,C |
| 106–117 | N,C | N,C | N,C | C | C | N,C | N,C |
| 120–145 | N,C | N,C | N,C | N | N,C | C | N,C |
| 128–138 | N,C | N,C | N,C | N | N | N,C | C |
| 133–139 | N,C | N,C | N,C | N | N | N,C | C |
| 133–140 | N,C | N,C | N,C | N | N | C | C |
| 133–143 | N,C | N,C | N,C | N | N | C | N |
| 133–145 | N,C | N,C | N,C | N | N | C | N |
| 135–143 | N,C | N,C | N,C | N | N | C | N |
| 135–145 | N,C | N,C | N,C | N | N | C | N |
| 137–143 | N,C | N,C | N,C | N,C | N | C | N,C |
| 133–151 | N,C | N,C | N,C | N,C | N | C | N,C |
| 153–171 | N,C | N,C | N,C | N,C | N,C | N,C | N |

$\underline{S}^e$

| 110-137 | N,C | N,C | N,C | N,C | N,C | N,C | N,C |
|---------|-----|-----|-----|-----|-----|-----|-----|
| 117-137 | N,C | N,C | N,C | N,C | N,C | N,C | N,C |
| 122-137 | N,C | N,C | N,C | N,C | N,C | N,C | N,C |
| 135-155 | N,C | N,C | N,C | N,C | N,C | N,C | N,C |

a   Position numbers of polypeptide (a) sequences from the pre-S region of HBsAg.

b   HBsAg polypeptides.

c   N = the indicated polypeptide (b) is linked to the amino$(NH_2)$-terminus of polypeptide (a). C = the indicated polypeptide (b) is linked to the carboxy-terminus of polypeptide (a).

d   Position numbers of polypeptide sequences corresponding to the pre-S region of HBsAg.

e   Position numbers of polypeptide sequences corresponding to the S region of HBsAg.

In addition to operatively linked composite polypeptides described before the genomes for the S, pre-S1 and pre-S2 regions of HBsAg are known, and can be appropriately linked together and expressed from microorganisms such as bacteria like E. coli or yeast like S. cerevisiae, as well as by mammalian cells such as Chinese hamster ovary (CHO) cells to provide a recombinant composite immunogen. For example, Tiollas and co-workers [Michel et al., Proc. Natl. Acad. Sci. USA, (1984) 81:7708] have reported the expression from CHO cells of a recombinant particle containing the pre-S2 and S regions of HBsAge linked together in the order in which those sequences are found in nature.

A recombinant composition HBsAg immunogen can be similarly prepared (expressed) that contains a pre-S1 T cell epitope polypeptide sequence and a B cell epitope polypeptide sequence as are described hereinbefore. The immunogen so expressed can be free from amino acid residue sequences that do not provide desired T cell or B cell stimulation such as those sequences that do not induce protective antibodies where the immunogen is utilized in a vaccine.

Additionally, the recombinant composite immunogen can be engineered to contain more than one desired epitopic sequence such as p12-21 of pre-S1 by simply placing two genomic sequences for the desired epitopic sequence into the recombinant genome used to express the composite immunogen.

Still further, a recombinant composite immunogen can be made to express multiple subtype epitopes by including two genomes for the same sequences that code for subtype variants such as those for positions 120-140 of the ad and ay subtypes.

A recombinant HBsAg composite immunogen contains at least a polypeptide (a) that consists essentially of p12-21 of the pre-S1 region, a polypeptide (b) that is the p25 S region polypeptide, which are operatively linked together by a peptide bond or an amino acid residue. In preferred practice, those two sequences are directly linked through a terminal residue of each sequence by the peptide bond. More preferably, the pre-S p12-21 sequence is linked through its carboxy-terminal residue to the amino-terminal residue of p25.

Additional T cell and B cell epitopic sequences as discussed herein can also be present in a recombinant HBsAg composite immunogen, and can be placed between the p12-21 and p25 polypeptides in the expressed sequence or on either side thereof. Exemplary composite polypeptide immunogens are listed below, and are illustrated from left to right and in the direction from amino-terminus to carboxy-terminus, using the previously described polypeptide sequence position notation, wherein the individual sequences are separated by a virgule (/) for ease of understanding, and the S region polypeptide, p25, is referred to as "S".

1. p12-21/S;
2. S/p12-21;
3. p12-21/p94-117/S;
4. p12-21/S/p94-117;
5. p12-21/p94-117/p120-151/S;
6. p12-32/p120-126/S/p12-21;
7. p32-53/p12-21/S/p12-27;
8. p12-21/p133-145/S;
9. p12-21/p12-21/p94-117/p120-151/S; and
10. S/p120-151/p94-117/p12-21.

An above-described recombinant composite immunogen is prepared using well known techniques of genetic engineering. As noted previously, the amino acid residue sequences for the pre-S and S regions of HBsAg are known as are the genomes that code for those sequences.

Thus, using commercially available gene synthesizing technology, one can synthesize the desired, known genome from commercially available reagents. Vectors specifically designed for cloning a genome to provide quantities useful for subseqent expression of the desired composite immunogen are also commercially available as are vectors specifically designed for expression. Bacteria, yeast and mammalian cells suitable for transfection by the expression vector and subsequent expression of the immunogen are also commercially available.

In addition to Michel et al., Proc. Natl. Acad. Sci. USA, (1984) 81:7708, exemplary disclosures that describe techniques for genetically enginnering a composite immunogen described before can be found in: U.S. Patents No. 4,428,941 to Galibert et al., No. 4,237,224 to Cohen et al.; No. 4,273,875 to Manis; No. 4,431,739 to Riggs; No. 4,363,877 to Goodman et al., Rodriguez & Tait, Recombinant DNA Techniques: An Introduction, The Benjamin/Cummings Publishing Co., Inc. Menlo Park, CA (1983), whose disclosures are incorporated by reference.

## B. Useful For Inducing Antibody To Non-HBsAg B Cell Epitopes

The present invention also contemplates composite immunogens that are useful for inducing antibodies to non-HBsAg B cell epitopes. In such immunogens, a polypeptide corresponding to a portion of the pre-S region of HBsAg containing a T cell epitope is operatively linked to a non-HBsAg B cell epitope. Such composite immunogens are particularly useful when high titer humoral antibody levels are desired. Specifically, antibodies for use in diagnostics and other assays can be induced without the need for binding a B cell epitope to which the antibodies are desired to commonly used carriers such as KLH, tetanus toxoid, bovine serum albumin or olvalbumin.

The polyclonal antibodies thereby induced do not contain antibodies to carrier, but minimal antibody to T cell epitope may be present. Therefore, those antibody preparations are more homogeneous than when a carrier protein is used. While it is preferred to include native T cell epitopes from the protein to which the antibodies are induced in an immunogen used in a vaccine, pre-S region HBsAg T cell epitopes are suitable to stimulate antibody secretion when a high level of circulating antibody is the desired result as compared to protection against subsequent challenge.

In particular, the invention contemplates a composite immunogen comprising a polypeptide operatively linked to a non-HBsAg B cell epitope. The polypeptide consists essentially of an amino acid residue sequence that corresponds to a portion of the pre-S region of HBsAg containing a T cell epitope located between an amino-terminal and carboxy-terminal position, respectively, selected from the group consisting of 12-21, 94-105, 106-117, 120-126, 120-132, 120-140 and 140-151 from the amino-terminus of the pre-S region.

Such immunogens are capable of inducing antibodies to the B cell epitope included in the immunogen. Although many assays utilize denatured protein, it is preferable in some instances to induce antibody that binds to native protein. In those instances, the B cell epitope selected is a native B cell epitope of a protein, preferably a surface -protein of a pathogen.

The non-HBsAg epitope is preferably a polypeptide containing a sequence of about 7 to about 40 residues. Examplary of such B cell epitopes are the polypeptides of the foot-and-mouth virus as disclosed in U.S. Patent No. 4,544,500, and those of the influenza virus disclosed in U.S. patent application Serial No. 527,401 filed August 29, 1983 (see US-A-4625015), both of whose disclosures are incorporated by reference.

The polypeptide containing the T cell epitope can be operatively linked to the HBsAg or non-HBsAg B cell epitope-containing portion of the immunogen in a manner previously discussed.

## C. Methods

The T cell containing polypeptides of this invention described herein are useful to enhance the immunogenicity of an immunogen. Further, pre-S1 region T cell epitopes can be used in a method to mitigate nonresponsiveness to a vaccine against HBV.

In particular, the present invention contemplates a method of enhancing the immunogenicity of an immunogen. The method comprises operatively linking the immunogen to a polypeptide that consists essentially of a T cell epitope having an amino acid residue sequence that corresponds to a portion of the pre-S region of HBsAg located between an amino-terminal and carboxy-terminal position, respectively, selected from the group consisting of 12-21, 94-105, 106-117, 120-126, 120-132, 120-140 and 140-151 from the amino-terminus of the pre-S region.

In another embodiment the present invention contemplates a method that mitigates nonresponsiveness to an HBV vaccine. That method comprises operatively linking an amino acid residue sequence corresponding to a native T cell epitope in the pre-S1 region of HBsAg to the immunogen of the vaccine. Preferably, the native T cell epitope is located at positions 12-21, 94-105 or 106-117 from the amino-terminus of the pre-S region. In another preferred embodiment of the method 39 kd HBsAg polypeptide is included in the immunogen of the vaccine.

Operative linking of the T cell epitope polypeptide to an immunogen described above can be accomplished as discussed previously.

### V. A Pre-S1 Specific T Cell Response Bypassed Nonresponsiveness To The Pre-S2 and S Regions Of HBsAg

#### A. Discussion

The studies described herein represent an examination of the T and B cell immune responses to the pre-S1 region of HBsAg. The results obtained indicated: (1) the pre-S1 region was immunogenic at the T and B cell levels; (2) anti-pre-S1 specific antibody production (secretion) was regulated by H-2-linked genes and can be independent of anti-S and anti-pre-S2 antibody production; (3) immunization with HBsAg/p39 particles containing the pre-S1 region bypassed nonresponsiveness to the S and pre-S2 regions in terms of antibody production; (4) a pre-S1-specific humoral immune response can occur during the course of acute HBV infection in man; (5) two synthetic peptides p32-53 and p94-117 defined murine and human antibody binding sites on the pre-S1 region, p1-21 and p12-32 define additional human antibody binding sites; (6) pre-S1-specific T cells were elicited in S and pre-S2 region nonresponder mice, and provided functional help for S, pre-S2 and pre-S1 specific antibody production; and (7) a T cell recognition site in the pre-S1 region, p12-32 was identified.

Two doses of 2 micrograms (ug) each of HBsAg/p39 were required to elicit a pre-S1-specific antibody response. However, the HBsAg/p39 preparation used contained only 0.052 ug of pre-S1 region protein (polypeptide) per 4 ug of S region protein (77-fold difference). Therefore, a direct comparison between the anti-S and anti-pre-S1 antibody titers was not informative. However, a total dose of 0.052 ug of S region protein was nonimmunogenic in S region low and nonresponder strains, and minimally immunogenic in high responders, whereas, immunization with HBsAg containing 0.052 ug of pre-S1 region protein (polypeptide) elicited both anti-pre-S1 and anti-S antibody production in S region nonresponder as well as responder strains.

In the B10.M strain, which lacks T cell recognition of the S and pre-S2 regions, pre-S1-specific T cells are required for anti-S region antibody production. However, in the HBsAg particles used for immunization only 2.5% of the polypeptides contained sequences in the pre-S1 region, yet B10.M mice produced a significant (1:10,240) anti-S region antibody response. This suggests efficient pre-S1-specific T cell helper function. Similarly, pre-S1-specific T cells were primed with 0.1 ug of pre-S1 region protein, whereas, a minimum of 2-4 ug of HBsAg/p25 was required to prime S region-specific T cells; Milich et al., (1985) J. Immunol., 134:4194. Cumulatively, these findings indicated to us that the pre-S1 region was an efficient immunogen in the mouse at the T and B cell level. Furthermore, the identification of pre-S1-specific antibody in the sera of HBV-infected human patients indicated that pre-S1 region immunogenicity was not unique to the murine model.

Comparison of the humoral responses of a panel of H-2-congenic murine strains following immunization with three forms of HBsAg particles containing either p25 alone, p25 and p33, or p25, p33 and p39 showed that anti-S, anti-pre-S2, and anti-pre-S1 antibody productions were independently regulated by H-2 linked genes. This was clearly demonstrated by the identification of an S region nonresponder strain (B10.S) that could nonetheless respond to the pre-S2 region, and an S region, pre-S2 region nonresponder strain (B10.M) that could nonetheless respond to the pre-S1 region. Another finding of interest was that immune response to the one region could influence the responses to the other regions. This observation was also possible because of the existence of variably nonresponsive strains. Immunization of the S and pre-S2 region nonresponder B10.M strain with HBsAg/p39 that contains the pre-S1 region elicited S- and pre-S2-specific antibody responses as well as pre-S1-specific antibody T cell responses.

A similar situation relative to the pre-S2 region in the S region nonresponding B10.S strain was previously observed; Milich et al., (1985) Science, 228:1195.

The fact that antibody production to the three regions of HBsAg is independently H-2 restricted and yet exhibits overlapping regulatory mechanisms suggested the influence of region-specific T cell recognition of HBsAg, which could function to help B cell clones responsive to all regions. This was directly demonstrated by the fact that B10.M strain T cells immunologically primed with HBsAg/p39 recognized only the pre-S1 region, whereas, this strain produces anti-S-, anti-pre-S2- and anti-pre-S1-specific antibodies following HBsAg/p39 immunization. Therefore, it is now clear that pre-S1-specific T cells can provide functional help for B cell clones that recognize S, pre-S2, and pre-S1 region determinants presumably on the p39 polypeptide of HBsAg.

It does appear, however, that qualitative or at least quantitative differences may exist between T cell help towards B cells recognized by T cells versus help for B cells recognizing determinants on a region not recognized by T cells. For example, the B10.M strain was a high responder in terms of anti-pre-S1 antibody production, but a low responder in terms of anti-S and anti-pre-S2 antibody production following HBsAg/p39 immunization. Similarly, following immunization with HBsAg/p33 B10.S mice recognized only the pre-S2 region at the T cell level, and produced more pre-S2 specific antibody than S-specific antibody. Furthermore, the anti-S antibody produced by the B10.S strain was primarily subtype-specific as opposed to the group- and subtype-specific anti-S antibodies produced by strains that recognize the S region at both the B cell and the T cell levels; Milich et al., (1985) Proc. Natl. Acad. Sci. U.S.A., 82:8168.

Several candidate sequences were identified to map T and B cell recognition sites on the pre-S1 region of HBsAg. Immunization with native HBsAg/p39 particles elicited an IgG response specific for the p94-117 sequence of pre-S1 in all B10, H-2 congenic strains studied, and a p32-53-specific response in the B10, B10.M strains. The variable antibody response to the p32-53 sequence further suggests the influence of H-2-linked T cell specificity on antibody specificity since all these strains shared the same B10 genetic backgrounds and presumably the same B cell repertoires.

The only significant difference between murine and human anti-pre-S1 positive sera was the amino-terminal directed responses (p1-21, p12-32) observed in human sera. This specificity has been observed in human HBV patient sera previously by other investigators; Neurath et al., (1985) Nature (London), 315:154. The lack of this specificity in the murine area may be unique to the B10 strain and must be examined in mice of other genetic backgrounds.

At the T cell level, the pre-S1-specific p12-32 sequence appeared to represent a T cell recognition site at least in the SJL/J strain. SJL/J mice primed with HBsAg/p39 demonstrated both HBsAg/p39- and p12-32-specific T cell activation in vitro. Similarly, SJL/J mice primed with p12-32 demonstrated both HBsAg/p39- and p12-32-specific T cell responses in vitro. T cells primed with synthetic peptide p12-32 responded better to HBsAg/p39 than to p12-32 on a molar basis. Assuming the efficiency of antigen (immunogen) processing of a particle as compared to a polypeptide is not responsible, these data suggest that a high percentage of p12-32-primed SJL/J T cells recognize this sequence on HBsAg/p39. Further studies will be required to determine the frequency of these T cells within the pre-S1-specific population, and the distribution of this T cell specificity in other H-2 genotypes.

The cumulative results of the current studies on the immune response to the pre-S regions of HBsAg have important implications in terms of future HBsAg vaccine development. Previous work suggested an unexpectedly low number of T cell recognition sites on HBsAg/p25 considering its particulate nature and the size of the subunit polypeptide (226 amino acids); Milich et al., (1985) J. Immunol., 134:4203. This was reflected in the ability to identify at least two murine H-2 haplotypes (H-2f,s) that were total nonresponders, and one (H-2t4) that was only responsive to subtype determinants following HBsAg/p25 immunization; Milich et al., (1984) J. Exp. Med., 159:41. In contrast, a nonresponding haplotype following HBsAg/p39 immunization has not yet been identified amongst the following H-2 haplotypes studied to date: q, d, s, k, b, p, f, t4.

As discussed above, the lack of genetic nonresponsiveness to HBsAg.p39 can be attributed to an increased complexity of T cell recognition. At the T cell level, the pre-S2 and pre-S1 regions provide additional antigenic heterogeneity to HBsAg/p25, which comprises the current plasma-derived, American vaccine (Heptavax-B, Merck Sharp and Dohme, Philadelphia, PA), Therefore, inclusion of pre-S sequences in HBsAg vaccines can decrease the incidence of genetic nonresponsiveness, and provide for a more immunogenic HBsAg particle based on the enhanced immunogenicity of the pre-S2 region; Milich et al., (1985) Science, 228:1195.

It is important to note that the serum-derived HBsAg/p39 particles studies herein contained lesser amounts of pre-S1 and pre-S2 region proteins in a specific ratio to the major S region protein. Strategies to produce recombinant HBsAg particles for vaccine development should take into account the possibility that drastic changes in the ratio of pre-S to S region protein may affect antigenicity and/or immunogenicity.

The implications of these results with respect to viral clearance are not as clear as they are to vaccine development. Although pre-S-containing polypeptides represent a minor component (1-10%) of protein in serum-derived, HBsAg 22 nanometer (nm) spherical particles, these higher molecular weight polypeptides (HBsAg/p33 and HBsAg/p39) can account for up to 40% of the total protein in the intact virion; Heerman et al., (1984) J. Virol., 52:396. Therefore, the immune response to HBsAg/p39 particles most closely approximates an HBV-specific immune response.

It is clear from the present work and previous studies (Neurath et al., (1984) Science, 224:392, Neurath et al., (1985) Nature (London), 315:154) that pre-S1 and pre-S2-specific, as well as S-specific, antibodies are produced during HBV infection in man. However, production of specific antibodies to the pre-S1, pre-S2 and S regions of HBsAg are not predictive of specific T cell responses since T cell help may derive from a population of T cells limited to recognition of a single region. This is important in the context of the hypothesis that HBV clearance is mediated at the T cell level. For example, a pre-S2-specific helper T cell or cytotoxic T cell response may be required for viral clearance, but not required for anti-pre-S2 antibody production as was observed in the B10.M strain.

It has been reported that a number of HBsAg/p25 vaccine nonresponders that were subsequently infected with HBV not only cleared the infection (i.e. did not become chronic carriers), but also produced an anti-S region antibody response; Walker et al., (1981) Transfusion, 21:601 (Abstract). One possible explanation of this phenomenon is that upon infection with HBV, pre-S-specific T cell provided help for S region-specific B cells resulting in anti-S production by vaccine nonresponders, analogous to the B10.S and B10.M murine models. Since these patients cleared the infection, the role of S region-specific T helper cells in viral clearance remains problematical. However, the requirement for pre-S-specific T cell function needs to be examined.

The recent product of transgenic mice, which express both S and pre-S antigenic determinants (Chisari et al., (1985) Science, 230:1157), should provide a model to study S- and pre-S-specific, cytolytic T cell responses. It is believed that the spectrum of clinical manifestations of HBV infection correlates with the genetic ability of the host to mount an appropriate T cell response to a single or group of viral determinants. It is clear from this study that the envelope polypeptidies of the HBV present an array of T cell determinants that the host can recognize, and the specificity of this recognition process an be influenced by major histocompatibility complex (MHC)-linked genes.

## B. Polypeptide Composition Of The HBsAg Particles Used In This Study

Since HBsAg particles can be variably composed of p25, p33 and p39, it was important to characterize the HBsAg particles utilized with respect to the proportion of these polypeptides. Purified preparations of HBsAg were assayed for S, pre-S2, and pre-S1 antigenicity by solid phase sandwich ELISA described in detail in Section VII C2. Relative antigenicities of three HBsAg preparations, (1) HBsAg/p39 particles that contained pre-S1, pre-S2 and S region polypeptides; (2) HBsAg/p33 particles that contained pre-S2 and S region polypeptides; and (3) HBsAg/p25 particles that contained S region polypeptide only, are illustrated in Figure 2.

By comparison with a standard HBsAg preparation of known polypeptide composition, the approximate percentage of molecules possessing a given region could be calculated. For example, the HBsAg preparations shown in Figure 2 had the following compositions: for HBsAg/p39- pre-S1 5.1%, pre-S2 6.5%, S 100%; with respect to HBsAg/p33, pre-S1 0%, pre-S2 38.5%, S 100%; and for HBsAg/p25-pre-S1 0%, pre-S2 0%, S 100%. Stated differently using the HBsAg/p39 as an example, all of the molecules of the particles contained S polypeptide antigen, while 6.5% contained pre-S2 polypeptide, and only 5.1% also contained pre-S1 polypeptide antigen.

## C. In Vivo Antibody Production Following Immunization With HBsAg/p39

Groups of five mice each from a panel of H-2 cogenic strains were immunized and boosted with an HBsAg/p39 preparation representing a dose of 2.0 ug of S region polypeptide, 0.04 ug of pre-S2 region polypeptide and 0.026 ug of pre-S1 region polypeptide as described in Section VII B. Two weeks following the boost, sera were pooled and tested for reactivity against a panel of antigens as illustrated in Table 3.

## TABLE 3

### The Humoral Response Following Immunization
### With HBsAg/p39

| Strain | H-2 | Antibody Titer (1/dilution) | | | | |
|--------|-----|----------|----------|----------|----------|----------|
| | | | | Solid Phase-Bound Antigen | | |
| | | HBsAg/p39 | HBsAg/p33 | HBsAg/p25 | Pre-S2 Peptide | Pre-S1 Peptide |
| B10.D2 | d | 81,920 | 40,960 | 81,920 | 5.120 | 640 |
| B10 | b | 20,480 | 40,960 | 20,480 | 40,960 | 10,240 |
| B10.BR | k | 5,120 | 5,120 | 5,120 | 1,280 | 2,560 |
| B10.S | s | 10,240 | 10,240 | 5,120 | 10,240 | 1,280 |
| B10.M | f | 10,240 | 2,560 | 10,240 | 1,280 | 10,240 |

[a] Pre-S2 peptide, p120-145 from the ayw sequence of the pre-S of region HBsAg.

[b] Pre-S1 peptide, p94-117 from the adw$_2$ sequence of the pre-S region of HBsAg.

Significantly, all strains produced antibodies specific for S, pre-S1, and pre-S2 determinants even though pre-S polypeptide was present in only trace amounts compared to the amount of S region polypeptide. This is also noteworthy since the B10.S and B10.M strains were nonresponsive to the S region following HBsAg/p25 immunization; Milich et al., (1984) J. Exp. Med., 159:41. The B10.M strain was also nonresponsive to the pre-S2 region following HBsAg/p33 immunization; Milich et al., (1985) Science, 228:1195. This suggested that the immune response to the pre-S1 region can influence the antibody responses to other regions. That the pre-S2 region could positively influence the responses to S region determinants was previously demonstrated; Milich et al., (1985) Proc. Natl. Acad. Sci. U.S.A., 82:8168, Milich et al., (1985) Science, 228:1195.

The hierarchy of response status to the S region upon immunization with HBsAg/p25 is: B10.D2 greater than B10 greater than B10.BR and B10.S and B10.M strains are nonresponders; Milich et al., (1984) J. Exp. Med., 159:41. However, following HBsAg/p39 immunization this hierarchy, which was based on at least a 4-fold difference (significant) between strains, changed to B10.D2 greater than B10, greater than B10.M, B10.BR, and B10.S as analyzed on HBsAg/p25. When the antibody responses to the pre-S2 region following HBsAg/p39 immunization were examined utilizing a pre-S2 specific synthetic polypeptide, p120-145, as the solid phase-bound ligand, another pattern was evident: B10 greater than B10.S and B10.D2 greater than B10.BR and B10.M. Finally, when the antibody responses specific for the pre-S1 region following HBsAg/p39 immunization were categorized, a third pattern emerged: B10.M and B10 greater than B10.BR and B10.S greater than B10.D2, as illustrated in Table 3 before. Since these strains are H-2 congenic, the data indicate

17

that the three antibody responses to S, pre-S2, and pre-S1 region polypeptides can be independently regulated by H-2 linked genes. This predicts that T helper (Th) cells can recognize unique determinants on each region, and those Th cells are independently H-2 restricted.

The existence of distinct regulation of the immune responses to S, pre-S2 and pre-S1 region determinants becomes more clear when the secondary responses following HBsAg/p39 immunization are directly compared to the secondary responses following HBsAg/p25 and HBsAg/p33 immunizations in a panel of H-2 cogenic mice as shown in Table 4, below.

## TABLE 4

### Influence Of H-2 Genotype On The
### Humoral Response To HBsAg
### Particles Of Varied Composition

| Immunogen | Strain | H-2 | Specific Antibody Titer To: (1/dilution) | | |
|---|---|---|---|---|---|
| | | | S | pre-S2 | pre-S1 |
| HBsAg/p25 | B10.D2 | d | 81,920 | 0 | 0 |
| | B10 | b | 20,480 | 0 | 0 |
| | B10.BR | k | 5,120 | 0 | 0 |
| | B10.S | s | 0 | 0 | 0 |
| | B10.M | f | 0 | 0 | 0 |
| HBsAg/p33 | B10.D2 | d | 40,960 | 10,240 | 0 |
| | B10 | b | 10,240 | 40,960 | 0 |
| | B10.BR | k | 1,280 | 2,560 | 0 |
| | B10.S | s | 640[a] | 10,240 | 0 |
| | B10.M | f | 0 | 0 | 0 |
| HBsAg/p39 | B10.D2 | d | 81,920 | 5,120 | 640 |
| | B10 | b | 20,480 | 40,960 | 10,240 |
| | B10.BR | k | 5,120 | 1,280 | 2,560 |
| | B10.S | s | 5,120 | 10,240 | 1,280 |
| | B10.M | f | 10,240 | 1,280 | 10,240 |

[a] The underscored numbers represent an antibody response to a specific region of HBsAg that was not observed when the strain was immunized with that same antigen; i.e., B10.S was nonresponsive to the S region when immunized with HBsAg/p25.

The above data illustrate that immunization with HBsAg/p25 elicited anti-S region responses in all strains except B10.S and B10.M. Immunization with HBsAg/p33 elicited anti-pre-S2 responses in all strains except the B10.M strain. Note that it also elicited an anti-S response in the S region nonresponder, B10.S strain, indicated by the underscored number in Table 4.

The anti-S response of the B10.S strain resulted from pre-S2-specific T cell help for S-specific B cell clones; Milich et al., (1985) Proc. Natl. Acad. Sci. U.S.A., 82:8168, Milich et al., (1985) Science, 228:1195. Therefore, HBsAg/p33 immunization provided an additional T cell recognition site(s) for B10.S mice.

The availability of a nonresponder strain to both S and pre-S2 region determinants (B10.M) allowed the determination of whether the pre-S1 region provided alternative T cell recognition sites that the B10.M was able to recognize, and if so, whether recognition of pre-S1 at the T cell level influenced the B10.M antibody response to the pre-S2 and S regions. HBsAg/p39 immunization elicited anti-pre-S1-specific responses in all strains, and furthermore, elicited anti-S and anti-pre-S2 responses in all strains including an S-specific response in the nonresponder B10.S strain and S- and pre-S2-specific responses in the nonresponder B10.M strain, as indicted by the underscored numbers in Table 4. This result, suggests that the B10.M strain possessed pre-S1-specific T cells that were capable of helping B cell clones specific for S and pre-S2 region determinants as well as pre-S1-specific B cell clones.

D. Fine Specificity Of The Murine Pre-S1 Specific Humoral Response

The synthetic peptide utilized above in Section C and described in Table 3 (p94-117) as a pre-S1-specific ligand was chosen based on the observation that only synthetic peptide p94-117 bound antibody from all the strains immunized with HBsAg/p39 as illustrated in Table 5, below.

0 250 253

## TABLE 5

### Fine Specificity Of The Antibody Response
### To The Pre-S1 Region Of The HBsAg/p39

| Strain | Antibody Titer (1/dilution) | | | | |
|---|---|---|---|---|---|
| | Pre-S1 Synthetic Polypeptide Sequence (adw) | | | | |
| | p1-21 | p12-32 | p32-53 | p53-74 | p94-117 |
| **HBsAg/p39 as immunogen** | | | | | |
| B10.D2 | 0 | 0 | 2,560 | 0 | 640 |
| B10 | 0 | 0 | 160 | 0 | 10,240 |
| B10.BR | 0 | 0 | 0 | 0 | 2,560 |
| B10.S | 0 | 0 | 0 | 0 | 1,280 |
| B10.M | 0 | 0 | 640 | 0 | 10,240 |
| **HBsAg/p25 as immunogen** | | | | | |
| B10.D2 | 0 | 0 | 0 | 0 | 0 |
| **HBsAg/p33 as immunogen** | | | | | |
| B10.D2 | 0 | 0 | 0 | 0 | 0 |

The data of Table 5 also show that the B10.D2, B10, and B10.M strains produced an antibody that reacted with p32-53 of a pre-S1 region. Mice immunized with HBsAg particles not bearing a pre-S1 region polypeptide

21

produced no antibody reactive with the panel of pre-S1-specific peptides as exemplified by the B10.D2 responses to immunization with HBsAg/p25 and HBsAg/p33.

SJL mice were immunized with 100 ug of p12-32 per mouse in another study. The in vitro T cell proliferative response to p12-32, p1-21, two preparations of HBsAg/p39, as well as HBsAg/p33 and HBsAg/p25 were determined. As illustrated in Figure 9, p12-32, p1-21 and both HBsAg/p39 preparations stimulated T cell proliferation, while HBsAg/p33 and HBsAg/p25 did not stimulate proliferative responses. Since p12-32 and p1-21 both stimulated T cell response, the T cell epitope was localized to p12-21, the residues contained in both peptides.

Humoral antibody induced by p12-32 was analyzed by ELISA for reactivity with p12-32 and p1-21. As illustrated in the insert bar graph of Figure 9, high titer anti-p12-32 and no antibody to p1-21 was present. Therefore p12-21 does not contain a complete B cell epitope recognized by that murine strain. Polypeptide p12-32 contained a B cell epitope. That epitope is p16-27.

Table 6, below, illustrates that pre-S1 synthetic polypeptides primed in vivo antibody production to the pre-S1, pre-S2 and S regions of HBsAg.

## TABLE 6

### Pre-S1 Synthetic Polypeptides Primed In Vivo Production To The Pre-S1, Pre-S2 And S Regions Of HBsAg

|  |  | Antibody Titer To: (1/Dilution) |  |  |  |  |
|  |  | S | Pre-S2 | Pre-S1 Epitopes | | |
| Immunogen[a] | Time[b] | HBsAg/p25 | p120-145 | p12-32 | p32-53 | p94-117 |
| 0 | 1 | 0 | 0 | 0 | 40 | 0 |
| HBsAg/p39 | 3 | 1280 | 640 | 0 | 40 | 0 |
| p12-32 | 1 | 0 | 0 | 640 | 0 | 0 |
| 0 | 3 | 0 | 0 | 640 | 0 | 0 |
| p12-32 | 1 | 80[c] | 2560 | 2560 | 40 | 0 |
| HBsAg/p39 | 3 | 10,240 | 2560 | 5120 | .40 | 40 |
| p94-117 | 1 | 0 | 0 | 0 | 0 | 640 |
| 0 | 3 | 0 | 0 | 0 | 0 | 640 |
| p94-117 | 1 | 40 | 640 | 0 | 2560 | 20,480 |
| HBsAg/p39 | 3 | 1280 | 5120 | 0 | 1280 | 10,240 |

aGroups of 4 mice were primed with 100 ug of the indicated peptides or no immunogen (0) in CFA, and boosted 4 weeks later with 1 ug of HBsAg/p39 or no immunogen (0) in incomplete Freud's adjuvant as shown.

bWeeks after boost.

cThe underlined numbers represent at least a 4-fold difference from the unprimed control group.

The data of the above Table demonstrates that mice primed with p12-32 produce antibody to pre-S1, pre-S2 and S polypeptide regions. However, while mice primed with p94-117 produced significantly increased antibodies to pre-S2 and pre-S1 region polypeptides, at three weeks following secondary (booster) immunization with HBsAg/p39, the mice produced antibody of the same titer to the S region as unprimed controls. Therefore, p94-117 did not prime for S region antibody production in SJL strain mice.

In addition, use of p1-21, and p12-21 and p12-32 in T cell proliferation studies showed comparable results regardless of which of the three polypeptides was used. Those results from the proliferation studies as well as the results discussed before demonstrate the presence of a T cell epitope at positions 12 through 21 from the amino-terminus of the HBsAg pre-S1 region at both the helper and proliferative function levels.

E. Fine Specificity Of The Human Pre-S1 Specific Humoral Response During Acute HBV Infection

Sera from seven acute HBV patients collected during or soon after clinical illness and sera from four normal human (NHS) donors (donors who had not been infected with HBV) were examined for the presence of pre-S1-specific, IgG antibody. The pre-S1 syntghetic polypeptides used to analyze the specificity of the murine response were utilized as solid phase-bound antigens. Those data are illustrated in Table 7 below.

23

## Table 7

### Fine Specificity Of The Human Pre-S1-specific
### Humoral Response During Acute HBV Infection

| Patient No. | Antibody $(OD_{490}$ units)[a] Pre-S1 Synthetic Peptide Sequence (adw) | | | | |
|---|---|---|---|---|---|
| | p1-21 | p12-32 | p32-53 | p53-74 | p94-117 |
| 2673 | 0.03 | 0.06 | 0.09 | 0.02 | 0.40[b] |
| 2674 | 0.06 | 0.16 | 0.26 | 0.08 | 0.95 |
| 2681 | 0.04 | 1.50 | 0.52 | 0.07 | 0.50 |
| 2686 | 2.00 | 1.50 | 1.20 | 0.02 | 0.70 |
| 2689 | 0.40 | 0.34 | 0.22 | 0.18 | 0.10 |
| 2690 | 0.08 | 0.30 | 0.18 | 0.03 | 0.03 |
| 2693 | 0.29 | 0.06 | 0.08 | 0.05 | 0.07 |
| NHS | 0.07 | 0.03 | 0.03 | 0.01 | 0.03 |
| NHS | 0.07 | 0.02 | 0.04 | 0.04 | 0.03 |
| NHS | 0.03 | 0.03 | 0.04 | 0.02 | 0.04 |
| NHS | 0.03 | 0.03 | 0.02 | 0.02 | 0.03 |

a 1:50 dilutions of human sera from patients with acute hepatitis B infection (numbers) or normal persons (NHS) who have not been exposed to HBV were incubated (admixed and maintained) with indicated solid phase-bound polypeptides as antigens, and probed with enzyme-labelled monoclonal anti-human IgG. Control values ranged from 0.01-0.08.

b Underscored readings are at least 4-fold (significant) or greater than the reading on control wells not coated with polypeptide.

As illustrated in Table 7, all the acute HBV sera assayed contained antibodies specific for at least one of the pre-S1 polypeptides, whereas, all four normal human sera (NHS) were negative. Similar to HBsAg/p39-immunized murine antisera, a synthetic polypeptide containing pre-S residues 94-117 (p94-117) and p32-53 represented native human antibody binding sites, whereas, p53-74 was only recognized by one patient serum. Unlike the murine response (using B10 as background), four of seven human acute HBV sera bound the amino-terminal sequence p1-21 and four bound p12-32. The production of p12-32-specific antibodies in acute phase serum has previously been reported; Neurath et al., (1985) Nature (London), 315:154.

It is clear from these data that the human anti-pre-S1 response can include multiple specificities. These results confirm the occurrence of a pre-S1-specific humoral reponse during the course of an acute HBV infection in man.

## F. The Specificity Of The T Cell Proliferative Responses To HBsAg/p39 Was Dependent On The Genotype Of The Responding Strain

To directly examine the T cell response to HBsAg/p39, and to confirm the hypothesis that following HBsAg/p39 immunization pre-S1-specific Th cells could bypass nonresponse to the S and pre-S2 regions, Balb/c (H-2d), SJL/J (H-2s) and B10.M (H-2f) mice were immunized with HBsAg/p39. The T cell proliferative responses specific for HBsAg/p25, HBsAg/p33 and HBsAg/p39 were determined. Since eliciting a pre-S1-specific T cell response was desired, HBsAg/p39 particles containing a greater quantity of pre-S1 than the particles used in the antibody studies discussed in Section V C, above, were utilized (i.e. 5.1% vs. 2.5% pre-S1). The antibody response to HBsAg/p39 immunization in the Balb/c strain was equivalent to the H-2-identical B10.D2 strain. The SJL/J strain antibody response was similar to the H-2-identical B10.S response.

These H-2 haplotypes wre chosen because they represented three distinct patterns of antibody production in terms of specificity. The Balb/c strain was responsive to the S, pre-S2 and pre-S1 polypeptide regions upon immunization with the appropriate HBsAg particle. The SJL/J strain was nonresponsive to the S region when immunized with HBsAg/p25, but was reponsive to the pre-S2 and pre-S1 regions. The B10.M strain was nonresponsive to the S and pre-S2 regions upon immunization with HBsAg/p25 and HBsAg/p33, and only responded to HBsAg/p39 immunization as demonstrated in Table 4, before.

The Balb/c T cell proliferative response following HBsAg/p39 immunization is shown in Figure 3. HBsAg/p39-primed T cells were activated by HBsAg/p25, HBsAg/p33 and HBsAg/p39 particles in vitro. The dose response curves indicated that S region- and pre-S2 region-specific T cells are present in the Balb/c strain. However, the almost equivalent stimulation induced with HBsAg/p33 and HBsAg/p39 make it difficult to interpret the contribution of pre-S1-specific T cells in the proliferative response of this strain.

In contrast, the SJL/J strain clearly demonstrated a pre-S2-specific response since HBsAg/p25 was nonstimulatory, whereas HBsAg/p33 induced a proliferative response. Those results are illustrated in Figure 4. The additive effect observed when HBsAg/p39 was the challenge antigen clearly indicated a pre-S1-specific T cell population was also present.

Therefore, H-2s-bearing strains were nonresponsive to the S region at the T cell level whether the immunogen was HBsAg/p25, HBsAg/p33 (Milich et al., (1985) Proc. Natl. Acad. Sci. U.S.A., 82:8168), or HBsAg/p39 (Figure 4). Yet that strain produced anti-S antibodies following HBsAg/p33 or HBsAg/p39 immunization, as shown in Table 4, before. Following HBsAg/p39 immunization in the B10.M strain, only HBsAg/p39 induced a proliferative T cell response in vitro, as illustrated in Figure 5, indicating an exclusive pre-S1-specific response.

Therefore, H-2f-bearing strains were nonresponsive to the S and pre-S2 region polypeptides at the T cell level whether priming antigen was HBsAg/p25 (Milich et al., (1985) J. Immunol., 134:4194), HBsAg/p33 (Milich et al., (1985) Proc. Natl. Acad. Sci. U.S.A., 82:8168) or HBsAg/p39, as shown in Figure 5. Yet that strain produced anti-S and anti-pre-S2 antibody following HBsAg/p39 immunization as shown in Table 4. This indicates that in the B10.M strain all antibody specificities produced following HBsAg/p39 immunization resulted from pre-S1-specific T cell helper function.

To demonstrate that the pre-S1 specific T cell proliferative response of the B10.M strain was not unique to the immunogen, HBsAg/p39-primed T cells were challenged in vitro with five separate HBsAg/p39 particle preparations (three of the ad; and two of the ay subtype), possessing varying quantities of p39, as illustrated in Figure 6. All HBsAg/p39 preparations regardless of subtype induced a proliferative response that correlated with the amount of p39 present in the HBsAg preparation. This indicated the presence of a group-specific T cell response in the B10.M strain, but does not preclude the existence of a subtype-specific T cell population.

## G. Definition Of A T Cell Recognition Site On The Pre-S1 Region of HBsAg/p39

To examine T cell fine specificity for pre-S1 region polypeptide determinants, HBsAg/p39-primed SJL/J T cells were challenged with pre-S1 region synthetic peptides as shown in Figure 7. All the synthetic polypeptides induced proliferative responses at relatively high concentrations [50-200 micrograms per milliliter (ug/ml)]. Pre-S2 and S region-specific synthetic polypeptides did not elicit a response. Only p12-32 induced a significant, although low, proliferative response through the entire dose response curve.

For this reason, SJL/J mice were immunized with 100 ug of p12-32 in complete Freund's adjuvant (CFA). The in vitro proliferative T cell responses elicited with p12-32, two preparations of HBsAg/p39 and HBsAg/p33 were determined.

As shown in Figure 8, p12-32-primed T cells proliferated in response to the homologous polypeptide and both the HBsAg/p39 preparations, but not to HBsAg/p33, which lacks the pre-S1 region. On a weight basis, the synthetic peptide elicited a slightly greater T cell proliferative response. However, on a molar basis HBsAg/p39 was superior; i.e., to elicit 20,000 CPM, 0.03 micromoles (uM) of HBsAg/p39 and 0.18 uM of p12-32 were required. Therefore, it appears that in SJL/J mice the synthetic polypeptide p12-32 primed T cells capable of recognizing the homologous pre-S1-specific sequence on the presumably processed p39 polypeptide.

## VI. A Synthetic Polypeptide Corresponding to Portion of HBsAg Pre-S2 Region Contained Nonoverlapping T and B Cell Determinants and Distinct Overlapping B Cell Determinants

## A. Nonoverlapping T and B Cell Determinants

T cell recognition of a HBsAg, pre-S2 region synthetic polypeptide (p120-145) previously shown to be immunogenic (Neurath et al., (1984) Science, 224:392), and to represent a dominant antibody binding site on the native pre-S2 region [Milich et al., (1985) Science, 228:392] was examined. The results of this study indicated the following: (1) the anti-p120-145 response was regulated by H-2-linked genes, but differed from the pattern of H-2 restriction observed for the native anti-pre-S2 response; (2) nonoverlapping, dominant T cell and B cell recognition sites were identified on the synthetic peptide p120-145; (3) the fine specificity of T cell recognition of p120-145 was defined by a single amino acid substitution; (4) the synthetic peptide containing both T and B cell determinants was highly immunogenic in responder strains, whereas, separate T cell or B cell peptide determinants were minimally immunogenic; and (5) the synthetic T cell recognition site primed T cell help for antibody production to the synthetic B cell site, which was cross-reactive with the native pre-S2 region.

Previous work demonstrated the influence of H-2-linked genes on antibody production and the T cell proliferative response to the native pre-S2 region of HBsAg; Milich et al., (1985) Science, 228:392, Milich et al., (1985) Proc. Natl. Acad.-Sci. U.S.A., 82:8168. The rank order of responsiveness at the antibody level correlated with the T cell proliferative responses and was as follows: B10 greater than B10.D2 greater than B10.S greater than B10.BR greater than B10.M (nonresponder). Since antibody specific for the native pre-S2 region and the synthetic polypeptide were highly cross-reactive as described in Milich et al., (1985) Science, 228:392, and hereinafter, it was possible to compare the influence of H-2-linked genes on in vitro antibody production to the native pre-S2 region versus the synthetic polypeptide p120-145.

Although the pre-S2 region exists on a larger polypeptide (p33), which includes S region polypeptide antigenic determinants, and in the context of HBsAg particles, the pre-S2-specific antibody and T cell responses have been shown to be of greater magnitude, preceded in time, occurred at a lower HBsAg dose and were independently regulated as opposed to the S region-specific responses [Milich et al., (1985) Science, 228:392, Milich et al., (1985) Proc. Natl. Acad. Sci. U.S.A., 82:8168.] Therefore, genetic and other influences of the immune response to the S region on the immune response to the pre-S2 region can be discriminated and/or negated.

The rank order of responsiveness to p120-145 following immunization with the unconjugated, homologous synthetic polypeptide p120-145: B10.BR greater than B10 and B10.S greater than B10.D2 and B10.M (nonresponders) was clearly different from that observed following immunization with pre-S2 region-containing particles (HBsAg/p33). For example, the B10.D2 strain produced anti-p120-145 antibodies following immunization with HBsAg/p33, but was totally nonresponsive when immunized with p120-145. Since the B10.D2 and Balb/c (both H-2d) strains were very responsive at the T cell level to the native pre-S2 region (Milich et al., (1985) Proc. Natl. Acad. Sci. U.S.A., 82:8168) but not to p120-145, it is apparent that p120-145 does not represent the T cell determinant on the pre-S2 region recognized by H-2d-bearing strains. Conversely, the B10.BR strain was a low responder when immunized with the intact pre-S2 region, and a high responder following p120-145 immunization. This finding suggests that suppressor-activating determinants may exist elsewhere on the native protein, which down regulate the p120-145-specific response.

Suppressor mechanisms have been implicated in the low responder status of the B10.BR strain following HBsAg/p25 immunization; Milich et al., (1985) J. Immunol., 134:4194, Milich et al., (1984) J. Exp. Med., 159:41. These results indicate that the immunogenicity of a constituent polypeptide, even though that polypeptide represents a dominant antibody binding site on the native protein, was not necessarily predictive of the immunogenicity of the intact molecule and vise versa. Furthermore, immunogenicity of a carrier-free polypeptide was critically dependent on T cell helper function, which is regulated by H-2 linked genes.

Since the pattern of H-2 restriction was different following peptide or HBsAg/p33 immunization, it is clear that T cell recognition sites on p120-145 need not be identical to those recognized when the native pre-S2 region was the immunogen. This was more directly demonstrated by the observation that p120-145-primed T cells from most strains did not recognize the native pre-S2 region upon in vitro challenge, and did not prime an in vivo anti-pre-S2 antibody response following a boost with HBsAg/p33. This was true even in the C3H.Q strain, which is a high responder in terms of anti-p120-145 antibody production whether immunized with p120-145 or HBsAg/p33. Therefore, a constituent peptide can represent an antibody binding site on the native protein, but lack a T cell recognition site relevant to the native protein.

Although the 120-145 sequence of the pre-S2 region does not appear to possess a T cell recognition site relevant to the native protein in most mouse strains, a dominant T cell determinant, relevant to p120-145 immunization, was identified on the amino-terminus of p120-145 (p120-132). This T cell determinant did not overlap with the dominant antibody (B cell) binding site on the carboxy-terminus (p133-145) of p120-145. In the C3H.Q strain, the p120-132 sequence represented the dominant T cell recognition site on p120-145 as shown by its ability to activate p120-145-primed T cells approximately as well as the immunogen, and by the ability of p120-132 to prime T cell help for antibody production to the p133-145 sequence.

The fine specificity of T cell recognition of p120-132 was further refined by examining the influence of subtype. The ayw and adw2 subtypes differ by a single amino acid substitution at residue 126 in the 120-132 sequence. Nevertheless, p120-132/ayw-specific T cells were virtually non-cross-reactive on p120-132/adw2. Reciprocally, p120-132/adw2-specific T cells were minimally cross-reactive with p120-132/ayw.

The trimolecular complex model of T cell recognition proposed by Heber-Katz et al., (1983) J. Mol. Cell. Immunol, 1:3 suggests that class II-encoded Ia moleculess and antigen physically interact at one subsite on the antigen (agretope) and another subsite on the antigen contacts the T cell receptor (epitope). With respect to the peptide antigen p120-132, a single amino acid residue substitution at residue 126 drastically reduced T cell activation. Furthermore, immunization with the substituted polypeptide elicited a comparable T cell response specific for the substitution.

In the context of the trimolecular model, these results were consistent with residue 126 representing a contact site with the T cell receptor (epitope); Schwartz et al., (1985) J. Immunol., 135:2598. The fact that both polypeptides were equivalently immunogenic in the C3H.Q(H-2q) strain, regardless of the nonconservative threonine to alanine substitution, suggests that the agretope would reside in a shared sequence outside residue 126. If this were true, it may be predicted that the two peptides should compete for the same Ia-binding site (Werdelin, (1982) J. Immunol., 129:1883, Rock et al., (1983) J. Exp. Med., 157:1618) and p120-132/adw2 would inhibit the activation of p120-132/ayw-primed T cells elicited by p120-132/ayw. However, no such inhibition occurred, suggesting residue 126 may be involved in the interaction with Ia; i.e., an agretope.

In this case, it would have to be assumed that both substitutions at 126 are permissible in the context of Iaq (i.e. immunogenic), and that threonine vs. alanine-Iaq interactions variably influence the trimolecular complex to the extent that at least two noncross-reactive T cell clones would be generated to a single primary sequence (epitope).

Since agretope inhibition studies have not been universally successful (Schwartz, (1985) Ann. Rev. Immunol., 3:237), the interpretation that residue 126 represented a contact site with the T cell receptor is favored by the present inventors and their coworkers. However further studies with truncated and substituted synthetic peptides in a series of B10, H-2 congenic strains will be required to resolve this question.

Examination of the T cell proliferative response to a third subtype sequence, p120-132/adw, which differs from the ayw sequence at residues 126, 127, 130, 132, and from the adw2 sequence at residues 127, 130 and 132, reiterated the importance of residue 126 and revealed that residues 127, 130 and 132 were not critical to T cell recognition. Therefore, a dominant T cell determinant on p120-145 was tentatively localized to residues 120-126.

Having identified a dominant site on p120-145 recognized by T cells of the C3H.Q and other strains, it was of interest to examine the specificity of antibodies produced following p120-145 immunization in C3H.Q mice. In contrast to T cell recognition, the majority of antibodies specific for p120-145 bound to the carboxy-terminal sequence p133-145, and demonstrated a high degree of cross-reactivity for the adw2 sequence. Furthermore, a large proportion of anti-p120-145 antibodies cross-reacted with native HBsAg/p33 unlike p120-145-primed T cells. Additionally, antibodies raised to the p120-132 sequence appeared to recognize distinct regions of the polypeptide as compared to T cell recognition. These results indicate that T and B cell recognition sites can be distinct even on a synthetic peptide as small as 13 residues.

Since the pre-S2 and S regions exist on the same polypeptide, the use of a single composite immunogen containing both T and B cell epitopes is exemplified and such a strategy has the advantage that a T cell response to the pre-S2 region circumvents nonresponsiveness to the S region in S region nonresponder mice; Milich et al., (1985) Proc. Natl. Acad. Sci. U.S.A., 82:8168. Since the synthetic T cell determinant p120-132 was capable of priming T cell help for antibody production to the synthetic B cell determinant p133-145, and this antibody reacted with the native pre-S2 region, this system provided evidence that synthetic T and B cell recognition sites can be combined to yield a functional, composite immunogen useful in a vaccine or other inoculum.

Due to the MHC-dependent restrictions imposed on T cell recognition of antigen, several T cell recognition sites or a combination of free and conjugated polypeptide are to ensure responsiveness in a high percentage of vaccine recipients. In this regard, the p120-132 sequence served as a T cell recognition site in mice bearing the H-2q,s,k haplotypes but not the H-2b haplotype.

An ideal synthetic immunogen is composed of T and B cell recognition sites identical to those recognized on the native molecule. Such an immunogen induces protective antibodies and elicits T and B cell memory relevant to the pathogen in the eventuality of a subsequent infection after a protective antibody level wanes. A synthetic T cell determinant also obviates the requirement to conjugate synthetic B cell sites to a heterologous protein carrier such as keyhole limpet hemocyanin or bovine serum albumin, although such carriers for both B and T cell epitopes can still be used. While p120-145 may not represent the ideal synthetic pre-S2 region immunogen in mice, it does represent a completely synthetic and functional immunogen, similar to a peptide-carrier conjugate, that does not require the heterologous protein carrier to elicit T cell helper function.

These results and the identification of T cell recognition sites within the S [Milich et al., (1985) J. Immunol., 134:4203] and pre-S1 regions of native HBsAg (as described hereinbefore) increase the feasibility of a completely synthetic HBV vaccine.

## 1. Summary

T cell recognition of an HBsAg pre-S2 region synthetic polypeptide, p120-145, in terms of fine specificity, H-2-linked genetic influences, comparison to antibody binding, and relevance to T cell recognition of the native protein was examined. It was demonstrated that the immune response to the synthetic peptide was regulated by H-2-linked genes, but the pattern of H-2 restriction differed from that observed for the native anti-pre-S2 response. Dominant and nonoverlapping T cell and B cell recognition sites were identified on the synthetic polypeptide p120-145. T cell recognition was focussed on the amino-terminal sequence (120-132) and antibody (B cell) recognition was focussed on the carboxy-terminal sequence (133-145). The fine specificity of T cell recognition of p120-145 was defined by a single, subtype-dependent, amino acid substitution. The fine specificity of B cell recognition is described hereinafter.

With respect to the immunogenicity of p120-145, the synthetic peptide containing both T and B cell determinants was highly immunogenic in responder strains, whereas, separate T cell or B cell peptide determinants were minimally immunogenic. Furthermore, the synthetic T cell recognition site primed T cell help for antibody production to the synthetic B cell site, which was cross-reactive with the native pre-S2 region on HBsAg/p33 particles. This system demonstrated that totally synthetic T cell and B cell recognition sites can be combined to yield an effective immunogen.

B. Overlapping B Cell Determinants

The fine specificity of the humoral response to the pre-S2 region of HBsAg was examined. The results indicated: (1) the murine antibody response to the pre-S2 region was directed primarily to residues 133 to 143; (2) two distinct but overlapping epitopes were identified within these 11 continuous residues; (3) one epitope was group-specific, and one epitope was influenced by a subtype-dependent, single residue substitution and the degree of influence varied with the size of the antigen; (4) the minimum size of both antibody binding sites was seven amino acid residues; (5) the physical and chemical form of the immunogen influenced antibody fine specificity; (6) human antisera recognized pre-S2 region sequences in common with murine antisera; and (7) antibodies with nonoverlapping as well as overlapping fine specificities for the pre-S2 region were indistinguishable by antibody competition analysis.

Utilizing pre-S2-specific monoclonal antibodies and truncated and subtype-specific synthetic polypeptides, two dominant antibody binding sites within 11 continuous residues of the pre-S2 region of HBsAg were mapped. A group-specific epitope (epitope 1) represented by synthetic peptide p133-139 and a subtype-dependent epitope (epitope 2) represented by p137-143 shared three overlapping residues. In the p137-143 sequence a single amino acid substitution at residue 141 of leucine (adw2) for phenylalanine (ayw) distinguished the subtypes. However, the influence of residue 141 was "covert" in that it was only detected on synthetic antigens (polypeptide immunogens) 19 amino acids in length or smaller and not on a synthetic polypeptide of 26 residues of native particles.

This suggests that while residue 141 was involved in the antibody binding site, other residues outside the binding site (i.e., p120-132) can influence the binding site to the extent that leucine or phenylalanine at 141 was not critical. Whether this represented a nonspecific influence of peptide size or was specific for the pre-S2 sequence will be investigated. However, size was implicated since subtype influence became more apparent progressively as polypeptide size decreased from a 26-mer to a 19-mer to a 13-mer and was absolute on a 11-mer or smaller.

In terms of vaccination strategy, this "covert" subtype influence was of little practical significance following immunization with native HBsAg/p33 particles since the predominant response was directed to the group specific epitope p133-140. Similarly, although immunization with synthetic polypeptides p120-145 or p133-151 of the ayw subtype elicited a significant epitope 2-specific response, anti-p137-143 antibodies bound native particles of both subtypes equivalently.

It is also noteworthy that although all murine and numerous human anti-pre-S2-specific antisera demonstrated a predominant response to the p133-143 sequence, human antisera were also identified that reacted preferentially to the p120-132 sequence. The human and murine p120-132-specific antibody responses were not significantly influenced by the subtype-dependent amino acid residue substitution at residue position 126 as described hereinbefore.

The minimum size of epitopes 1 and 2 was seven amino acids. However, polypeptides of 8-11 residues in length demonstrated significantly greater antibody binding. Monoclonal antibodies specific for epitopes 1 and 2 were capable of mutual quantitative inhibition to 100%, which may be expected based on the three amino acid residue overlap of these epitopes. However, antibodies to a nonoverlapping determinant separated by at least 13 residues (amino-terminus of the binding site on p153-171 has not been defined) in the primary sequence quantitatively inhibited to at least 80% the binding of epitope 1- and 2-specific monoclonals to native HBsAg/p33 particles.

Although the cumulative data suggests the conformation-independence of pre-S2 region determinants, secondary structure may influence the spatial relationship between p133-140 and p153-171 in the intact molecule. Alternatively, antibodies that bind determinants separated by 13 residues can be subject to steric hinderance. Whatever the mechanism of inhibition, it is clear that antibody-antibody inhibition analysis was not sufficiently sensitive to allow interpretation of antibody fine specificities for the pre-S2 region of HBsAg.

The ability to map antibody binding sites within the pre-S2 region of HBsAg suggested that this will be a useful model to address the possible influence of T cell helper function on antibody fine specificity. For example, it was demonstrated that immunization with HBsAg/p33 particles elicited a dominant anti-epitope 1 response, whereas, immunization with free peptide (p120-145) yielded both epitope 1- and 2-specific responses, and immunization with carrier-peptide conjugate (p120-145) appeared to favor an epitope 2-specific response.

As demonstrated hereinbefore, T cells primed alternatively with HBsAg/p33 or p120-145 were not cross-reactive, and carrier-specific T cells would also not be expected to be cross-reactive Therefore, these three immunogens expressing the same B cell determinants expressed unique T cell determinants in terms of specificity and regulatory mechanisms; i.e., Ir gene restriction. It is believed that carrier-primed T cells can more efficiently help epitope 2-specific versus epitope 1-specific B cell clones, and that T cells primed with free polypeptide; i.e., 120-132-specific in the C3H.Q strain, are efficient at providing help for both B cell specificities. Other as yet undefined T cell specificities are operative following HBsAg/p33 immunization. It could also be argued that other influences, including antigen processing can affect B cell fine specificity.

However, an intriguing observation described herein was that in the B10 strain, p133-151/ad immunization elicited anti-epitope 1- and 2-specific responses, whereas, immunization with p133-151/ay resulted in an exclusive anti-epitope 2 response. Although T cell recognition of p133-151 has not been determined, preliminary data suggest the carboxy-terminus and residue 141 were involved since T cells primed with p133-151 were not activated by p120-145, and p133-151/adw2 and ayw primed T cells did not cross-react. Therefore, it appears that T cells with distinct fine specificities defined by a single amino acid; i.e., residue 141, provide differential help for the same B cell clones. T cells primed with p133-151/ay provided functional help for the p137-143-specific B cell clone but not for the p133-140-specific B cell clone, whereas, T cells primed with p133-145/ad provided help for both B cell specificities.

It is clear from previous studies that T cells specific for one region of HBsAg (pre-S2) can provide functional T cell help for multiple specificities of B cell clones which recognize pre-S2 and S region determinants; Milich et. al., (1985) Science, 228:1195. However, other studies in the HBsAg system suggest the presence of subtype-specific T cells, which may selectively cooperate with subtype-specific B cell clones; Milich et. al., (1982) J. Immunol., 129:320. For example. B10.S(9R) mice hyperimmunized with the multideterminant HBsAg/p25 produce only subtype-specific and no measurable group-specific antibodies.

The existence of determinant-specific T cell help and T cell help engaging all HBsAg-specific B cell clones were not mutually exclusive. The molecular mechanism by which T cells can provide differential help for B cell clones with different specificities on the same polypeptide is difficult to explain in the context of T-B cell interaction models, but has been observed in other antigen systems as well; Checka et. al., (1976) Eur. J. Immunol. 6:639, Campos-Netro et. al., (1982) Cell. Immunol., 69:128, Berzofsky et. al., (1979) Proc. Natl. Acad. Sci. USA, 76:4046. A T-B cell reciprocity circuit in which B cell-Ia-antigen interactions limit T cell specificity, which in turn limits B cell specificity has been proposed; Berzofsky, (1983) Surv. Immunol. Res., 2:223. Further analysis of T and B cell recognition of the pre-S2 region of HBsAg will be required to address this interesting question.

It has been proposed that pre-S2 region determinants be included in second generation HBV vaccines; Neurath et. al., (1984) Science, 224:392, Milich et. al., (1985) Science, 228:1195, Michel et. al., (1984) Proc. Natl. Acad. Sci. U.S.A., 81:7708. The results of this and the previously described study have implications relative to a synthetic HBsAg vaccine and/or synthetic diagnostic reagents. For example, the small polypeptides can be used to determine the fine specificity of antibody response to see whether antibody to only one of two epitopes is protective. Comparison of the two published synthetic polypeptide sequences of the pre-S2 region, namely p120-145 (Neurath et. al., (1984) Science, 224:392) and p133-151 (Okamoto et. al, (1985) J. Immunol., 134:1); revealed: (1) both peptides possessed dominant murine and human antibody binding sites within the p133-143 sequence; (2) the p120-145 peptide also expressed the p120-132 region which is recognized by some human antisera; (3) the free p120-145 peptide was more immunogenic than the free p133-151 peptide in murine strains by virtue of an efficient T cell recognition site (p120-132); and (4) although less immunogenic, a greater proportion of antibody elicited by p133-151 cross-reacted with native HBsAg/p33 particles than that elicited with p120-145 as described before. Knowledge of the protective efficacy and relevance of pre-S2-specific antibody production and T cell recognition in disease will be a prerequisite to determining the clinical application of these and other pre-S2-specific reagents.

## 1. Summary

The fine specificity of the humoral immune response to the pre-S2 region of HBsAg was studied. It was demonstrated that the murine antibody response to the pre-S2 region was focussed on residues 133-143, and two distinct but overlapping epitopes were identified within 11 continuous residues. One epitope was group-specific, and one epitope was influenced by a subtype-dependent, amino acid substitution at residue 141.

However, the influence of residue 141 was covert in that it was only detected on synthetic antigens 19 residues in length of smaller and not when a synthetic polypeptide of 26 residues or native particles were used as the solid phase ligand. The minimum size of both epitopes (p133-139 and p137-143) was seven amino acid residues. The physical and chemical form of the immunogen (i.e., protein vs. relatively shorter polypeptide; carrier-conjugated vs. free polypeptide) influenced antibody fine specificity. In quantitative antibody inhibition studies, it was demonstrated that antibodies with nonoverlapping as well as overlapping fine specificities were capable of mutual inhibition. Finally, human HBV-infected, patient sera were shown to possess anti-pre-S2 region antibodies that recognized sequences in common with the murine antisera. These results have implications relevant to the design of synthetic and recombinant second generation HBV vaccines and diagnostic reagents.

## C. The Anti-Polypeptide (p120-145) Pre-S Response Was H-2 Restricted, But In a Manner Distinct From The H-2 Restriction Of The Response To The Native Pre-S(2) Region

Since it was previously demonstrated that the immune response to the native pre-S2 region is regulated by H-2-linked genes and was mediated at the T cell level (Milich et al., (1985) Proc. Natl. Acad. Sci. U.S.A.,

82:8168), it was of interest to determine if the response to the synthetic pre-S2 peptide is similarly regulated. A panel of B10, H-2 congenic strains was immunized intraperitoneally (i.p.) with either HBsAg/p33 particles or with the free pre-S2-specific synthetic polypeptide p120-145, both of the ayw subtype, as illustrated in Figure 11. The anti-pre-S2 region responses were analyzed by radioimmunoassay (RIA) using p120-145 as the solid phase-bound ligand. Anti-HBsAg/p33 sera were assayed 24 days following primary immunization. (Pre-S2 response predominated over the S response at this time point; Milich et al., (1985) Science, 228:392.) Anti-polypeptide sera were assayed following secondary immunization.

As illustrated in Figure 11, HBsAg/p33 immunization elicited pre-S2-specific antibody in all strains except B10.M. The hierarchy of responsiveness was as follows: B10 greater than B10.D2 greater than B10.S greater than B10.BR, indicating that the anti-pre-S2 response was influenced by H-2-linked genes.

The results of immunization with p120-145 also indicated the influence of H-2-linked genes on the antibody response. However the order of responsiveness, B10.BR greater than B10 and B10.S greater than B10.D2 and B10.M (nonresponders), was significantly different as compared to HBsAg/p33 immunization. For example, the B10.D2 strain produced anti-p120-145-specific antibody following HBsAg/p33 immunization and yet was a total nonresponder to the p120-145 peptide upon immunization to synthetic polypeptide; i.e., 1:80,000 titer. The $C_3H.Q$ (H-2q) strain demonstrated the highest response to p120-145 immunization with a secondary titer of $1:1.2 \times 10^6$. Therefore, the $C_3H.Q$ strain was utilized in many of the analyses described hereinafter.

Since H-2 restriction was determined at the T cell level, these results argue very strongly that T cell recognition sites on the native pre-S2 region (i.e., residues 120-174) need not be identical to those recognized when the synthetic peptide p120-145 was the immunogen. The B10.D2 murine strain demonstrated a pre-S2-specific T cell proliferative response following HBsAg/p33 immunization [Milich et al., (1985) Proc. Natl. Acad. Sci. U.S.A., 82:8168] and posessed p120-145-specific B cell clones. In that strain, it is clear that the antibody response to the pre-S2 region was not mediated through T cell recognition of the p120-145 sequence, since this strain was nonresponsive to immunization with p120-145. In other words, p120-145 does not contain a T cell determinant recognizable in the context of the H-2d haplotype. [Balb/c mice (H-2d) are also nonresponsive to this peptide.] Consistent with this is the finding that the B10.D2 and Balb/c strains primed with p120-145 did not demonstrate a p120-145-specific T cell proliferative response.

#### D. T Cell Recognition Of p120-145 Focused On The Amino-terminus, Whereas, B Cell (Antibody) Recognition Focused On The Carboxy-terminal Sequence

To examine the fine specificity of the T cell and B cell (antibody) recognition of the pre-S2 region synthetic peptide p120-145, $C_3H.Q$ mice were immunized with p120-145/ayw. The specificity of the in vitro proliferative T cell response and the in vivo antibody response were determined. For this purpose an amino-terminal peptide (p120-132), a carboxy-terminal peptide (p133-145), and an overlapping peptide (p128-138) were synthesized as shown in Figure 10.

To determine possible subtype-specific influences, p120-145/adw2 was also synthesized. Note that the adw2 subtype differs from the adw subtype at amino acid residue positions 126 and 141.

Following 120-145/ay priming, p120-145/ay and p120-132/ay were capable of eliciting significant proliferative T cell responses, whereas, p133-145/ay was minimally reactive and p128-138 was totally nonstimulatory as shown in Figure 12. The fact that there was a less than two-fold difference (not significant) between the proliferative response of p120-145/ay-primed T cells induced by p120-145 and p120-132 through most of the dose response curve localized a predominant T cell recognition site to the amino-terminal 120-132 residues. Furthermore, the minimum degree of cross-reactivity between p120-145 of the ayw versus the adw2 subtype indicated that residue 126 was critical to T cell activation.

Polypeptide p120-145-primed T cells were also challenged in vitro with native HBsAg/p33 particles, which did not elicit a proliferative response. The reciprocal study of priming with HBsAg/p33 and challenging with p120-145 was also negative. This observation reiterates that p120-145 need not represent the T cell recognition site on the native pre-S2 region, as suggested by the H-2 restriction data.

The specificity of the antibody response following p120-145/ay immunization is shown in Figure 13. Sera were assayed 2 weeks following primary (1°) and secondary (2°) immunization.

The antibody response to p120-145 differed from the T cell response in several significant ways. First, anti-p120-145 bound native HBsAg/p33 almost to the same extent as the homologous polypeptide. [Anti-native HBsAg/p33 also binds p120-145 very efficiently; Milich et al., (1985) Science, 228:392.] Second, the majority of antibodies bound to the carboxy-terminal p133-145 sequence (1:409,600) as opposed to p120-132 (1:4096), which elicited the greatest T cell proliferative response. Third, a small percentage of antibodies bound p128-138, whereas, this polypeptide did not activate p120-145-primed T cells. Finally, anti-p120-145/ayw antibodies demonstrated a high degree of cross-reactivity for the p120-145/adw2 subtype polypeptide in contrast to the T cell response. These data indicate that p133-145 represented the dominant antibody binding site on p120-145 as well as a major binding site on native HBsAg/p33, and that p120-132 represented the dominant T cell recognition site on the synthetic polypeptide p120-145, but not with respect to the native protein, at least in the $C_3H.Q$ strain.

Since dominant, independent T and B cell epitopes on p120-145 were identified, it was of interest to compare the relative immunogenicities of the T cell epitope (p120-132) and the B cell epitope (p133-145) with the intact immunogen (p120-145). Although, p120-132 induced a primary (four weeks) antibody response reactive with its own sequence, it did not induce a response cross-reactive on native HBsAg/p33 as illustrated

in Table 8.

## TABLE 8

### Comparative Immunogenicity Of Uncoupled Pre-S2 Region Synthetic Peptides In $C_3H.Q$ Mice

| Immunogen[a] | Antibody Titer and Specificity[b] | | | |
|---|---|---|---|---|
| | p120-145 | p120-132 | p133-145 | HBsAg/p33 |
| p120-145 | 1:512,000 | 1:5120 | 1:256,000 | 1:160,000 |
| P120-132 | 1:160 | 1:640 | 0 | 0 |
| p133-151 | 1:10,240 | 0 | 1:10,240 | 1:10,240 |

[a] Groups of 5 mice were immunized intraperitoneally with 100 ug of each peptide of the ayw subtype in CFA.

[b] Sera were collected and pooled four weeks following primary immunization.

Polypeptide 133-151 was used to represent the B cell epitope in this study, and elicited a primary antibody response that reacted equivalently with native HBsAg/p33 and the p133-145 sequence; i.e., 1:10,240. However, as shown in Table 8, these responses were quite low as compared to the response elicited by p120-145, which possessed both T and B cell determinants.

In addition, preliminary data indicate the presence of a T cell epitope at about p140-151. Here, mice primed with p133-151d and challenged with p133-151d showed a proliferative T cell response, while challenge with p133-151y or p120-145d showed no proliferation.These data also indicate the importance of residue 141 in T cell recognition.

Still further, preliminary data using baboons as host animals indicates the presence of a T cell epitope within p120-145 and p133-151 since both polypeptides used as immunogens without a carrier were capable of inducing antibodies. Thus, the data from the murine system were confirmed in a primate system.

### E. A Single Amino Acid Substitution Defined T Cell Recognition Of Synthetic Polypeptide 120-132.

Because p120-132/ay efficiently induced proliferation of p120-145/ay-primed T cells, and subtype appeared important, $C_3H.Q$ mice were primed with either the ayw or the adw2 subtype sequence of p120-132. T cells were challenged in vitro with p120-132/ay, p120-145/ay, 120-132/ad or 120-145/ad. The ayw and adw2 sequences of p120-132 differ only at residue 126 (threonine, alanine, respectively).

T cells primed with the ayw sequence of 120-132 proliferated in response to p120-132/ay and p120-145/ay but only marginally to the adw2 sequences (66.6-fold difference), as illustrated in Figure 14a. In the reciprocal study, T cells primed with the adw2 sequence of p120-132 proliferated upon challenge with p120-132/ad and p120-145/ad, and were only minimally reactive to the ayw sequences (32-fold difference) as shown in Figure 14b.

These data indicated the importance of residue 126 in the T cell recognition of p120-132, and demonstrated that a single amino acid dramatically affected the fine specificity of T cell recognition since the ayw and adw2 sequences were relatively non-cross-reactive. To further define the T cell recognition site, $C_3H.Q$ mice were

primed with p120-131 of the adw sequence, which differs from both the ayw and adw2 sequences at residues 127 and 130 and lacks residue 132. See Figure 10.

As illustrated in Figure 15, the adw and adw2 sequences were quite cross-reactive, and the ayw sequence was nonstimulatory for p120-131/adw-primed T cells. This finding reiterates the importance of residue 126 and indicates that residues 127, 130 and 132 were not critical to T cell recognition of the p120-132 sequence, which implies that residues of p120-126 represented the dominant T cell recognition site on p120-145.

To examine whether T cell recognition of p120-145 was unique to the C3H.Q strain, several other murine strains were immunized with p120-145/ayw, and T cell proliferative responses specific for p120-132/ayw were determined, as shown in Figure 16. All strains except the B10 strain demonstrated a subtype-dependent T cell proliferative response to the immunogen p120-145/ayw. Although the C3H.Q strain responded best to p120-132, all strains tested except the B10 strain (SJL.J, B10.S, B10.BR and C3H) demonstrated a significant response to p120-132 following p120-145 priming.

Therefore, it appears that p120-132 can be recognized in the context of the H-2q, H-2s, H-2k haplotypes, but not the H-2b haplotype. Interestingly, B10 strain T cells immunized with p120-145/ayw proliferated equivalently to the adw2 and ayw subtype sequences of p120-145, and B10 was the only strain that demonstrated at least a marginal response to challenge with native HBsAg/p33. The absence of a subtype-specific response indicates that residue 126 was not critical to T cell recognition of p120-145 in the B10 strain.

It was also of interest to examine the fine specificity of in vivo antibody production to p120-132. Therefore, C3H.Q mice were immunized with p120-132/ayw. Primary and secondary sera were analyzed for reactivity on solid phase-bound p120-132/ayw, 120-132/adw2 and 120-131/adw as illustrated in Table 9 below.

## TABLE 9

### Reactivity Of Anti-p120-132/ay With Subtype_Sequences

| Immunogen | Time[a] | Antibody Titer and Specificity | | |
|---|---|---|---|---|
| | | p120-132/ ayw | p120-132/ adw2 | p120-131/ adw |
| p120-132/ay | 1° | 1:1280 | 1:640 | 1:80 |
| | 2° | 1:5120 | 1:2560 | 1:40 |
| p120-145/ay | 1° | 1:10,240 | 1:5120 | 1:640 |

[a] Sera were collected 4 weeks following primary immunization (1°), and 2 weeks after secondary immunization (2°).

In contrast to T cell recognition, antibodies to 120-132/ayw cross-reacted with the adw2 sequence quite well (2-fold difference) indicating that the alanine for threonine substitution at residue 126 did not severely affect the antibody binding site on p120-132. Conversely, anti-p120-132/ay antibodies did not bind well to 120-131/adw indicating that the carboxy-terminus of p120-132 was involved in antibody recognition.

For comparison, primary anti-p120-145/ayw serum was also examined for fine specificity and demonstrated similar results, except the more immunogenic p120-145 elicited a greater response to all antigens as demonstrated in Table 9. Note that at the T cell level the adw2 and adw sequences were very cross-reactive. These data indicate that even with respect to a synthetic peptide as small as 13 amino acids, T and B cell recognition sites were distinct.

F. The Amino-terminal Polypeptide 120-132 Primed T Cell Help For The Carboxy-terminal Polypeptide 133-145

Because it appeared that distinct T cell and B cell epitopes existed on p120-145, the ability of the isolated T cell site (p120-132) to prime T helper cell function for the dominant antibody binding site on p120-145 (p133-145) was examined. C3H.Q mice were either primed with 100 ug of p120-132/ay in CFA or injected with CFA alone, and four weeks later boosted with either 100 ug of p120-145/ay or 1.0 ug of HBsAg/p33/ay in incomplete Freund's adjuvant (IFA). Sera were analyzed for an IgG response specific for various determinants one week following the booster immunization. As illustrated in Table 10, p120-132 primed the anti-p133-145 response following the boost with p120-145.

## TABLE 10

## Ability Of p120-132 To Prime *In Vivo* Antibody Production

| Immunogen[a] | Antibody Titer and Specificity | | | |
| --- | --- | --- | --- | --- |
| | p120-145 | p120-132 | p133-145 | HBsAg/p33 |
| p120-132 0 | 1:160 | 1:640 | 0 | 0 |
| 0 p120-145 | 0 | 0 | 0 | 0 |
| p120-132 p120-145 | 1:2560 | 1:5120 | 1:640 | 1:1280 |
| 0 HBsAg/p33 | 1:320 | 1:40 | 1:160 | 1:160 |
| p120-132 HBsAg/p33 | 1:640 | 1:640 | 1:40 | 1:320 |

[a] Groups of four $C_3H.Q$ mice, were primed with 100 ug each of p120-132 in CFA administered i.p. or CFA alone (0), and 4 weeks later boosted with either 100 ug of p120-145 or 1 ug of HBsAg/p33 in incomplete adjuvant (IFA). Mice were bled one week following the boost (2° immunization), and sera were assayed for IgG specific for the indicated antigens. All antigens were of the *ayw* subtype.

33

Unprimed mice did not produce anti-pre-S2 antibodies of any specificity. However, p120-132-primed mice produced an IgG titer of 1:640 specific for the p133-145 peptide. The other solid phase-bound antigens all possessed the 120-132 sequence, so it is difficult to discriminate anti-p133-145 antibodies from anti-p120-132 antibodies on these ligands. Although p120-132 primed the anti-p133-145 response following a p120-145 boost, p120-132 did not prime any pre-S2-specific response following the HBsAg/p33 boost. See Table 10. This finding confirmed that C$_3$H.Q, p120-133-primed T cells did not recognize this sequence on the native protein which is consistent with the lack of a HBsAg/p33-induced proliferative response, and the H-2 restriction data.

G. Monoclonal Antibodies Define Two Distinct But Overlapping Determinants In The Pre-S2 Region

To determine the fine specificity of five pre-S2-specific murine monoclonal antibodies, dilutions representing from 0.007 nanograms per milliliter (ng/ml) to 500 ng/ml of each monoclonal antibody were assayed for binding reactivity to HBsAg/p33 particles and pre-S2-specific synethetic polypeptides of the ayw and adw$_2$ subtype sequences. All the monoclonals were raised to polypeptide antigens (immunogens) expressing the ayw subtype. Each monoclonal antibody bound HBsAg/p33 particles very efficiently at a minimun concentration of 0.03 ng/ml and did not distinguish between subtypes. The results are shown in Table 12, and expressed as the minimum antibody concentrations in ng/ml required to detect binding greater than five times the background binding value.

34

## TABLE 12

Reactivity Of A Panel Of Monoclonal Antibodies With HBsAg/p33 Particles And Pre-S2 Region-Specific Synthetic Polypeptides Corresponding To Residues 120 to 151.

| Solid Phase Antigen | Pre-S2-Specific Monoclonal Antibody[a] | | | | |
|---|---|---|---|---|---|
| | 5520 | 5521 | 5535 | 5161 | 4408 |
| HBsAg/p33/ay | 0.03 | 0.03 | 0.03 | 0.03 | 0.03 |
| HBsAg/p33/ad | 0.03 | 0.03 | 0.03 | 0.03 | 0.03 |
| p120-145/ay | 0.06 | 0.03 | 0.06 | 0.03 | 0.03 |
| p120-145/ad | 0.06 | 0.06 | 0.03 | 0.12[b] | 0.03 |
| p120-132/ay | 0[c] | 0 | 0 | 0 | 0 |
| p120-132/ad | 0 | 0 | 0 | 0 | 0 |
| p133-151/ay | 0.12 | 0.06 | 0.06 | 0.03 | 0.06 |
| p133-151/ad | 0.06 | 0.25 | 0.12 | 4.0 | 0.06 |
| p133-145/ay | 0.12 | 0.25 | 0.06 | 0.12 | 0.25 |
| p133-145/ad | 4.0 | 8.0 | 1.0 | 32.0 | 0.25 |

a   Minimum monoclonal antibody concentration required for binding, ng/ml.

b   Underlined numbers represent a four-fold or greater difference between binding to the ay versus ad subtype sequence.

c   No detectable binding at a concentration of 500 ng/ml.

The largest synthetic polypeptide assayed, p120-145 also was an excellent antigen substrate. However, monoclonal antibody 5161 demonstrated a preference for the ay subtype (immunogen subtype) on this ligand. The amino-terminal sequence of p120-145, namely 120-132, was not reactive with any of the assayed monoclonals. This was expected with respect to Mabs 5520, 5521 and 5535 since they were raised against the intact p133-151 sequence. But Mabs 4408 and 5161, which were raised against the intact p33 polypeptide, also did not bind p120-132. It is noted that the p120-132 sequence represented a dominant T cell recognition site in a number of murine strains as described hereinbefore.

Although polypeptide p133-151, a 19-mer, was the immunogen for Mabs 5520, 5521 and 5535, it was nonetheless, unexpectedly, equivalently antigenic or slightly less antigenic than the larger 26 amino acid residue peptide, p120-145. This minimized the possible influence of residues 146-151.

Furthermore, monoclonal 5521 as well as 5161 demonstrated subtype preference on p133-151. When the p133-145 (13-mer) sequence was assayed, all the monoclonals with the exception of 4408 expressed a strong preference for the ayw-subtype sequence as shown in Table 12. Since the presence of the 146-151 sequence was demonstrated to not affect binding on p133-151 and was conserved between the ayw and adw2 subtypes, it appears that size more than the presence of the carboxy-terminal sequence 146-151 was responsible for the subtype-specificity evidenced on the 13-mer as compared to the 26-mer or the 19-mer.

To further define the specificity of these monoclonal antibody reagents, another series of truncated polypeptides was synthesized and used as solid phase-bound ligands as illustrated in Table 13.

## TABLE 13

### .Reactivity Of A Panel Of Monoclonal Antibodies With Pre-S2-Specific Synthetic Peptides Representing Residues 133 to 145

| Solid Phase Peptide | Pre-S2-Specific Monoclonal Antibody Binding[a] | | | | |
|---|---|---|---|---|---|
| | 5520 | 5521 | 5535 | 5161 | 4408 |
| p133-143/ay | 0.12 | 0.12 | 0.06 | 0.12 | 0.5 |
| p133-143/ad | 16.0[b] | 4.0 | 4.0 | 16.0 | 0.5 |
| p133-140 | 250 | 0[c] | 64.0 | 64.0 | 0.25 |
| p133-139 | 0 | 0 | 0 | 0 | 0.5 |
| p128-138 | 0 | 0 | 0 | 0 | 0 |
| p135-145/ay | 0.12 | 0.12 | 0.12 | 1.0 | 0 |
| p135-145/ad | 250 | 0 | 64.0 | 0 | 0 |
| p135-143/ay | 0.12 | 0.12 | 0.25 | 0.5 | 0 |
| p135-143/ad | 0 | 0 | 64.0 | 0 | 0 |
| p137-145/ay | 4.0 | 0.5 | 4.0 | 8.0 | 0 |
| p137-145/ad | 0 | 0 | 0 | 0 | 0 |

a    Minimum Concentration Required for binding, ng/ml.

b    Underlined numbers represent a four-fold or greater difference between binding to the ay versus ad subtype sequences.

c    No detectable binding at a concentration of 500 ng/ml.

Deletion of two carboxy-terminal residues, 144 and 145 from the p122-145 sequence did not significantly affect binding by any of the monoclonals; i.e., p133-145 compared with p133-143. However, deletion of five carboxy-terminal residues, leaving the group-specific peptide p133-140, virtually eliminated binding of monoclonals 5520 and 5521, and drastically reduced (533- to 1066-fold as compared to p133-143/ay) binding of monoclonals 5535 and 5161. On the other hand, monoclonal 4408 bound p133-140 equivalently to p133-145/ay.

These results indicated residues 141, 142 and 143 were critical to the binding site recognized by monoclonals 5520, 5521, 5535 and 5161, but irrelevant for monoclonal 4408. Since 4408 also bound p133-139 efficiently, residue 140 was also not critical to the binding site of this antibody.

Monoclonal 4408 was unreactive on p128-138, which defined the carboxy-terminus of the 4408 binding site as residue 139. The amino-terminus of the 4408 binding site was defined as 133-134, since Mab 4408 was unreactive on p135-145 of either subtype. Therefore, the recognition site of monoclonal 4408 can be defined as the seven-mer p133-139, possibly p134-139. This sequence was conserved in all HBsAg subtypes, and predictably 4408 did not demonstrate subtype-specificity on any ligand.

Monoclonals 5520, 5521, 5535 and 5161 were minimally reactive on p133-140 and nonreactive on p133-139 as shown in Table 13 before. All four monoclonals bound p135-145 and p135-143 efficiently, however, in a subtype-specific manner (minimal to no binding on the adw2 sequences). These results indicate that the carboxy-terminus of the binding site recognized by monoclonals 5520, 5521, 5535 and 5161 was at least residue 143. Although binding to p137-145/ay was significantly reduced as compared to p135-145/ay with respect to all four monoclonals, the positive reactivity defined the amino-terminus of the binding site recognized by these four monoclonals to at least residue 137. Therefore the recognition site of monoclonals 5520, 5521, 5535 and 5161 can be minimally defined as the seven-mer p137-143. Significantly enhanced binding (immunoreaction) occurred on p135-143.

It is noted that while monoclonals 5520, 5521, 5535 and 5161 all demonstrated subtype-specificity, this subtype preference was only observed on synthetic polypeptides of 19 amino acids or smaller, and not on the 26-mer (except 5161) or intact HBsAg/p33 particles, as shown in Tables 12 and 13. This subtype specificity must focus on residue 141 since the phenylalanine/leucine substitution at this position was the only subtype difference in the p133-151 sequence.

The fine specificities of the representative monoclonals: 5521, 5161 and 4408 assayed on pertinent synthetic peptides are summarized in Figure 18. Cumulatively, these data indicated the existence of two distinct, and overlapping antibody binding sites each of seven amino acid residues in length within residues 133 to 143 of the pre-S2 region. Epitope 1 was minimally defined by monoclonal 4408 as p133-139, and epitope 2 was minimally defined by the other four monoclonals as p137-143. Synthetic polypeptides p133-140 and p135-145 bound antibody more efficiently with the same degree of specificity, and were therefore used as solid phase-bound antigens to represent epitopes 1 and 2, respectively, in further studies.

Epitope 1 is a group-specific since it was conserved in all subtypes, whereas, epitope 2 exhibited subtype specificity depending on the size of the ligand examined. The overlapping sequence shared by both epitopes, residues 137, 138, 139, was conserved in all HBsAg subtypes as shown in Figure 19.

## H. Pre-S2-Specific Monoclonal Antibodies With Distinct But Overlapping Fine Specificities Mutually Inhibited Binding To HBsAg/p33 Particles

Since monoclonal 4408 was specific for a distinct epitope on the pre-S2 region as compared to monoclonals 5520, 5521, 5535 and 5161, it was of interest to determine if monoclonal 4408 binding to the pre-S2 region on native HBsAg/p33 particles would be inhibited by the other monoclonals and vice versa. As illustrated in Figure 20, all pre-S2-specific monoclonal antibodies regardless of fine specificity quantitatively inhibited the binding of 4408 on HBsAg/p33ay to 100%. This was true for both subtypes of HBsAg/p33.

Comparing the concentrations required to inhibit 50% of binding, there was only a 4.5-fold difference between the best (Mab 5535) and worst (Mab 5521) inhibitor. In a reciprocal analysis, each monoclonal quantitatively inhibited the binding of 5521 on HBsAg/p33 to 100% with a five-fold difference between the most and least efficient inhibitor, as illustrated in Figure 21. All combinations of competing and labelled monoclonals gave similar results.

These data indicated that although the fine specificity of monoclonal 4408 was distinct from the other monoclonals, the proximity of the epitopes (3 residue overlap) was sufficient so that they mutually inhibited the binding of the other.

Monoclonal antibody competition studies were also performed using synthetic polypeptides as solid phase-bound ligand. However, since the efficiency of binding the synthetic polypeptides varied somewhat between monoclonals and the competition was commensurate with direct binding, these analyses were less informative.

## I. Specificity Of The Murine, Polyclonal Response To Native HBsAg/p33 Particles

To determine the specificity of the murine pre-S2-specific humoral response following immunization with HBsAg/p33 particle, C3H.Q mice were immunized with HBsAg/p33/ay. Primary and secondary antisera were screened on a panel of synthetic peptides encompassing virtually the entire pre-S2 sequence as illustrated in Figure 22.

It is noteworthy that the titer of primary and secondary antibody detected on the synthetic peptide p120-145 was equivalent to the titer detected on the intact particles, and very little antibody specific for the carboxy-terminal p153-171 was obseved. This indicated that the majority of pre-S2-specific antibody elicited by immunization with native HBsAg/p33 particles was directed to residues 120 through 145. Furthermore, most of the HBsAg/p33-specific antibody was directed to the carboxy-terminal sequence p133-145 (1:40,960) as opposed to the amino-terminal p120-132 (1:5120), as was previously observed following immunization with synthetic polypeptide p120-145, as described before.

With respect to fine specificity of the anti-native pre-S2 response, p133-140 (epitope 1) represented the dominant antibody binding site. C3H.Q polyclonal anti-p120-145 [Figure 23(a)] and anti-HBsAg/p33 [Figure 23(b)] antisera inhibited the binding of monoclonal antibodies 5521, 5161 and 4408 to HBsAg/p33, thus confirming that the p133-143 sequence represented a predominant antibody binding site for these polyclonal antisera. Numerous inbred strains of mice were immunized with HBsAg/p33 particles, and the pre-S2-specific responses were quite similar to that of the C3H.Q strain.

## J. The Physical Form Of The Immunogen Influenced The Fine Specificity Of The Pre-S2-Specific Humoral Response

Alternate antigenic forms of the pre-S2 region can be considered for vaccine or other inoculum purposes. Therefore, it was of interest to compare the effects of immunization with pre-S2 determinants expressed on intact particles, free polypeptide, polypeptide-protein conjugate, and a p33 polypeptide preparation on the fine specificity of the response. Antisera were analyzed for reactivity with solid phase peptides: p133-145 (representing epitopes 1 and 2); p133-140 (representing epitope 1); and p135-145 (representing epitope 2) of both subtypes (except for the group-specific p133-140).

As illustrated in Table 14, below, $C_3H.Q$ and B10 strains immunized with HBsAg/p33 particles produced predominantly p133-140-specific antibody. The influence of subtype was minimal, as demonstrated by the marginal responses to the p135-145 sequence. Immunization of $C_3H.Q$ mice with the relatively large, free peptide (120-145) elicited a heterogeneous antibody response consisting of a subtype-specific response detected on p135-145 of the appropriate subtype and a group-specific response detected on p135-140.

38

## TABLE 14

### Influence Of Physical Form Of
### The Immunogen On Anti-Pre-S2 Fine Specificity

| | Anti-Pre-S2 Titer (1/dilution) | | | | |
|---|---|---|---|---|---|
| | | Epitope | | | |
| | 1+2 (p133-145) | | 1 (p133-140) | 2 (p135-145) | |
| Antisera | d | y | | d | y |
| **Particle** | | | | | |
| B10 a-HBs/p33/ay[a] | 10,240 | 20,480 | 10,240 | 0 | 80 |
| B10 a-HBs/p33/ad | 5,120 | 5,120 | 10,240 | 0 | 0 |
| $C_3H.Q$ a-HBs/p33/ay | 10,240 | 40,960 | 20,480 | 0 | 160 |
| **Free polypeptide** | | | | | |
| $C_3H.Q$ a-p120-145/ay[a] | 25,600 | 409,600 | 51,200 | 200 | 102,900 |
| $C_3H.Q$ a-p120-145/ad | 51,200 | 6,400 | 12,800 | 12,800 | 400 |
| B10 a-p133-151/ay[b] | 0 | 320 | 0 | 0 | 160 |
| B10 a-p133-151/ad | 2,560 | 2,560 | 2,560 | 640 | 40 |
| **OVAL - Coupled polypeptide[c]** | | | | | |
| Balb/c a-p133-151/ay[d] (Mab 5521) | 128,000 | $4 \times 10^6$ | 0 | 0 | $4 \times 10^6$ |
| **p33 polypeptide** | | | | | |
| Balb/c a-p33/ay[d] (Mab 4408) | $2 \times 10^6$ | $2 \times 10^6$ | $4 \times 10^6$ | 0 | 0 |
| Balb/c a-p33/ay (Mab 5161) | 32,000 | $8 \times 10^6$ | 4,000 | 0 | $1 \times 10^6$ |

[a] Polyclonal antisera collected following secondary immunization.

[b] Polyclonal antisera collected following primary immunization.

[c] OVAL-Coupled polypeptide = polypeptide coupled to ovalbumin as a conjugate.

[d] Monoclonal antibodies, stock concentration 1 mg/ml.

It is noted that the antibodies detected on 133-140 accounted for the anti-p133-145 antibody that bound to the heterologous subtype sequence, whereas, antibodies reactive with the p135-145 sequence were virtually subtype-specific. Immunization of the B10 strain with the 19-mer (p133-151) of the ayw sequence induced a low titered, exclusively subtype-specific response. In contrast, immunization with the adw2 subtype of p133-151 elicited both group-specific (epitope 1) and subtype-specific (epitope 2) responses of higher titer as compared to p133-151/ay immunization. This interesting observation indicated that the single amino acid substitution between subtypes at residue 141 influenced both immunogenicity and antibody specificity of the immune response to p133-151 in the B10 strain.

Monoclonal 5521 as well as 5520 and 5535 were produced following immunization with p133-151/ayw coupled to ovalbumin, and all these monoclonals are highly subtype-specific. Since monoclonal 5521 is nonreactive on p133-140 as shown in Table 14, the reactivity on the heterologous p133-145 subtype represents cross-reactivity rather that a dual specificity as seen in the polyclonal sera. Since these are monoclonal antibodies, the fact that they are all subtype-specific does not imply that coupled-polypeptide only elicited epitope 2-specific antibody, but does indicate that such immunization induced epitope 2-specific B cell clones.

However, rats immunized with free p120-145 produced predominantly group-specific antibody, whereas, rats immunized with p120-145 coupled to keyhole limpet hemocyanin (KLH) produced predominantly subtype-specific antibody. Therefore, it is possible that immunization with coupled versus free polypeptide influences antibody fine specificity. The specificity of the two monoclonals produced by immunization with p33 polypeptide preparations (Mab 4408 and Mab 5161) indicated that such immunization elicited either group-specific (4408) or subtype-specific (5161) antibody specificity as shown in Table 14.

K. A Pre-S2-Specific Nonoverlapping Antibody Inhibited Anti-p133-143 Binding To HBsAg/p33

Unlike the amino-terminal half of the pre-S2 region, the carboxy-terminal half is highly substituted with respect to the adw2 and ayw sequences as shown in Figure 17. A murine polyclonal antiserum to p153-171/adw2 was prepared, which was quite subtype-specific. As shown in Figure 24, this antibody preparation efficiently inhibited both p133-139-specific monoclonal 4408, and p137-143-specific monoclonal 5161 binding to the HBsAg/p33/adw subtype but not to the ayw subtype. Therefore two distinct and nonoverlapping antibody binding sites; i.e., p133-139 and p153-171, separated by at least 13 amino acid residues in the primary sequence, cannot simultaneously be occupied by their respective antibodies.

L. Fine Specificity Of The Human Anti-pre-S2 Humoral Response Following HBV Infection

A number of human sera from both acute and chronic HBV-infected patients were screened for antibody specific for the pre-S2 region of HBsAg using synthetic polypeptide p120-145 as the solid phase-bound antigen. Positive sera were selected and the fine specificity for truncated polypeptides determined. Numerous patterns of fine specificity were observed. The results for representative sera are illustrated in Table 15.

TABLE 15

Fine Specificity Of The Human
Anti-Pre-S2 Response Following HBV Infection'

| HBV Serum Number | Antibody Titer To:[a] Pre-S2 region synthetic peptide (1/dilution) | | | | | |
|---|---|---|---|---|---|---|
| | p120-145 | p120-132 | p133-145 | p133-140 | p135-145 | p153-171 |
| 1 | 800 | 100 | 160 | 50 | 50 | 50 |
| 2 | 3200 | 50 | 800 | 20 | 10 | 10 |
| 3 | 6400 | 200 | 3200 | 3200 | 200 | 50 |
| 4 | 1600 | 100 | 1600 | 50 | 1600 | 100 |
| 5 | 6400 | 3200 | 100 | 50 | 50 | 100 |
| 6 | 6400 | 3200 | 800 | 200 | 50 | 50 |
| NHS[b] | 50 | 50 | 50 | 50 | 100 | 50 |
| NHS | 50 | 100 | 50 | 100 | 100 | 50 |
| NHS | 10 | 20 | 50 | 20 | 10 | 50 |
| NHS | 20 | 10 | 10 | 10 | 20 | 10 |

a   Dilutions of human sera were incubated (admixed and maintained) with solid phase-bound peptides or uncoated wells, and bound antibody was determined by probing with an enzyme-labelled anti-human IgG reagent. The titer was defined as the highest serum dilution to yield an $OD_{490}$ reading of 0.10. Binding at a four-fold or greater serum dilution than observed on uncoated wells was considered peptide-specific, and is underscored. Serum dilutions for uncoated wells (non-specific, background binding) ranged from 10 to 100.

b   NHS, normal human serum.

None of the sera demonstrated binding to the pre-S2 region carboxy-terminal 153-171 residues. The fine specificities of some of the sera were not assayed since antibody was only detected on the large peptide (p120-145), as exemplified by patient serum no. 1. Serum no. 2 illustrates the most commonly observed binding pattern in which antibodies specific for p120-145 and for p133-145 were detected, but antibody binding to the smaller truncated peptides was not observed. However, several sera did demonstrate binding to p133-140 and p135-145, as exemplified by sera nos. 3 and 4, respectively. Interestingly, serum 4 bound to the ayw sequences of p120-145 and p135-145 preferentially as compared to the adw2 sequences, implicating residue 141. Sera representing other patterns of specificity did not demonstrate significant subtype-specificity. While all the murine sera and the majority of human sera preferentially recognized the p133-145 sequence, several human sera recognized the amino-terminal p120-132 sequence either exclusively (serum No. 5) or in addition to the p133-145 sequence (serum No. 6).

Cumulatively, these results confirmed an earlier report that a pre-S2-specific humoral response occurred during HBV infection (Neurath, et. al. (1984) Science, 224:392), and indicated that this response was directed to the p120-145 region, and primarily to the p133-145 sequence as was observed for the murine response following immunization. However, it should be noted that several human antisera also recognized the p120-132 sequence efficiently, unlike the murine response.

M. p12-21 Linked To A B Cell Determinant Acts As A T Cell Helper Determinant

A group of 5 SJL/J mice were immunized with either p133-151 or the composite peptide p(12-21)-133-151, in which the 12-21 peptide sequence served as a polypeptide (a) and the 133-151 sequence served as a polypeptide (b). As shown in Table 16, immunization with p133-151 alone elicited no antibody production after four weeks, however, immunization with p(12-21)-133-151 resulted in antibody production to p133-151 in the same time period. The antibodies so induced cross-reacted with native HBsAg/gp33 particles. Only after a secondary immunization with p133-151 were anti-p133-151 antibodies detected, and their titers were approximately 10-fold less than that measured against p133-151 and 32-fold less than that measured against HBsAg/gp33 as compared to sera from p(12-21)-133-151-immunized mice.

TABLE 16

Ability of the p12-21 sequence to function as carrier for a synthetic pre-S(2) region B cell determinant (p133-151).

| IMMUNOGEN | TIME[1] | Antibody Titer Against Antigens | | | |
|---|---|---|---|---|---|
| | | p12-21 | p133-151 | p(12-21)-133-151 | HBsAg/gp33 |
| p133-151 | 2 wk | 0 | 0 | 0 | 0 |
| | 4 wk | 0 | 0 | 0 | 0 |
| | 2° | 0 | 1,280 | 160 | 160 |
| p(12-21)-133-151 | 2 wk | 0 | 0 | 0 | 0 |
| | 4 wk | 0 | 1,280 | 1,280 | 160 |
| | 2° | 0 | 10,240 | 5,120 | 5,120 |

[1]Time in weeks (wk). 2° = secondary immunization.

The results shown in Table 16 illustrate that p12-21 can function as a T cell helper determinant [polypeptide (a)] for a synthetic B cell determinant [polypeptide (b)] in a strain that is responsive to p12-21 at the T cell level. It is noted that the p133-151 sequence is not adjacent to the p12-21 sequence in the native molecule, and in fact, those sequences are separated by 111 amino acid residues in the linear sequence.

N. Composite-Subtype Vaccines

The commercial HBsAg S region vaccines developed to date (plasma-derived and recombinant) have consisted of subviral particles of a single subtype (i.e., ad or ay). The rationale has been that antibodies to the group-specific (a) determinant(s) are protective, and subtype-specific antibodies are not required for protection. This practice has not taken into account the desirability of priming subtype-specific T cell memory.

Thus, when an individual was immunized with one subtype, subtype-specific memory T cells would be incapable of evoking an anamnestic response in the event of a subsequent viral infection with the heterologous subtype.

It has been previously demonstrated that a number of murine stains immunized with HBsAg/P25(ad) recognized subtype-specific T cell sites exclusively, and no group-specific sites within the S region, but nonetheless produced anti-a antibodies as well as anti-d antibodies. Therefore, subtype-specific T cells are able to help group-specific B cell antibody production. Milich et al., J. Immunol., 134:4194 (1985).

It is now found that when murine strains B10.S and B10.M were examined for the influence of subtype on the pre-S(2) immune response, T cell recognition of the pre-S(2) region was also found to be subtype-specific. That is, pre-S(2)/ad-primed T cells recognized pre-S(2)ad-containing HBsAg particles, but not pre-S(2)/ay-containing HBsAg particles. Furthermore, as can be seen from Table 17, below, B10.M mice, which are nonresponsive to the pre-S(2) and S regions when immunized with pre-S(2) or S-HBsAg/ay particles, are responsive to pre-S(2)-HBsAg/ad particles, and produce both anti-S and anti-pre-S(2) antibodies.

## TABLE 17

### SUBTYPE SPECIFICITY OF ANTIBODY RESPONSES

| | | | ANTIBODY TITER | |
|---|---|---|---|---|
| IMMUNOGEN | STRAIN[1] | TIME[2] | ANTI-S | ANTI-PRE-S(2) |
| HBsAg/P33(ay) | B10.S | 10d. | 0 | 0 |
| | | 24d. | 0 | 1:640 |
| | | 2° | 1:640 | 1:10,240 |
| | B10.M | 10d. | 0 | 0 |
| | | 24d. | 0 | 0 |
| | | 2° | 0 | 0 |
| HBsAg/P33(ad) | B10.S | 10d. | 0 | 1:640 |
| | | 24d. | 1:640 | 1:640 |
| | B10.M | 10d. | 0 | 1:160 |
| | | 24d. | 1:2560 | 1:5120 |

[1]B10.S mice are nonresponsive to the S region of HBsAg of both subtypes when immunized with HBsAg/P25. B10.M mice are nonresponsive to the S region of HBsAg of both subtypes (i.e., HBsAg/P25), and to the pre-S(2) region of the ay subtype [i.e., HBsAg/gP33(ay)], but are responsive to the ad subtype [i.e., HBsAg/gP33(ad)].

[2]Time in days (d.) after primary immunizations. 2° = response after secondary immunization.

These results indicate that S- and pre-S(2)-containing HBsAg vaccines benefit from inclusion of both pre-S(2) and S ad and ay subtypes for at least two reasons: (1) the priming of T cell memory relevant to subtype infection with either subtype of the virus: and (2) nonresponders to one subtype can be responsive to the other subtype in terms of antibody production and therefore, the number of genetic nonresponders can be reduced.

Thus, the present invention further contemplates a vaccine against HBV infection comprising, in admixture with physiologically tolerable diluent, a first immunogenic polypeptide displaying the HBV pre-S(2) and S ad T cell subtype and a second immungenic polypeptide displaying the HBV pre-S(2) and S ay T cell subtype, the first and second polypeptides each being present in an amount sufficient to induce a subtype-specific T cell response. The polypeptide of vaccine can be in the form of polypeptide particles as in the HBsAg/gP33 particles discussed herein or as composite immunogens are also described herein. Both subtype polypeptides can also be present in a single polypeptide sequence as in the composite immunogens

43

discussed herein, except that such an immunogen contains a polypeptide (a) for each subtype.

## VII. Materials And Methods

### A. Materials

The C57BL/10 (B10), B10.D2, B10.BR, B10.S, B10.M, SJL/J, C3H.Q. C3H and Balb/c inbred murine strains were obtained from the breeding colony at the Research Institute of Scripps Clinic, La Jolla, CA. Female mice between six and eight weeks of age at the initiation of the studies were used in all studies.

Recombinant HBsAg/p33/ay particles were provided by Dr. P. Tiollais (Pasteur Institute, Paris). Those particles were prepared from chinese hamster ovary (CHO) cells transfected with a plasmid containing the S gene and the pre-S region gene of the ayw subtype of HBV. The CHO-derived particles are composed of the S-encoded p25 plus the pre-S2 and S-encoded p33 in a ratio of approximately 3 to 1, as described in Michel et al., Proc. Natl. Acad. Sci. USA., (1984) 81: 7708, which is incorporated herein by reference.

Purified, serum-derived HBsAg/p33/ad particles were provided by Dr. D Peterson (Medical College of Virginia, Richmond, VA). Those particles were derived from the adw2 subtype and contain p33 represented as approximately 8.2 percent of the constituent polypeptides. A plasma-derived HBsAg/p39 preparation was also provided by Dr. Peterson.

Purified preparations of HBsAg/p25/ad were provided by Dr. R Louis (Cutter Laboratories, Berkeley, CA). That preparation was purified by Cutter Laboratories from human plasma by a combination of standard procedures including ultracentrifugation, ammonium sulfate precipitation, pepsin digestion and gel chromatography. The resulting preparation was composed exclusively of the 25 kilodalton (kd) polypeptide (p25) and its glycosylated form (gp28).

Plasma-derived, purified HBsAg preparations representing both the ad and ay subtypes and consisting of various proportions of p25, p33 and p39 were provided by Dr. J. Gerin (Georgetown University, Rockville, MD). The preparation containing the highest proportion of pre-S1 (p39) was used predominantly throughout his study, and was designated HBsAg/p39.

There was variation in the amount of S region-specific antigenicity per unit weight of particles in the HBsAg preparations utilized herein. Therefore, all HBsAg preparations were first equilibrated with respect to S region-specific antigenicity in terms of antibody binding using a solid phase, sandwich enzyme-linked immunosorbent assay (ELISA) described hereinafter. Then the proportions of p25, p33 and p39 present in the various HBsAg particle preparations were similarly determined. In a sandwich ELISA assay, which is well known in the art, the antigen (HBsAg) to be measured was sandwiched between a solid phase-bound monoclonal antibody specific for either S, pre-S1 or pre-S2 and a peroxidase-labelled second anti-S monoclonal (non-cross-reactive with the solid phase anti-S monoclonal) used herein as a probe.

Two monoclonal antibodies (Mabs) specific for the S region of HBsAg were provided by Dr. P. Kaplan (Ortho Diagnostics, Inc., Raritan, NJ). Mab specific for the pre-S1 region of HBsAg (A18/7) were provided by Dr. W. Gerlich (University of Gottingen, Gottingen, Germany). Those Mabs are described in Heerman et al., (1984) J. Virol., 52: 396. Mab 5520 5521 and 5535 specific for pre-S2 were produced following immunization of Balb/c mice with the synthetic peptide p133-151 (subtype ayw) coupled to ovalbumin and were provided by Dr. T. Nakamura (Institute of Immunology, Tokyo, Japan), as described in Okamoto et al., J. Immunol., (1985) 134: 1212. Mab 4408 and Mab 5161 specific for pre-S2, produced following immunization of Balb/c mice with intact p33 polypeptide derived from HBsAg/ayw particles as described by Machida et al., (1984) Gastroenterol., 86: 910, were also provided by Dr. T. Nakamura.

### B. Immunization

To study in vivo antibody production, groups of mice were immunized using inocula containing either 1-2 micrograms of native HBsAg in its various forms, p25, p33 or p39, or 100 micrograms of the various synthetic polypeptides as immunogen dispersed in complete Freund's adjuvant (CFA) by intraperitoneal (IP) injection as the primary immunization (1°). A secondary immunization (2°) using an inoculum containing 2 micrograms (ug) of HBsAg in its various forms in CFA of containing 0.5 ug HBsAg/p33 or 50 ug of a synthetic polypeptide as immunogen in incomplete Freund's adjuvant (IFA) was administered IP about four weeks after primary immunization (1°). Mouse sera were obtained from the retroorbital plexus 24 days following primary immunization and again 2 weeks following secondary immunization, unless otherwise stated.

In vivo priming for the T-cell proliferative assay was accomplished by hind footpad injections of either 4 ug of HBsAg or 100 ug of synthetic polypeptide in CFA.

### C. Measurement Of Anti-HBs And Anti-pre-S

Anti-S and anti-pre-S antibodies induced by immunization with a synthetic polypeptide or native HBsAg, and anti-polypeptide antibodies induced by polypeptide immunizations were measured by the following methods.

#### 1. Radioimmunoassay

Murine sera were evaluated for anti-S, anti-pre-S1 or anti-pre-S2 and anti-synthetic polypeptide reactivity in an indirect, immunoglobulin class-specified, radioimmunoassay (RIA) utilizing as the solid phase-bound ligand either a synthetic polypeptide (1-2 ug per well), or purified HBsAg particles (0.1 micrograms per well). Goat anti-mouse IgG, were utilized to sandwich the murine antibodies bound to the solid phase-bound ligand. After rinsing away any unbound goat anti-mouse antibodies, $^{125}$I-labelled swine anti-goat IgG were admixed and maintained with the goat anti-mouse antibodies bound to the solid phase as described in Milich et al., (1982) J. Immunol., 129: 320. HBsAg/p25/ad was used as a solid phase-bound ligand to assay anti-S-region specific antibody. Synthetic polypeptide p120-145 was used as a solid phase-bound ligand to assay pre-S2-specific antibody. Similarly, synthetic polypeptide p94-117 was used to assay pre-S1-specific antibody. Numerous other HBsAg preparations and synthetic peptides were also used as solid phase-bound ligands in these studies as indicated in the relevant tables.

Data are expressed as an antibody titer representing the highest dilution to yield three times the counts of pre-immunization sera values. Monoclonal antibodies specific for these same antigens were assayed by the same indirect solid phase RIA except that the data are expressed as antibody titer representing the minimum concentration required to detect binding greater than five times the counts of background value.

## 2. ELISA

Human sera were assayed for antibody reactivity using specific pre-S1 or pre-S2 synthetic peptides as solid phase-bound ligands (1.0 microgram per well) in an enzyme-linked immunosorbent assay (ELISA). Assayed sera were first admixed and maintained (incubated) with the solid phase-bound ligands. After removing any unbound serum constituents, the resulting solid phases were probed with a peroxidase-labelled anti-human IgG monoclonal antibody. Sample optical density values at 490 nanometers (O.D. 490) greater than four times that of normal sera were considered positive using 1:50 dilutions of human sera that were admixed and maintained (incubated) with ligand. In some cases, dilutions of sera, e.g., Table 15, were incubated with ligand. Here the titer was defined as the highest serum dilution to yield an O.D. 490 reading of 0.10.

Antibody-antibody competition assays were performed by pre-admixing and maintaining (pre-incubating) varying concentrations [0.6 - 625 nanograms per milliliter (ng/ml)] of the unlabelled competing antibody with a predetermined limiting concentration of solid phase-bound ligand (HBsAg/p33 0.1 ug well) for a time period of about 18 hours (overnight) at 4 degrees C, followed by admixing and maintaining (incubating) of a predetermined amount of enzyme-labelled (horseradish peroxidase) second antibody whose inhibition was being measured. The results are expressed as percent inhibition of the labelled antibody solid phase-bound ligand interaction. Monoclonal antibodies 4408, 5521 and 5161 were labelled with enzyme and used as described. The competing antibodies used in these studies were either monoclonal or polyclonal antibodies raised to either synthetic polypeptides or HBsAg particle preparations.

## D. HBsAg-Specific T Cell Proliferation Assay

Groups of four mice were immunized in the hind footpads with an emulsion of CFA and either 4 ug of HBsAg or 100 ug of synthetic polypeptide. Eight to ten days later, popliteal lymph node (PLN) cells were harvested and cultured in vitro to a concentration of $5 \times 10^5$ cells/ml with various challenge antigens (ligands). The in vitro antigens included native HBsAg [p25, p33 and p39 which contained various, known, ratios of S, pre-S1 and pre-S2], and the pre-S region synthetic polypeptides p1-21, p12-32, p32-53, p53-74, p94-117, p120-131/adw, p120-132/adw2, p120-132/ayw p120-140/ad, p120-145/ad, p120-145/ay, p128-138, and p133-145.

Draining popliteal lymph node cells were aseptically removed from each mouse and teased to yield a single cell suspension. The cells were washed twice with a balanced salt solution (BSS) containing phosphate-buffered saline (pH 7.2). The cells were resuspended in Click's medium containing BSS, L-glutamine, sodium pyruvate, antibiotics, 2-mercaptoethanol, essential and non-essential amino acids and vitamins. [See Click et al., (1972) Cell. Immunol., 3:264.] Click's medium was modified by the addition of 10 millimolar (mM) HEPES (N-2-hydroxyethyl piperazine-N'-2-ethanesulfonic acid) and gentamycin (10 ug/ml), and by the substitution of 0.5 percent syngeneic normal mouse serum for fetal calf serum.

The antigens were assayed in culture over a dose range of 0.003-1 ug/ml for HBsAg preparations, and 0.07-200 ug/ml for synthetic polypeptides.

Viable lymph node cells ($5 \times 10^5$) in 0.1 ml of medium were placed in flat-bottom microtiter wells (Falcon 3072, Falcon Plastics, Inc.) with: (a) 0.1 milliliter of medium containing various concentrations of HBsAg preparation of a synthetic peptide, (b) culture medium and ovalbumin [200 micrograms per milliliter (ug/ml)] as a negative control, or (c) purified protein derivative (PPD) 100 mg/ml; (Parke-Davis, Detroit, MI) as a positive control.

Cultures were maintained for five days at 37 degrees C in a humidified atmosphere containing 5 percent carbon dioxide in air.

On the fourth day, each culture was admixed and maintained (incubated) with one microcurie (uCi) $^3$H-thymidine ([3]HTdR) (6.7 Ci/millimole, New England Nuclear, Boston, MA) for 16 hours before harvesting. Cells were harvested onto filter strips and proliferation was determined by the incorporation of [3]HTdR into DNA. The data are expressed as counts per minute (cpm) corrected for background proliferation in the absence of antigen. It was demonstrated previously that the HBsAg-specific proliferation response of draining PLN cells harvested up to 13 days post-immunization was due to proliferating T cells; Milich et al., (1983) J. Immunol., 130:1401. Therefore, unfractionated PLN cells were used in analyses reported herein.

## E. Synthesis Of Polypeptides

The polypeptides corresponding to the pre-S2 region utilized herein were chemically synthesized by solid phase methods as described in Merrifield et at., (1963) J. Am. Chem. Soc., 85:2149. The solid phase method of polypeptide synthesis was practiced utilizing a Vega 250 Peptide Synthesizer and an Applied Biosciences 430A Peptide Synthesizer, available commercially from Vega Biotechnologies, Inc., Tucson, AZ and Applied Biosystems, Foster City, CA, respectively. The composition of each polypeptide was confirmed by amino acid analysis.

Briefly, in preparation a synthetic polypeptide by the above solid phase method, the amino acid residues are linked to a resin (solid support) through an ester linkage from the carboxy-terminal residue. When the polypeptide is to be linked to a carrier or another polypeptide via a Cys residue or reacted via terminal Cys residues, it is convenient to utilize that Cys residue as the carboxy-terminal residue that is ester-bonded to the resin.

The alpha-amino group of each added amino acid is typically protected by a tertiary-butoxycarbonyl (t-BOC) group prior to the amino acid being added into the growing polypeptide chain. The t-BOC group is then removed prior to addition of the next amino acid to the growing polypeptide chain.

Reactive amino acid side chains were also protected during synthesis of the polypeptides. Usual side-chain protecting groups were used for the remaining amino acid residues as follows: O-(p-bromobenzyloxycarbonyl) for tyrosine; O-benzyl for threonine, serine, aspartic acid and glutamic acid; 4 methylbenzyl and S-methoxybenzyl for cysteine, dinitrophenyl for histidine; 2-chlorobenzoxycarbonyl for lysine and tosyl for arginine.

The peptides synthesized on the Applied Biosystems Model 430A Peptide Synthesizer were made using the symmetrical anhydride method of Hagenmaier, H., and Frank, A. (1972), Hoppe-Seyler's Z. Physiol. Chem., 353:1973. The DCC in situ method, as described by Merrifield et al. (1963) J. Amer. Chem. Soc. 85:2149 was used to synthesize the peptides from the Vega 250 Peptide Synthesizer. Repeat coupling of the incoming protected amino acid was sometimes necessary to effect complete coupling efficiency. All coupling reactions were more than 99% complete by the quantitative ninhydrin test of Sarin (1981), Analytical Chemistry, 117:147.

After preparation of a desired polypeptide, a portion of the resulting, protected polypeptide (about 1 gram) was treated with two milliliters of anisole, and anhydrous hydrogen fluoride, about 20 milliliters, was condensed into the reaction vessel at dry ice temperature to form an admixture. The resulting admixture was stirred at about 4 degrees C for about one hour to cleave the protecting groups and to remove the polypeptide from the resin. After evaporating the hydrogen fluoride at a temperature of 4 degrees C with a stream of $N_2$ the residue was extracted with anhydrous diethyl ether three times to remove the anisole, and the residue was dried.

The dried material was extracted with 5 percent aqueous acetic acid (3 times 50 milliliters) to separate the free polypeptide from the resin. The extract-containing solution was lyophilized to provide the polypeptide.

The polypeptides derived from the pre-S1 region were chemically synthesized.

The resultant synthetic polypeptides were used as reagents in an enzyme-linked immunosorbent assay (ELISA) to detect anti-HBsAg antibodies. The synthetic polypeptides were also used in inocula, usually in CFA or IFA, as discussed hereinbefore.

The present invention has been described with respect to preferred embodiments. It will be clear to those skilled in the art that modifications and/or variations of the disclosed compositions and methods can be made without departing from the scope of the invention set forth herein.

## Claims

1. A polypeptide of about 6 to about 50 amino acid residues consisting essentially of an amino acid residue sequence that corresponds to a portion of the pre-S region of the HBsAg protein located between an amino-terminal and a carboxy-terminal position, respectively, selected from the group consisting of 12-21, 16-27, 94-105, 106-117, 120-126, 120-132, 120-140, 128-138, 133-139, 133-140, 133-143, 133-145, 134-139, 135-143, 135-145, 137-143, 137-145 and 140-151 from the amino-terminus of said pre-S region, said polypeptide being free of amino acid residues at the enumerated amino-terminal and the carboxy-terminal positions that correspond to contiguous amino acid residues in the linear sequence of HBsAg.

2. A composite immunogen of at least 14 amino acid residues comprising a first polypeptide (a) having a terminal residue that is operatively linked to a terminal residue of a second polypeptide (b) wherein:
polypeptide (a) consists essentially of a T cell epitope having an amino acid residue sequence that corresponds to a portion of the pre-S region of the HBsAg protein located between an amino-terminal and carboxy-terminal position, respectively, selected from the group consisting of 12-21, 94-105, 106-117, 120-126, 120-132, 120-140 and 140-151 from the amino-terminus of said pre-S region; and
polypeptide (b) consists essentially of an amino acid residue sequence that corresponds to an amino acid residue sequence of HBsAg that contains a native B cell epitope;
said terminal residue of polypeptide (a) that is operatively linked to said terminal residue of polypeptide (b) corresponding to positions separated in the linear HBsAg sequence by at least fifteen amino acid residues.

3. The composite immunogen of claim 2 wherein polypeptide (b) is selected from the group consisting of a 33 kilodalton HBsAg polypeptide and a 25 kilodalton HBsAg polypeptide.

4. The composite immunogen of claim 2 wherein polypeptide (b) corresponds to a portion of the pre-S region of HBsAg located between an amino-terminal and carboxy-terminal position selected from the group consisting of 1-21, 16-27, 32-53, 53-74, 94-105, 94-117, 106-117, 120-140, 120-145, 128-138, 133-139, 133-140, 133-143, 133-145, 133-151, 135-143, 135-145, 137-143, and 153-171.

5. The composite immunogen of claim 2 wherein polypeptide (b) corresponds to a portion of the S region of HBsAg located between an amino-terminal and carboxy-terminal position, respectively, selected from the group consisting of 110-137, 117-137, 122-137, and 135-155.

6. The composite immunogen of claim 2 wherein said terminal residue of polypeptide (a) operatively linked to said terminal residue of polypeptide (b) is the carboxy-terminal residue of polypeptide (a).

7. The composite immunogen of claim 2 wherein said terminal residue of polypeptide (a) operatively linked to said terminal residue of polypeptide (b) is the amino-terminal residue of polypeptide (a).

8. A composite immunogen comprising a polypeptide that consists essentially of an amino acid residue sequence that corresponds to a portion of the pre-S region of the HBsAg protein located between an amino-terminal and carboxy-terminal position, respectively, selected from the group consisting of 12-21, 120-126 and 120-132 from the amino-terminus of said pre-S region operatively linked to a non-HBsAg B cell epitope.

9. The composite immunogen of claim 8 wherein said B cell epitope is a polypeptide.

10. The composite immunogen of claim 8 wherein said B cell epitope contains an amino acid residue sequence that corresponds to a native B cell epitope of a protein.

11. The composite immunogen of claim 10 wherein said protein is a surface protein of a pathogen.

12. A method of enhancing the immunogenicity of an immunogen comprising operatively linking said immunogen to a polypeptide consisting essentially of an amino acid residue sequence that corresponds to a portion of the pre-S region of the HBsAg protein located between an amino-terminal and carboxy-terminal position, respectively, selected from the group consisting of 12-21, 94-105, 106-117, 120-126, 120-132, 120-140, 133-145 and 140-151 from the amino-terminus of said pre-S region.

13. The method of claim 12 wherein said immunogen is the immunogen of a vaccine against hepatitis B virus.

14. A method of mitigating nonresponsiveness to an HBV vaccine comprising operatively linking an amino acid residue sequence corresponding to a native T cell epitope in the pre-S1 region of HBsAg to the immunogen of said vaccine.

15. The method in accordance with claim 14 wherein said native T cell epitope is located at positions selected from the group consisting of 12-21, 94-105 and 106-117 from the amino-terminus of the pre-S region.

16. The method in accordance with claim 14 wherein a 39 kilodalton HBsAg polypeptide is included in the immunogen of the vaccine.

0250253

PRE-S POLYPEPTIDE

**FIGURE 1**

**FIGURE 2**

FIGURE 3

FIGURE 4

FIGURE 5

0250253

FIGURE 6

FIGURE 7

FIGURE 8

FIGURE 9

AMINO ACID SEQUENCE OF RESIDUES 120-145 IN THE PRE-S(2) REGION

SUBTYPE

*ayw*    M Q W N S T T F H Q T L Q D P R V R G L Y F P A G G

*adw₂*  — — — — — — A — — — — — — — — — — — — — — L — — — — —

*adw*  — — — — — — A L — — A — — — — — — — — — — L — — — —

```
120              T                                    F      145
|————————————————————————————————————————————————————————————|

120              A                                    L      145
|————————————————————————————————————————————————————————————|

120              T              132
|——————————————————————————————|

120              A              132
|——————————————————————————————|

120              A          131
|——————————————————————————|

                        133                    F           145
                        |——————————————————————————————————|

                  128                      138
                  |————————————————————————|
```

**FIGURE 10**

| Immunogen | Strain | H-2 | % Maximum Anti-Pre-S(2) Titer |
|-----------|--------|-----|-------------------------------|

FIGURE 11

0250253

FIGURE 12

FIGURE 13

**FIGURE 14**

0250253

FIGURE 15

0250253

FIGURE 16

0250253

## AMINO ACID SEQUENCE OF THE PRE·S(2) REGION

ayw  M Q W N S T T F H Q T L Q D P R V R G L Y F P A G G S S S G T V

adw₁  — — — — — — A — — — — — — — — — — — — — L — — — — — — — — —

ayw  N P V L T T A S P L S S I F S R I G D P A L N

adw₁  — — A P N I — — H I — — — S A — T — — — V T —

**FIGURE 17**

FIGURE 18

| Antigen | Subtype | Pre-S(2)-Specific Mab | | |
|---|---|---|---|---|
| | | 5521 | 5161 | 4408 |
| HBsAg/p33 22-nm particles | ay | + + + + | + + + + | + + + + |
| | ad | + + + + | + + + + | + + + + |
| 120 126 133 141 145 151 | ay | + + + + | + + + + | + + + + |
| | ad | + + + + | + + + | + + + + |
| 120 132 | ay | 0 | 0 | 0 |
| | ad | 0 | 0 | 0 |
| 133 151 | ay | + + + + | + + + + | + + + + |
| | ad | + + + | + | + + + + |
| 133 145 | ay | + + + | + + + | + + + |
| | ad | ± | ± | + + + |
| 133 139 | ay/ad | 0 | 0 | + + |
| 128 | ay/ad | 0 | 0 | 0 |
| 135 138 145 | ay | + + + | + + | 0 |
| | ad | 0 | 0 | 0 |
| 135 143 | ay | + + + | + + | 0 |
| | ad | 0 | 0 | 0 |
| 137 145 | ay | + + | + | 0 |
| | ad | 0 | 0 | 0 |

Epitope 2
(5520, 5521, 5535, 5161)

133 134 135 136 137 138 139 140 141 142 143

ayw
adr } D P R V R G L Y F P A

adw₂
adw } — — — — — — — — L — —

Epitope 1
(4408)

**FIGURE 19**

0250253

**FIGURE 20**

Percent Inhibition

Competing Antibody (ng/ml)

5521

5161

5520

5535

4408

0.6  1.2  2.5  5  10  40  156  625

20  40  60  80  100

FIGURE 21

FIGURE 22

0250253

**FIGURE 23**

FIGURE 24